# EUROPEAN PATENT APPLICATION

(11) **EP 4 572 589 A1**
(43) Date of publication of application: **18.06.2025**
(21) Application number: 24212248.9
(22) Date of filing: 12.11.2024
(51) Int. Cl.: H10K 85/60

(54) **HETEROCYCLIC COMPOUND, ORGANIC LIGHT EMITTING DEVICE COMPRISING THE SAME AND COMPOSITION FOR ORGANIC MATERIAL LAYER**

(30) Priority: 15.12.2023 KR 20230183322
(71) Applicant: LT Materials Co., Ltd., Yongin-si, Gyeonggi-do 17118 (KR)
(72) Inventor: LEE, Gi Back, 17118 Yongin-si, Gyeonggi-do (KR); MO, Jun Tae, 17118 Yongin-si, Gyeonggi-do (KR); KIM, Dong Jun, 17118 Yongin-si, Gyeonggi-do (KR); CHOI, Dae Hyuk, 17118 Yongin-si, Gyeonggi-do (KR)
(74) Representative: Goddar, Heinz J.

(57) **Abstract**

The present disclosure relates to a heterocyclic compound represented by Chemical Formula 1, an organic light emitting device including the same, and a composition for an organic material layer.

## Description

### [Technical Field]

This application claims priority to Korean Patent Application No. 10-2023-0183322, filed on December 15, 2023, the disclosure of which is incorporated herein by reference in its entirety.

The present disclosure relates to a heterocyclic compound, an organic light emitting device comprising the same, and a composition for an organic material layer.

### [Background Art]

An organic light emitting device is one type of self-emissive display devices, and has advantages of having a wide viewing angle and a high response speed as well as having an excellent contrast.

The organic light emitting device has a structure of disposing an organic thin film between two electrodes. When a voltage is applied to the organic light emitting device having such a structure, electrons and holes injected from the two electrodes bind and pair in the organic thin film, and then light is emitted as these annihilate. The organic thin film may be formed in a single layer or a multilayer as necessary.

A material of the organic thin film may have a light emitting function as necessary. For example, as a material of the organic thin film, compounds each capable of forming a light emitting layer themselves alone may be used, or compounds each capable of serving as a host or a dopant of a host-dopant-based light emitting layer may also be used. In addition thereto, compounds capable of performing roles of hole injection, hole transport, electron blocking, hole blocking, electron transport, electron injection and the like may also be used as a material of the organic thin film.

Development of an organic thin film material has been continuously required for enhancing performance, lifetime or efficiency of an organic light emitting device.

### Prior Art Documents

### Patent Documents

US Patent No. 4,356,429

### [Disclosure]

### [Technical Problem]

An object of present disclosure is to provide a heterocyclic compound, an organic light emitting device comprising the same, and a composition for an organic material layer.

### [Technical Solution]

In order to the object, one embodiment of the present disclosure provides a heterocyclic compound represented by the following Chemical Formula 1.

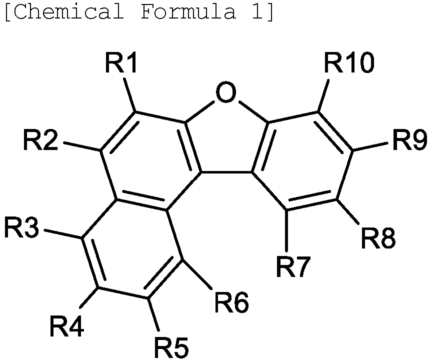

In Chemical Formula 1,
R1 to R10 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; Ar1; - P(=O)R101R102; -S1R101R102R103; and the following Chemical Formula 2, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group,
any one of R1 to R10 is the following Chemical Formula 2, and another one is Ar1, and
Ar1 is a substituted or unsubstituted C6 to C60 aryl group; or the following Chemical Formula 3, 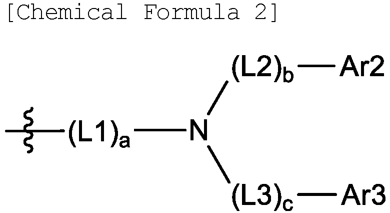
in Chemical Formula 2,
L1 to L3 are the same as or different from each other, and each independently a direct bond; a substituted or unsubstituted C6 to C60 arylene group; or a substituted or unsubstituted C2 to C60 heteroarylene group,
a is an integer of 0 to 5, and when a is 2 or greater, L1s are the same as or different from each other,
b is an integer of 0 to 5, and when b is 2 or greater, L2s are the same as or different from each other,
c is an integer of 0 to 5, and when c is 2 or greater, L3s are the same as or different from each other,
Ar2 and Ar3 are the same as or different from each other, and each independently a substituted or unsubstituted C6 to C60 aryl group; or the following Chemical Formula 3, and
any one of Ar1 to Ar3 is the following Chemical Formula 3, 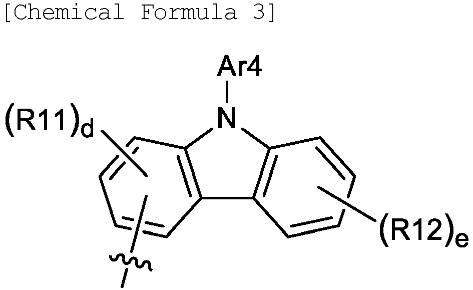
in Chemical Formula 3,
R11 and R12 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; -SiR101R102R103; and -NR101R102, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group,
d is an integer of 0 to 3, and when d is 2 or greater, R11s are the same as or different from each other,
e is an integer of 0 to 4, and when e is 2 or greater, R12s are the same as or different from each other, and
Ar4 is a substituted or unsubstituted C6 to C60 aryl group; or a substituted or unsubstituted C2 to C60 heteroaryl group.

In addition, the present disclosure provides an organic light emitting device including:
a first electrode;
a second electrode provided opposite to the first electrode; and
one or more organic material layers provided between the first electrode and the second electrode,
wherein one or more layers of the one or more organic material layers comprise the heterocyclic compound represented by Chemical Formula 1.

In addition, the present disclosure provides an organic light emitting device, wherein the organic material layer further includes a heterocyclic compound represented by the following Chemical Formula 4.

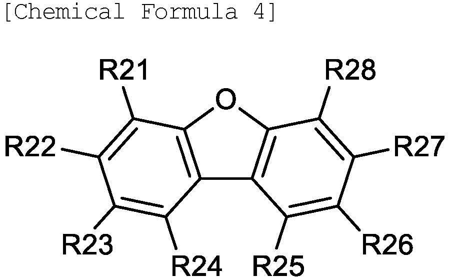

In Chemical Formula 4,
R21 to R28 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; -P(=O)R201R202; -SiR201R202R203; the following Chemical Formula 5; the following Chemical Formula 6; and the following Chemical Formula 7, or two or more groups adjacent to each other bond to each other to form a substituted or unsubstituted C6 to C60 aromatic hydrocarbon ring; or a substituted or unsubstituted C2 to C60 heteroring, and R201, R202 and R203 are the same as or different from each other and each independently a substituted or unsubstituted C1 to C60 alkyl group; a substituted or unsubstituted C6 to C60 aryl group; or a substituted or unsubstituted C2 to C60 heteroaryl group, and
any one of R21 to R28 is the following Chemical Formula 5, and another one is the following Chemical Formula 6; or the following Chemical Formula 7, 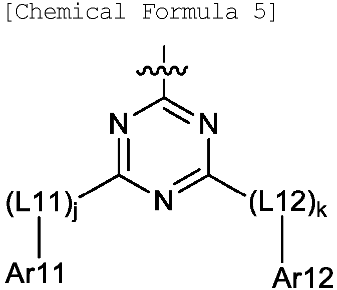 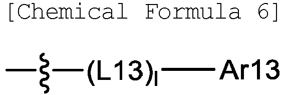 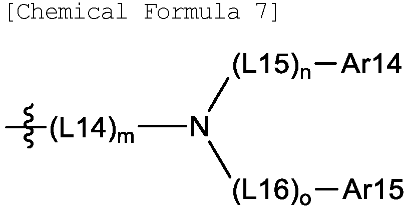
in Chemical Formulae 5 to 7,
L11 to L16 are the same as or different from each other, and each independently a direct bond; a substituted or unsubstituted C6 to C60 arylene group; or a substituted or unsubstituted C2 to C60 heteroarylene group,
j is an integer of 0 to 5, and when j is 2 or greater, L11s are the same as or different from each other,
k is an integer of 0 to 5, and when k is 2 or greater, L12s are the same as or different from each other,
l is an integer of 0 to 5, and when l is 2 or greater, L13s are the same as or different from each other,
m is an integer of 0 to 5, and when m is 2 or greater, L14s are the same as or different from each other,
n is an integer of 0 to 5, and when n is 2 or greater, L15s are the same as or different from each other,
o is an integer of 0 to 5, and when o is 2 or greater, L16s are the same as or different from each other, and
Ar11 to Ar15 are the same as or different from each other, and each independently a substituted or unsubstituted C6 to C60 aryl group; or a substituted or unsubstituted C2 to C60 heteroaryl group.

In addition, the present disclosure provides a composition for an organic material layer, the composition comprising: the heterocyclic compound represented by Chemical Formula 1; and the heterocyclic compound represented by Chemical Formula 4.

### [Advantageous Effects]

A compound described in the present specification can be used as an organic material layer material of an organic light emitting device. The compound is capable of performing roles of a hole injection layer material, a hole transport layer material, a light emitting layer material, an electron transport layer material, an electron injection layer material, an electron blocking layer material and the like in an organic light emitting device.

Particularly, the compound can be used as a hole transport layer material or an electron blocking layer material.

In addition, the compound can be used as a light emitting layer material of an organic light emitting device, and the compound can be used as a light emitting material either alone, or as a host material or a dopant material of a light emitting layer. Specifically, the compound can be used as a light emitting material either alone, or as a host material or a dopant material of a light emitting layer.

When the heterocyclic compound represented by Chemical Formula 1 is used in an organic material layer, it is possible to lower a driving voltage of an organic light emitting device, and enhance light emission efficiency and lifetime properties thereof

### [DESCRIPTION OF DRAWINGS]

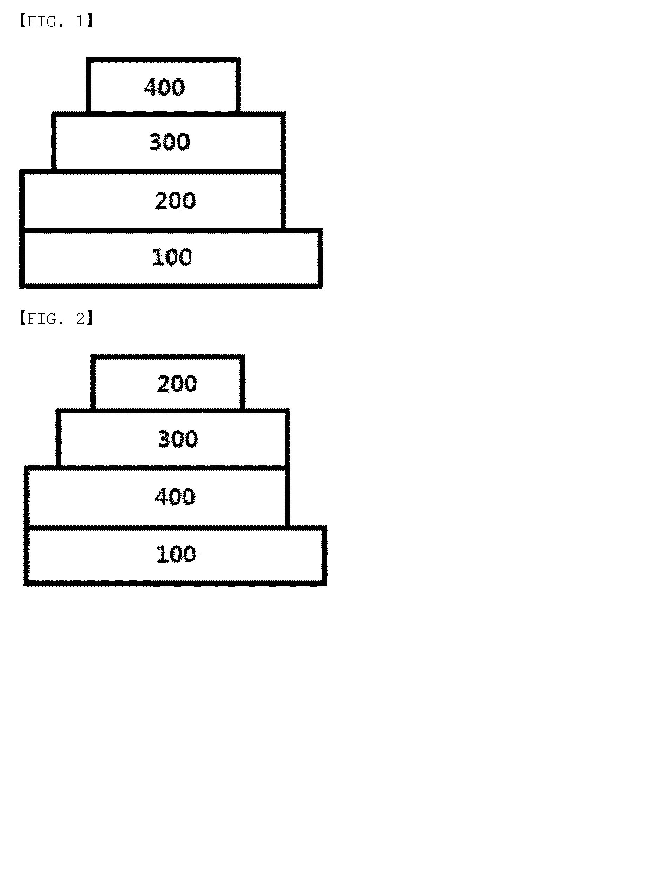

FIGS. 1 to 3 are diagrams each schematically illustrating a lamination structure of an organic light emitting device according to one embodiment of the present disclosure.

### [MODE FOR DISCLOSURE]

Hereinafter, the present disclosure will be described in more detail.

In the present specification, a term "substitution" means that a hydrogen atom bonding to a carbon atom of a compound is changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, "substituted or unsubstituted" means being substituted with one or more substituents selected from the group consisting of deuterium; halogen; a cyano group; a C1 to C60 linear or branched alkyl group; a C2 to C60 linear or branched alkenyl group; a C2 to C60 linear or branched alkynyl group; a C1 to C60 linear, branched or cyclic alkoxy group; a C3 to C60 monocyclic or polycyclic cycloalkyl group; a C2 to C60 monocyclic or polycyclic heterocycloalkyl group; a C6 to C60 monocyclic or polycyclic aryl group; a C2 to C60 monocyclic or polycyclic heteroaryl group; - SiRR'R"; -P(=O)RR'; a C1 to C20 alkylamine group; a C6 to C60 monocyclic or polycyclic arylamine group; and a C2 to C60 monocyclic or polycyclic heteroarylamine group or being unsubstituted, or being substituted with a substituent in which two or more substituents selected from among the substituents exemplified above are linked or being unsubstituted, and R, R' and R" are the same as or different from each other and each independently a substituted or unsubstituted C1 to C60 alkyl group; a substituted or unsubstituted C6 to C60 aryl group; or a substituted or unsubstituted C2 to C60 heteroaryl group.

In the present specification, the halogen may be fluorine; chlorine; bromine; or iodine.

In the present specification, the alkyl group includes a linear or branched form having 1 to 60 carbon atoms, and may be further substituted with other substituents. The number of carbon atoms of the alkyl group may be from 1 to 60, specifically from 1 to 40 and more specifically from 1 to 20. Specific examples of the alkyl group may include a methyl group; an ethyl group; an n-propyl group; an isopropyl group; an n-butyl group; an isobutyl group; a tert-butyl group; a sec-butyl group; a 1-methyl-butyl group; a 1-ethyl-butyl group; an n-pentyl group; an isopentyl group; a neopentyl group; a tert-pentyl group; an n-hexyl group; a 1-methylpentyl group; a 2-methylpentyl group; a 4-methyl-2-pentyl group; a 3,3-dimethylbutyl group; a 2-ethylbutyl group; an n-heptyl group; a 1-methylhexyl group; a cyclopentylmethyl group; a cyclohexylmethyl group; an n-octyl group; a tert-octyl group; a 1-methylheptyl group; a 2-ethylhexyl group; a 2-propylpentyl group; an n-nonyl group; a 2,2-dimethylheptyl group; a 1-ethyl-propyl group; a 1,1-dimethyl-propyl group; an isohexyl group; a 4-methylhexyl group; a 5-methylhexyl group and the like, but are not limited thereto.

In the present specification, the alkenyl group includes a linear or branched form having 2 to 60 carbon atoms, and may be further substituted with other substituents. The number of carbon atoms of the alkenyl group may be from 2 to 60, specifically from 2 to 40 and more specifically from 2 to 20. Specific examples of the alkenyl group may include a vinyl group; a 1-propenyl group; an isopropenyl group; a 1-butenyl group; a 2-butenyl group; a 3-butenyl group; a 1-pentenyl group; a 2-pentenyl group; a 3-pentenyl group; a 3-methyl-1-butenyl group; a 1,3-butadienyl group; an allyl group; a 1-phenylvinyl-1-yl group; a 2-phenylvinyl-1-yl group; a 2,2-diphenylvinyl-1-yl group; a 2-phenyl-2-(naphthyl-1-yl)vinyl-1-yl group; a 2,2-bis(diphenyl-1-yl)vinyl-1-yl group; a stilbenyl group; a styrenyl group and the like, but are not limited thereto.

In the present specification, the alkynyl group includes a linear or branched form having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples of the alkoxy group may include a methoxy group; an ethoxy group; an n-propoxy group; an isopropoxy group; an n-butoxy group; an isobutoxy group; a tert-butoxy group; a sec-butoxy group; an n-pentyloxy group; a neopentyloxy group; an isopentyloxy group; an n-hexyloxy group; a 3,3-dimethylbutyloxy group; a 2-ethylbutyloxy group; an n-octyloxy group; an n-nonyloxy group; an n-decyloxy group; a benzyloxy group; a p-methylbenzyloxy group and the like, but are not limited thereto.

In the present specification, the cycloalkyl group includes a monocyclic or polycyclic group having 3 to 60 carbon atoms, and may be further substituted with other substituents. Herein, the polycyclic group means a group in which the cycloalkyl group is directly linked to or fused with another cyclic group. Herein, the another cyclic group may be a cycloalkyl group, but may also be different types of cyclic groups such as a heterocycloalkyl group; an aryl group; and a heteroaryl group. The number of carbon atoms of the cycloalkyl group may be from 3 to 60, specifically from 3 to 40 and more specifically from 5 to 20. Specific examples of the cycloalkyl group may include a cyclopropyl group; a cyclobutyl group; a cyclopentyl group; a 3-methylcyclopentyl group; a 2,3-dimethylcyclopentyl group; a cyclohexyl group; a 3-methylcyclohexyl group; a 4-methylcyclohexyl group; a 2,3-dimethylcyclohexyl group; a 3,4,5-trimethylcyclohexyl group; a 4-tert-butylcyclohexyl group; a cycloheptyl group; a cyclooctyl group and the like, but are not limited thereto.

In the present specification, the heterocycloalkyl group includes O, S, Se, N or Si as a heteroatom, includes a monocyclic or polycyclic group having 2 to 60 carbon atoms, and may be further substituted with other substituents. Herein, the polycyclic group means a group in which the heterocycloalkyl group is directly linked to or fused with another cyclic group. Herein, the another cyclic group may be a heterocycloalkyl group, but may also be different types of cyclic groups such as a cycloalkyl group; an aryl group; and a heteroaryl group. The number of carbon atoms of the heterocycloalkyl group may be from 2 to 60, specifically from 2 to 40 and more specifically from 3 to 20.

In the present specification, the aryl group includes a monocyclic or polycyclic group having 6 to 60 carbon atoms, and may be further substituted with other substituents. Herein, the polycyclic group means a group in which the aryl group is directly linked to or fused with another cyclic group. Herein, the another cyclic group may be an aryl group, but may also be different types of cyclic groups such as a cycloalkyl group; a heterocycloalkyl group; and a heteroaryl group. The aryl group may include a spiro group. The number of carbon atoms of the aryl group may be from 6 to 60, specifically from 6 to 40 and more specifically from 6 to 20. Specific examples of the aryl group may include a phenyl group; a biphenyl group; a triphenyl group; a naphthyl group; an anthryl group; a chrysenyl group; a phenanthrenyl group; a perylenyl group; a fluoranthenyl group; a triphenylenyl group; a phenalenyl group; a pyrenyl group; a tetracenyl group; a pentacenyl group; a fluorenyl group; an indenyl group; an acenaphthylenyl group; a benzofluorenyl group; a spirobifluorenyl group; a 2,3-dihydro-1H-indenyl group; a fused ring group thereof, and the like, but are not limited thereto.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specifically, the phosphine oxide group may be substituted with an aryl group, and as the aryl group, the examples described above may be applied. Examples of the phosphine oxide group may include a diphenylphosphine oxide group; a dinaphthylphosphine oxide group and the like, but are not limited thereto.

In the present specification, the silyl group is a substituent including Si and having the Si atom directly linked as a radical, and is represented by -SiR101R102R103. R101 to R103 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may include a trimethylsilyl group; a triethylsilyl group; a t-butyldimethylsilyl group; a vinyldimethylsilyl group; a propyldimethylsilyl group; a triphenylsilyl group; a diphenylsilyl group; a phenylsilyl group and the like, but are not limited thereto.

In the present specification, the fluorenyl group may be substituted, and adjacent substituents may bond to each other to form a ring.

When the fluorenyl group is substituted, 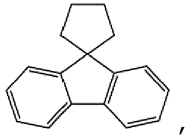 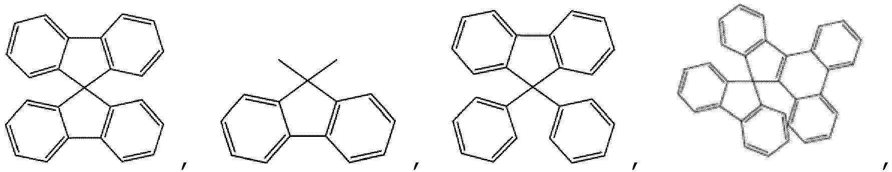 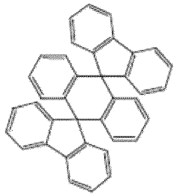 and the like may be included, however, the structure is not limited thereto.

In the present specification, the spiro group is a group including a spiro structure, and may have 15 to 60 carbon atoms. For example, the spiro group may include a structure in which a 2,3-dihydro-1H-indene group or a cyclohexane group spiro bonds to a fluorenyl group. Specifically, the spiro group may include any one of groups of the following structural formulae.

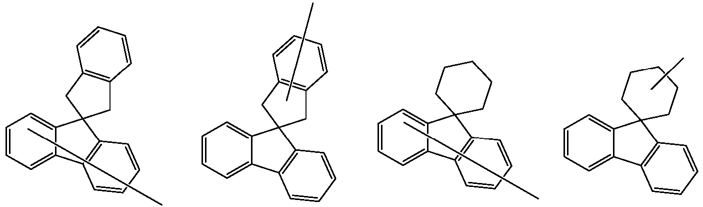

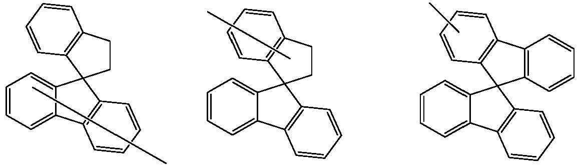

In the present specification, the heteroaryl group includes S, O, Se, N or Si as a heteroatom, includes a monocyclic or polycyclic group having 2 to 60 carbon atoms, and may be further substituted with other substituents. Herein, the polycyclic group means a group in which the heteroaryl group is directly linked to or fused with another cyclic group. Herein, the another cyclic group may be a heteroaryl group, but may also be different types of cyclic groups such as a cycloalkyl group; a heterocycloalkyl group; and an aryl group. The number of carbon atoms of the heteroaryl group may be from 2 to 60, specifically from 2 to 40 and more specifically from 3 to 25. Specific examples of the heteroaryl group may include a pyridyl group; a pyrrolyl group; a pyrimidyl group; a pyridazinyl group; a furanyl group; a thiophenyl group; an imidazolyl group; a pyrazolyl group; an oxazolyl group; an isoxazolyl group; a thiazolyl group; an isothiazolyl group; a triazolyl group; a furazanyl group; an oxadiazolyl group; a thiadiazolyl group; a dithiazolyl group; a tetrazolyl group; a pyranyl group; a thiopyranyl group; a diazinyl group; an oxazinyl group; a thiazinyl group; a dioxynyl group; a triazinyl group; a tetrazinyl group; a quinolyl group; an isoquinolyl group; a quinazolinyl group; an isoquinazolinyl group; a quinozolinyl group; a naphthyridyl group; an acridinyl group; a phenanthridinyl group; an imidazopyridinyl group; a diazanaphthalenyl group; a triazaindenyl group; a 2-indolyl group; an indolizinyl group; a benzothiazolyl group; a benzoxazolyl group; a benzimidazolyl group; a benzothiophenyl group; a benzofuranyl group; a dibenzothiophenyl group; a dibenzofuranyl group; a carbazolyl group; a benzocarbazolyl group; a dibenzocarbazolyl group; a phenazinyl group; a dibenzosilole group; a spirobi(dibenzosilole) group; a dihydrophenazinyl group; a phenoxazinyl group; a phenanthridyl group; a thienyl group; an indolo[2,3-a]carbazolyl group; an indolo[2,3-b]carbazolyl group; an indolinyl group; a 10,11-dihydro-dibenzo[b,f]azepinyl group; a 9,10-dihydroacridinyl group; a phenanthrazinyl group; a phenothiazinyl group; a phthalazinyl group; a naphthylidinyl group; a phenanthrolinyl group; a benzo[c] [1,2,5]thiadiazolyl group; a 5,10-dihydrodibenzo[b,e] [1,4]azasilinyl group; a pyrazolo[1,5-c]quinazolinyl group; a pyrido[1,2-b]indazolyl group; a pyrido[1,2-a]imidazo[1,2-e]indolinyl group; a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms is not particularly limited, but preferably from 1 to 30. Specific examples of the amine group may include a methylamine group; a dimethylamine group; an ethylamine group; a diethylamine group; a phenylamine group; a naphthylamine group; a biphenylamine group; a dibiphenylamine group; an anthracenylamine group; a 9-methyl-anthracenylamine group; a diphenylamine group; a phenylnaphthylamine group; a ditolylamine group; a phenyltolylamine group; a triphenylamine group; a biphenylnaphthylamine group; a phenylbiphenylamine group; a biphenylfluorenylamine group; a phenyltriphenylenylamine group; a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, an "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting at ortho positions in a benzene ring, and two substituents substituting at the same carbon in an aliphatic ring may be interpreted as groups "adjacent" to each other.

In the present disclosure, a "case of a substituent being not indicated in a chemical formula or compound structure" means that a hydrogen atom bonds to a carbon atom. However, since deuterium (²H, D) is an isotope of hydrogen, some hydrogen atoms may be deuterium.

In one embodiment of the present disclosure, a "case of a substituent being not indicated in a chemical formula or compound structure" may mean that positions to which substituents may come are all hydrogen or deuterium. In other words, since deuterium is an isotope of hydrogen, some hydrogen atoms may be deuterium that is an isotope, and herein, a content of the deuterium may be from 0% to 100%.

In one embodiment of the present disclosure, in a "case of a substituent being not indicated in a chemical formula or compound structure", hydrogen and deuterium may be used interchangeably in compounds when deuterium is not explicitly excluded such as "a deuterium content being 0%", "a hydrogen content being 100%" or "substituents being all hydrogen".

In one embodiment of the present disclosure, deuterium is one of isotopes of hydrogen, is an element having deuteron formed with one proton and one neutron as a nucleus, and may be expressed as hydrogen-2, and the elemental symbol thereof may also be written as D or ²H.

In one embodiment of the present disclosure, an isotope means an atom with the same atomic number (Z) but with a different mass number (A), and may also be interpreted as an element with the same number of protons but with a different number of neutrons.

In one embodiment of the present disclosure, a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 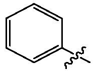 may mean that the total number of substituents that the phenyl group may have is 5 (T1 in the formula), and the number of deuterium atoms among these is 1 (T2 in the formula). In other words, having a deuterium content of 20% in a phenyl group may be represented by the following structural formulae.

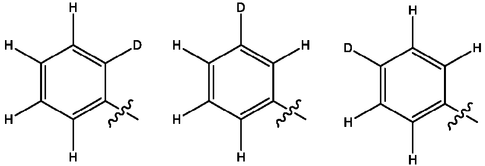

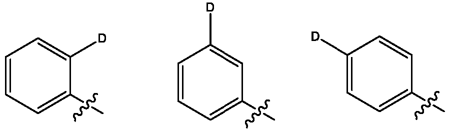

In addition, in one embodiment of the present disclosure, "a phenyl group having a deuterium content of 0%" may mean a phenyl group that does not include a deuterium atom, that is, a phenyl group that has 5 hydrogen atoms.

In the present disclosure, the C6 to C60 aromatic hydrocarbon ring means a compound including an aromatic ring formed with C6 to C60 carbons and hydrogens. Examples thereof may include phenyl, biphenyl, terphenyl, triphenylene, naphthalene, anthracene, phenalene, phenanthrene, fluorene, pyrene, chrysene, perylene, azulene and the like, but are not limited thereto, and include all aromatic hydrocarbon ring compounds known in the art satisfying the above-mentioned number of carbon atoms.

One embodiment of the present disclosure provides a heterocyclic compound represented by the following Chemical Formula 1.

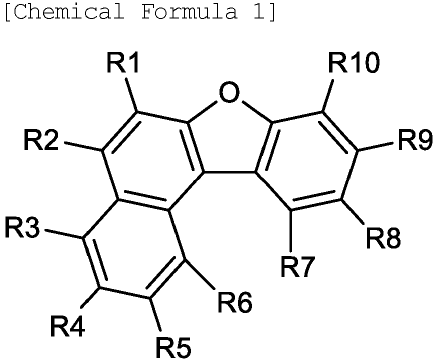

In Chemical Formula 1,
R1 to R10 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; Ar1; - P(=O)R101R102; -SiR101R102R103; and the following Chemical Formula 2, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group,
any one of R1 to R10 is the following Chemical Formula 2, and another one is Ar1, and
Ar1 is a substituted or unsubstituted C6 to C60 aryl group; or the following Chemical Formula 3, 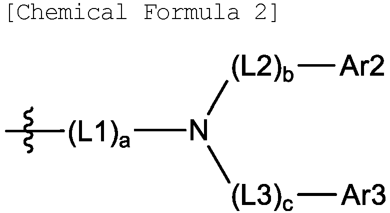
in Chemical Formula 2,
L1 to L3 are the same as or different from each other, and each independently a direct bond; a substituted or unsubstituted C6 to C60 arylene group; or a substituted or unsubstituted C2 to C60 heteroarylene group,
a is an integer of 0 to 5, and when a is 2 or greater, L1s are the same as or different from each other,
b is an integer of 0 to 5, and when b is 2 or greater, L2s are the same as or different from each other,
c is an integer of 0 to 5, and when c is 2 or greater, L3s are the same as or different from each other,
Ar2 and Ar3 are the same as or different from each other, and each independently a substituted or unsubstituted C6 to C60 aryl group; or the following Chemical Formula 3, and
any one of Ar1 to Ar3 is the following Chemical Formula 3, 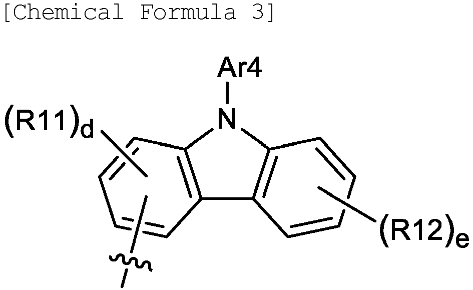
in Chemical Formula 3,
R11 and R12 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; -SiR101R102R103; and -NR101R102, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group,
d is an integer of 0 to 3, and when d is 2 or greater, R11s are the same as or different from each other,
e is an integer of 0 to 4, and when e is 2 or greater, R12s are the same as or different from each other, and
Ar4 is a substituted or unsubstituted C6 to C60 aryl group; or a substituted or unsubstituted C2 to C60 heteroaryl group.

In one embodiment of the present disclosure, R1 to R10 are the same as or different from each other, and each independently hydrogen; deuterium; halogen; a cyano group; a substituted or unsubstituted C1 to C30 alkyl group; a substituted or unsubstituted C2 to C30 alkenyl group; a substituted or unsubstituted C2 to C30 alkynyl group; a substituted or unsubstituted C1 to C30 alkoxy group; a substituted or unsubstituted C3 to C30 cycloalkyl group; a substituted or unsubstituted C2 to C30 heterocycloalkyl group; Ar1; - P(=O)R101R102; -S1R101R102R103; or Chemical Formula 2, or two or more groups adjacent to each other bond to each other to form a substituted or unsubstituted C6 to C30 aromatic hydrocarbon ring; or a substituted or unsubstituted C2 to C30 heteroring, and R101, R102 and R103 are the same as or different from each other and may be each independently a substituted or unsubstituted C1 to C30 alkyl group; a substituted or unsubstituted C6 to C30 aryl group; or a substituted or unsubstituted C2 to C30 heteroaryl group, and any one of R1 to R10 is Chemical Formula 2 and another one may be Ar1.

In another embodiment of the present disclosure, R1 to R10 are the same as or different from each other, and each independently hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; Ar1; - P(=O)R101R102; -SiR101R102R103; or Chemical Formula 2, or two or more groups adjacent to each other bond to each other to form a substituted or unsubstituted C6 to C20 aromatic hydrocarbon ring; or a substituted or unsubstituted C2 to C20 heteroring, and R101, R102 and R103 are the same as or different from each other and may be each independently a substituted or unsubstituted C1 to C20 alkyl group; a substituted or unsubstituted C6 to C20 aryl group; or a substituted or unsubstituted C2 to C20 heteroaryl group, and any one of R1 to R10 is Chemical Formula 2 and another one may be Ar1.

In another embodiment of the present disclosure, R1 to R10 are the same as or different from each other and each independently hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; Ar1; - P(=O)R101R102; -SiR101R102R103; or Chemical Formula 2, and R101, R102 and R103 are the same as or different from each other and may be each independently a substituted or unsubstituted C1 to C20 alkyl group; a substituted or unsubstituted C6 to C20 aryl group; or a substituted or unsubstituted C2 to C20 heteroaryl group, and any one of R1 to R10 is Chemical Formula 2 and another one may be Ar1.

In another embodiment of the present disclosure, R1 to R10 are the same as or different from each other and may be each independently hydrogen; deuterium; Ar1; or Chemical Formula 2, and any one of R1 to R10 is Chemical Formula 2 and another one may be Ar1.

In another embodiment of the present disclosure, R1 to R10 are the same as or different from each other and may be each independently hydrogen; deuterium; Ar1; or Chemical Formula 2, R1 is Chemical Formula 2, any one of R7 to R10 is Ar1, and the rest may be hydrogen; or deuterium.

In another embodiment of the present disclosure, R1 to R10 are the same as or different from each other and may be each independently hydrogen; deuterium; Ar1; or Chemical Formula 2, R2 is Chemical Formula 2, any one of R7 to R10 is Ar1, and the rest may be hydrogen; or deuterium.

In another embodiment of the present disclosure, R1 to R10 are the same as or different from each other and may be each independently hydrogen; deuterium; Ar1; or Chemical Formula 2, R3 is Chemical Formula 2, any one of R7 to R10 is Ar1, and the rest may be hydrogen; or deuterium.

In another embodiment of the present disclosure, R1 to R10 are the same as or different from each other and may be each independently hydrogen; deuterium; Ar1; or Chemical Formula 2, R4 is Chemical Formula 2, any one of R7 to R10 is Ar1, and the rest may be hydrogen; or deuterium.

In another embodiment of the present disclosure, R1 to R10 are the same as or different from each other and may be each independently hydrogen; deuterium; Ar1; or Chemical Formula 2, R5 is Chemical Formula 2, any one of R7 to R10 is Ar1, and the rest may be hydrogen; or deuterium.

In another embodiment of the present disclosure, R1 to R10 are the same as or different from each other and may be each independently hydrogen; deuterium; Ar1; or Chemical Formula 2, R6 is Chemical Formula 2, any one of R9 and R10 is Ar1, and the rest may be hydrogen; or deuterium.

In another embodiment of the present disclosure, R1 to R10 are the same as or different from each other and may be each independently hydrogen; deuterium; Ar1; or Chemical Formula 2, R7 is Chemical Formula 2, any one of R1 to R5, R9 and R10 is Ar1, and the rest may be hydrogen; or deuterium.

In another embodiment of the present disclosure, R1 to R10 are the same as or different from each other and may be each independently hydrogen; deuterium; Ar1; or Chemical Formula 2, R8 is Chemical Formula 2, any one of R1 to R6 and R10 is Ar1, and the rest may be hydrogen; or deuterium.

In another embodiment of the present disclosure, R1 to R10 are the same as or different from each other and may be each independently hydrogen; deuterium; Ar1; or Chemical Formula 2, R9 is Chemical Formula 2, any one of R1 to R7 is Ar1, and the rest may be hydrogen; or deuterium.

In another embodiment of the present disclosure, R1 to R10 are the same as or different from each other and may be each independently hydrogen; deuterium; Ar1; or Chemical Formula 2, R10 is Chemical Formula 2, any one of R1 to R8 is Ar1, and the rest may be hydrogen; or deuterium.

In one embodiment of the present disclosure, Ar1 may be a substituted or unsubstituted C6 to C30 aryl group; or Chemical Formula 3.

In another embodiment of the present disclosure, Ar1 may be a substituted or unsubstituted C6 to C20 aryl group; or Chemical Formula 3.

In another embodiment of the present disclosure, Ar1 may be a substituted or unsubstituted phenyl group; or Chemical Formula 3.

In one embodiment of the present disclosure, L1 to L3 are the same as or different from each other, and may be each independently a direct bond; a substituted or unsubstituted C6 to C30 arylene group; or a substituted or unsubstituted C2 to C30 heteroarylene group.

In another embodiment of the present disclosure, L1 to L3 are the same as or different from each other, and may be each independently a direct bond; a substituted or unsubstituted C6 to C20 arylene group; or a substituted or unsubstituted C2 to C20 heteroarylene group.

In another embodiment of the present disclosure, L1 to L3 are the same as or different from each other, and may be each independently a direct bond; or a substituted or unsubstituted C6 to C60 arylene group.

In another embodiment of the present disclosure, L1 to L3 are the same as or different from each other, and may be each independently a direct bond; or a substituted or unsubstituted C6 to C30 arylene group.

In another embodiment of the present disclosure, L1 to L3 are the same as or different from each other, and may be each independently a direct bond; or a substituted or unsubstituted C6 to C20 arylene group.

In another embodiment of the present disclosure, L1 may be a direct bond.

In another embodiment of the present disclosure, L2 and L3 are the same as or different from each other, and may be each independently a direct bond; or a substituted or unsubstituted C6 to C60 arylene group.

In another embodiment of the present disclosure, L2 and L3 are the same as or different from each other, and may be each independently a direct bond; or a substituted or unsubstituted C6 to C30 arylene group.

In another embodiment of the present disclosure, L2 and L3 are the same as or different from each other, and may be each independently a direct bond; or a substituted or unsubstituted C6 to C20 arylene group.

In another embodiment of the present disclosure, L2 and L3 are the same as or different from each other, and may be each independently a direct bond; a substituted or unsubstituted phenylene group; or a substituted or unsubstituted biphenylene group.

In one embodiment of the present disclosure, Ar2 and Ar3 are the same as or different from each other, and may be each independently a substituted or unsubstituted C6 to C30 aryl group; or Chemical Formula 3.

In another embodiment of the present disclosure, Ar2 and Ar3 are the same as or different from each other, and may be each independently a substituted or unsubstituted C6 to C20 aryl group; or Chemical Formula 3.

In another embodiment of the present disclosure, Ar2 and Ar3 are the same as or different from each other, and may be each independently a substituted or unsubstituted phenyl group; a substituted or unsubstituted biphenyl group; a substituted or unsubstituted terphenyl group; a substituted or unsubstituted naphthyl group; a substituted or unsubstituted phenanthrenyl group; or Chemical Formula 3.

In one embodiment of the present disclosure, Ar1 is Chemical Formula 3, and Ar2 and Ar3 may be a substituted or unsubstituted C6 to C60 aryl group.

In another embodiment of the present disclosure, Ar1 is Chemical Formula 3, and Ar2 and Ar3 may be a substituted or unsubstituted C6 to C30 aryl group.

In another embodiment of the present disclosure, Ar1 is Chemical Formula 3, and Ar2 and Ar3 may be a substituted or unsubstituted C6 to C20 aryl group.

In another embodiment of the present disclosure, Ar1 is Chemical Formula 3, and Ar2 and Ar3 may be a substituted or unsubstituted phenyl group; a substituted or unsubstituted biphenyl group; a substituted or unsubstituted terphenyl group; a substituted or unsubstituted naphthyl group; or a substituted or unsubstituted phenanthrenyl group.

In another embodiment of the present disclosure, Ar1 is a substituted or unsubstituted C6 to C60 aryl group, any one of Ar2 and Ar3 is a substituted or unsubstituted C6 to C60 aryl group, and the other one may be Chemical Formula 3.

In another embodiment of the present disclosure, Ar1 is a substituted or unsubstituted C6 to C30 aryl group, any one of Ar2 and Ar3 is a substituted or unsubstituted C6 to C30 aryl group, and the other one may be Chemical Formula 3.

In another embodiment of the present disclosure, Ar1 is a substituted or unsubstituted C6 to C20 aryl group, any one of Ar2 and Ar3 is a substituted or unsubstituted C6 to C20 aryl group, and the other one may be Chemical Formula 3.

In another embodiment of the present disclosure, Ar1 is a substituted or unsubstituted phenyl group, any one of Ar2 and Ar3 is a substituted or unsubstituted phenyl group; or a substituted or unsubstituted biphenyl group, and the other one may be Chemical Formula 3.

In one embodiment of the present disclosure, Ar4 may be a substituted or unsubstituted C6 to C30 aryl group; or a substituted or unsubstituted C2 to C30 heteroaryl group.

In another embodiment of the present disclosure, Ar4 may be a substituted or unsubstituted C6 to C20 aryl group; or a substituted or unsubstituted C2 to C20 heteroaryl group.

In another embodiment of the present disclosure, Ar4 may be a substituted or unsubstituted C6 to C60 aryl group.

In another embodiment of the present disclosure, Ar4 may be a substituted or unsubstituted C6 to C30 aryl group.

In another embodiment of the present disclosure, Ar4 may be a substituted or unsubstituted C6 to C20 aryl group.

In another embodiment of the present disclosure, Ar4 may be a substituted or unsubstituted phenyl group.

In one embodiment of the present disclosure, R11 and R12 are the same as or different from each other, and each independently hydrogen; deuterium; halogen; a cyano group; a substituted or unsubstituted C1 to C30 alkyl group; a substituted or unsubstituted C2 to C30 alkenyl group; a substituted or unsubstituted C2 to C30 alkynyl group; a substituted or unsubstituted C1 to C30 alkoxy group; a substituted or unsubstituted C3 to C30 cycloalkyl group; a substituted or unsubstituted C2 to C30 heterocycloalkyl group; a substituted or unsubstituted C6 to C30 aryl group; a substituted or unsubstituted C2 to C30 heteroaryl group; -P(=O)R101R102; -SiR101R102R103; or -NR101R102, or two or more groups adjacent to each other bond to each other to form a substituted or unsubstituted C6 to C30 aromatic hydrocarbon ring; or a substituted or unsubstituted C2 to C30 heteroring, and R101, R102 and R103 are the same as or different from each other and may be each independently a substituted or unsubstituted C1 to C30 alkyl group; a substituted or unsubstituted C6 to C30 aryl group; or a substituted or unsubstituted C2 to C30 heteroaryl group.

In another embodiment of the present disclosure, R11 and R12 are the same as or different from each other, and each independently hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; -P(=O)R101R102; -SiR101R102R103; or -NR101R102, or two or more groups adjacent to each other bond to each other to form a substituted or unsubstituted C6 to C20 aromatic hydrocarbon ring; or a substituted or unsubstituted C2 to C20 heteroring, and R101, R102 and R103 are the same as or different from each other and may be each independently a substituted or unsubstituted C1 to C20 alkyl group; a substituted or unsubstituted C6 to C20 aryl group; or a substituted or unsubstituted C2 to C20 heteroaryl group.

In another embodiment of the present disclosure, R11 and R12 are the same as or different from each other and each independently hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; -P(=O)R101R102; -SiR101R102R103; or -NR101R102, and R101, R102 and R103 are the same as or different from each other and may be each independently a substituted or unsubstituted C1 to C20 alkyl group; a substituted or unsubstituted C6 to C20 aryl group; or a substituted or unsubstituted C2 to C20 heteroaryl group.

In another embodiment of the present disclosure, R11 and R12 are the same as or different from each other, and may be each independently hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; or a substituted or unsubstituted C2 to C20 heteroaryl group.

In another embodiment of the present disclosure, R11 and R12 are the same as or different from each other, and may be each independently hydrogen; deuterium; a substituted or unsubstituted C6 to C20 aryl group; or a substituted or unsubstituted C2 to C20 heteroaryl group.

In another embodiment of the present disclosure, R11 and R12 are the same as or different from each other, and may be each independently hydrogen; or deuterium.

In one embodiment of the present disclosure, the heterocyclic compound represented by Chemical Formula 1 may be represented by any one of the following Chemical Formulae 1-1 to 1-3.

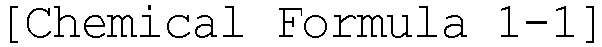

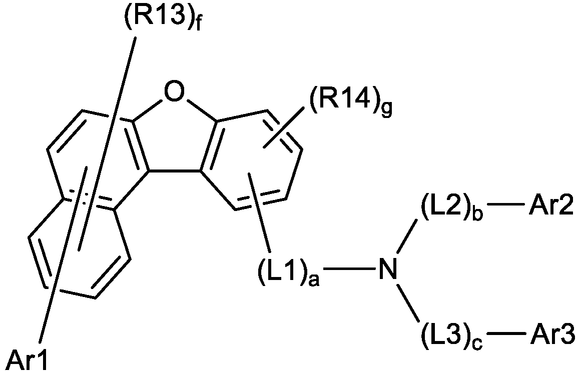

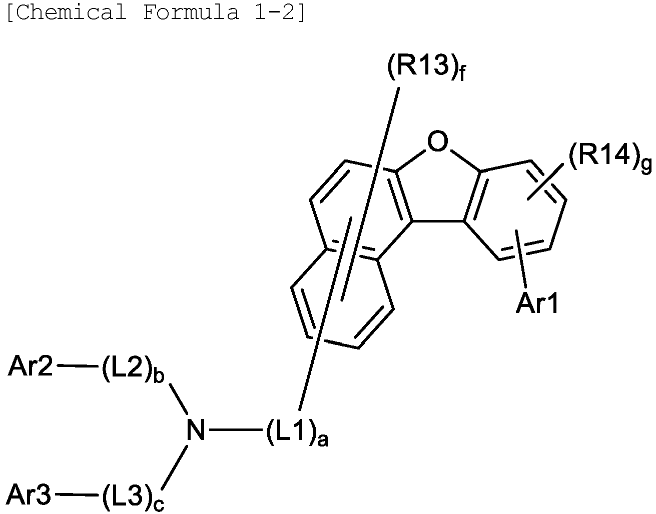

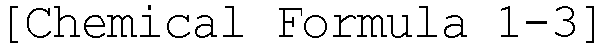

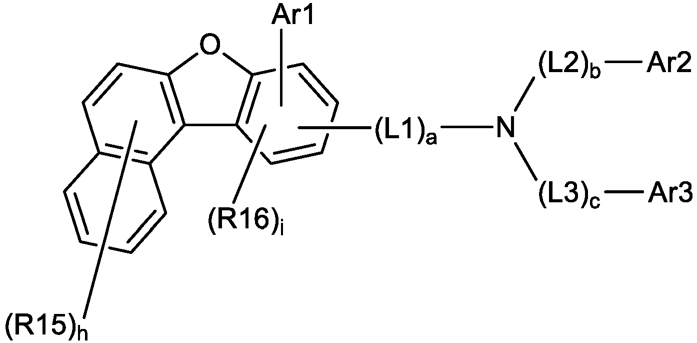

In Chemical Formulae 1-1 to 1-3,
R13 to R16 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; -SiR101R102R103; and -NR101R102, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group,
f is an integer of 0 to 5, and when f is 2 or greater, R13s are the same as or different from each other,
g is an integer of 0 to 3, and when g is 2 or greater, R14s are the same as or different from each other,
h is an integer of 0 to 6, and when h is 2 or greater, R15s are the same as or different from each other,
i is an integer of 0 to 2, and when i is 2 or greater, R16s are the same as or different from each other,
Ar1 has the same definition as in Chemical Formula 1, and
L1 to L3, Ar2, Ar3 and a to c have the same definitions as in Chemical Formula 2.

In one embodiment of the present disclosure, R13 to R16 are the same as or different from each other, and each independently hydrogen; deuterium; halogen; a cyano group; a substituted or unsubstituted C1 to C30 alkyl group; a substituted or unsubstituted C2 to C30 alkenyl group; a substituted or unsubstituted C2 to C30 alkynyl group; a substituted or unsubstituted C1 to C30 alkoxy group; a substituted or unsubstituted C3 to C30 cycloalkyl group; a substituted or unsubstituted C2 to C30 heterocycloalkyl group; a substituted or unsubstituted C6 to C30 aryl group; a substituted or unsubstituted C2 to C30 heteroaryl group; -P(=O)R101R102; -SiR101R102R103; or -NR101R102, or two or more groups adjacent to each other bond to each other to form a substituted or unsubstituted C6 to C30 aromatic hydrocarbon ring; or a substituted or unsubstituted C2 to C30 heteroring, and R101, R102 and R103 are the same as or different from each other and may be each independently a substituted or unsubstituted C1 to C30 alkyl group; a substituted or unsubstituted C6 to C30 aryl group; or a substituted or unsubstituted C2 to C30 heteroaryl group.

In another embodiment of the present disclosure, R13 to R16 are the same as or different from each other, and each independently hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; -P(=O)R101R102; -SiR101R102R103; or -NR101R102, or two or more groups adjacent to each other bond to each other to form a substituted or unsubstituted C6 to C20 aromatic hydrocarbon ring; or a substituted or unsubstituted C2 to C20 heteroring, and R101, R102 and R103 are the same as or different from each other and may be each independently a substituted or unsubstituted C1 to C20 alkyl group; a substituted or unsubstituted C6 to C20 aryl group; or a substituted or unsubstituted C2 to C20 heteroaryl group.

In another embodiment of the present disclosure, R13 to R16 are the same as or different from each other and each independently hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; -P(=O)R101R102; -SiR101R102R103; or -NR101R102, and R101, R102 and R103 are the same as or different from each other and may be each independently a substituted or unsubstituted C1 to C20 alkyl group; a substituted or unsubstituted C6 to C20 aryl group; or a substituted or unsubstituted C2 to C20 heteroaryl group.

In another embodiment of the present disclosure, R13 to R16 are the same as or different from each other, and may be each independently hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; or a substituted or unsubstituted C2 to C20 heteroaryl group.

In another embodiment of the present disclosure, R13 to R16 are the same as or different from each other, and may be each independently hydrogen; deuterium; a substituted or unsubstituted C6 to C20 aryl group; or a substituted or unsubstituted C2 to C20 heteroaryl group.

In another embodiment of the present disclosure, R13 to R16 are the same as or different from each other, and may be each independently hydrogen; or deuterium.

In one embodiment of the present disclosure, R1 to R16, Ar1 to Ar4 and L1 to L3 may all include hydrogen (H) that is not deuterated.

In another embodiment of the present disclosure, at least one of R1 to R16, Ar1 to Ar4 and L1 to L3 includes deuterium (D), and at least one of R1 to R16, Ar1 to Ar4 and L1 to L3 may include hydrogen that is not deuterated.

In another embodiment of the present disclosure, R1 to R16, Ar1 to Ar4 and L1 to L3 may all include deuterium.

In one embodiment of the present disclosure, the heterocyclic compound represented by Chemical Formula 1 may not include deuterium as a substituent, or may have a deuterium content of, for example, greater than 0%, 1% or greater, 10% or greater, 20% or greater, 30% or greater, 40% or greater or 50% or greater, and 100% or less, 90% or less, 80% or less, 70% or less or 60% or less with respect to the total number of hydrogen atoms and deuterium atoms.

In another embodiment of the present disclosure, the heterocyclic compound represented by Chemical Formula 1 may not include deuterium as a substituent, or may have a deuterium content of 1% to 100% with respect to the total number of hydrogen atoms and deuterium atoms.

In another embodiment of the present disclosure, the heterocyclic compound represented by Chemical Formula 1 may not include deuterium as a substituent, or may have a deuterium content of 20% to 90% with respect to the total number of hydrogen atoms and deuterium atoms.

In another embodiment of the present disclosure, the heterocyclic compound represented by Chemical Formula 1 may not include deuterium as a substituent, or may have a deuterium content of 30% to 80% with respect to the total number of hydrogen atoms and deuterium atoms.

In another embodiment of the present disclosure, the heterocyclic compound represented by Chemical Formula 1 may not include deuterium as a substituent, or may have a deuterium content of 50% to 70% with respect to the total number of hydrogen atoms and deuterium atoms.

In one embodiment of the present disclosure, the heterocyclic compound represented by Chemical Formula 1 may be represented by any one of the following compounds.

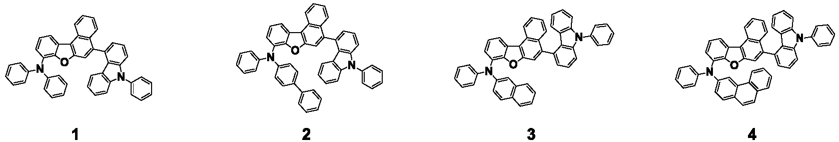

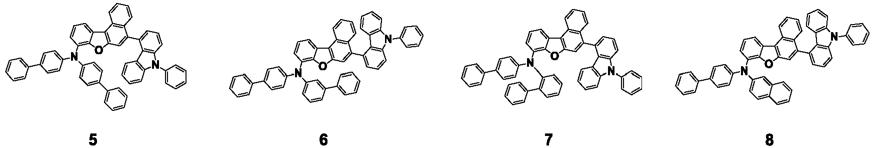

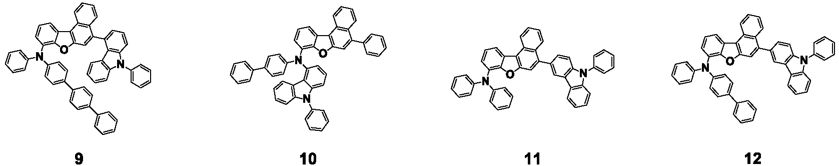

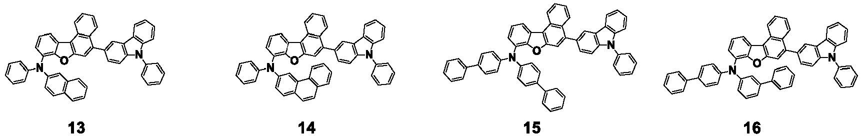

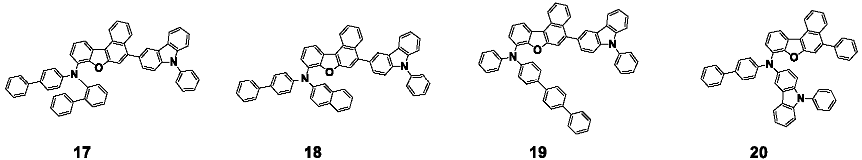

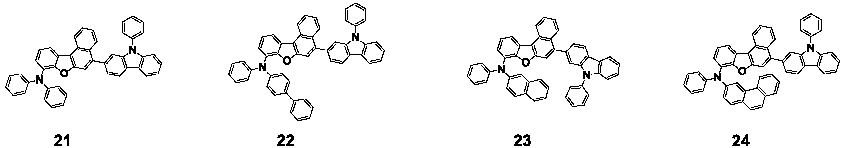

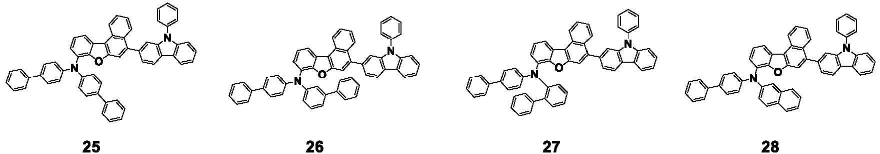

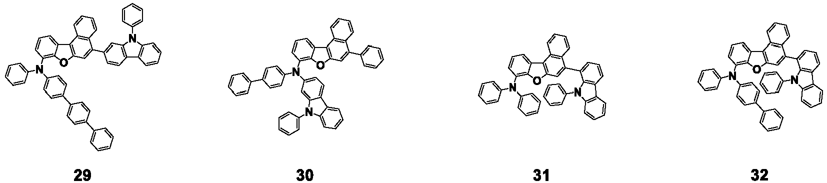

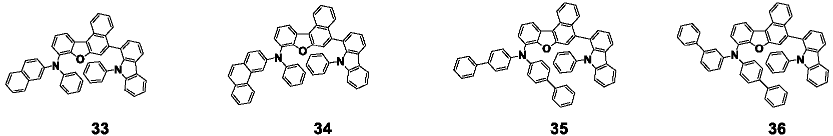

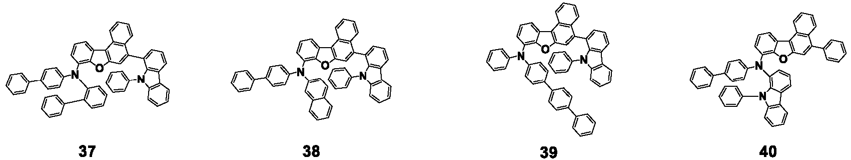

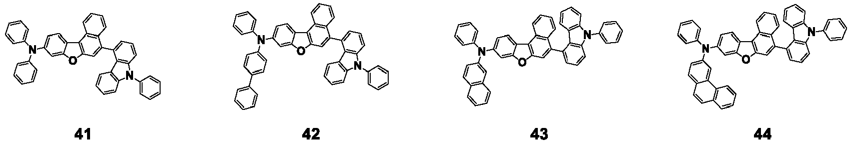

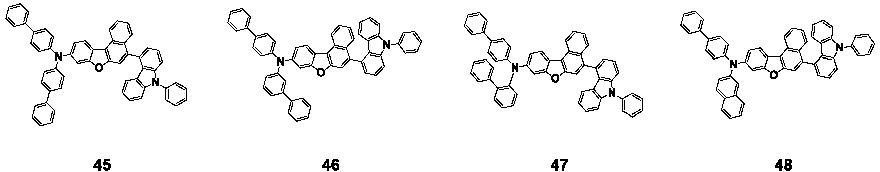

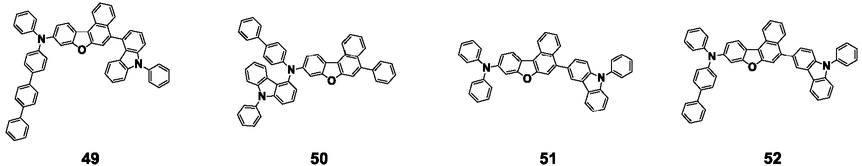

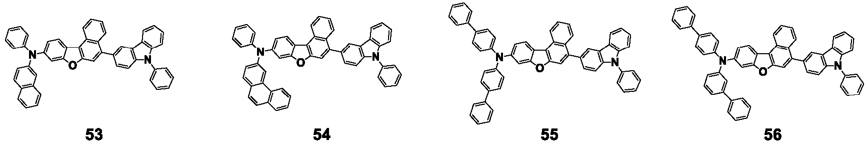

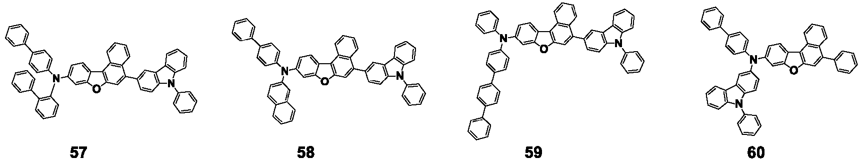

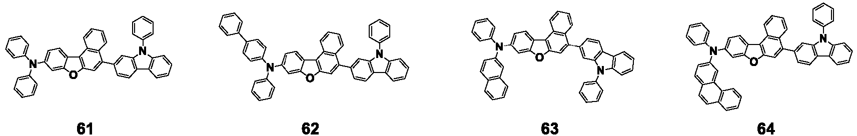

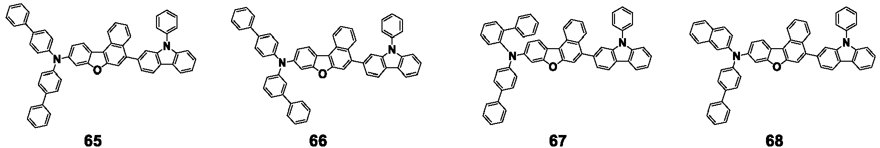

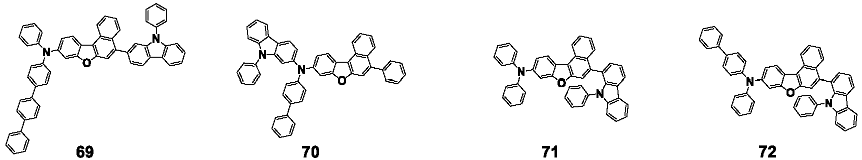

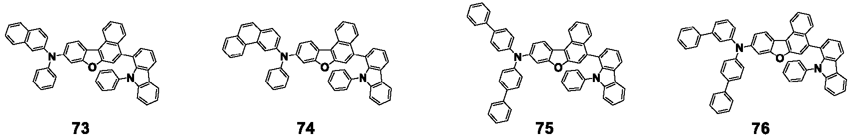

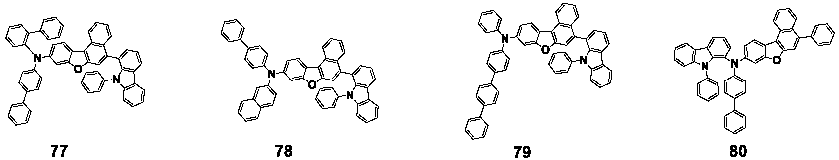

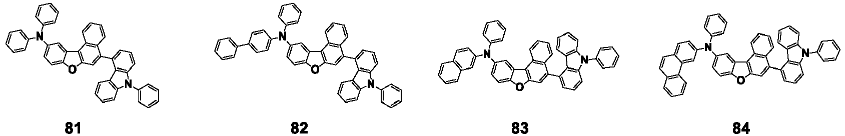

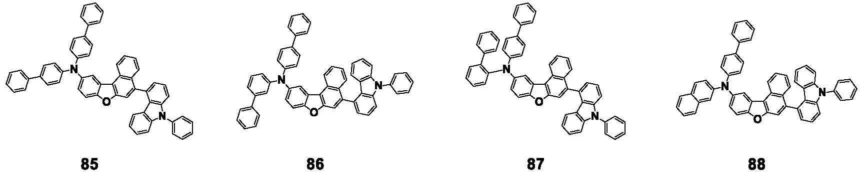

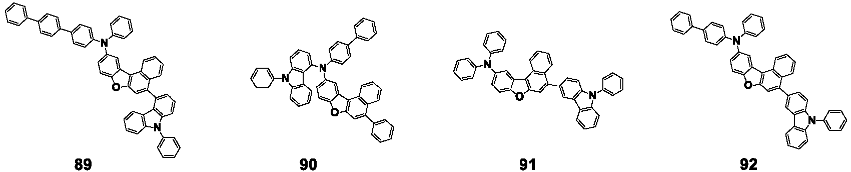

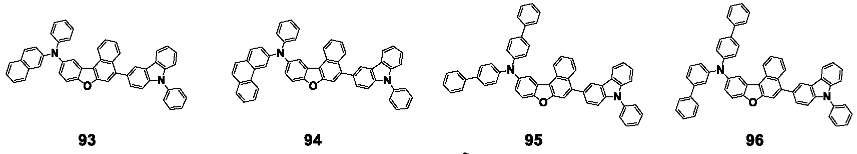

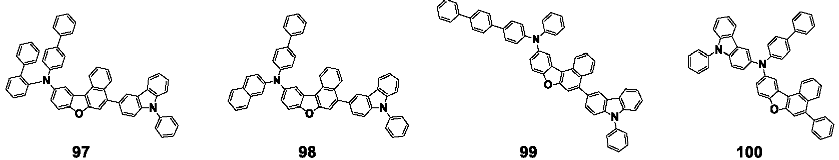

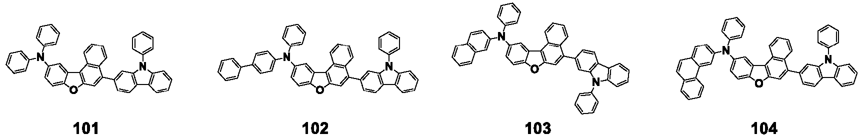

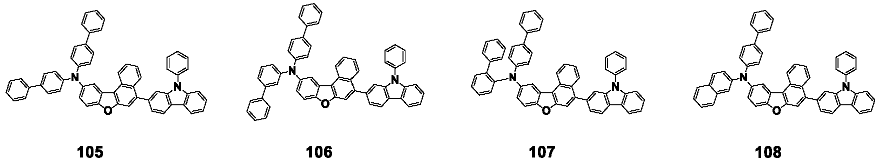

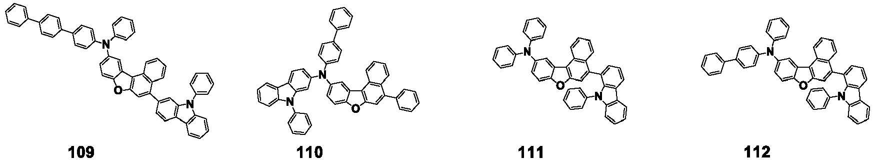

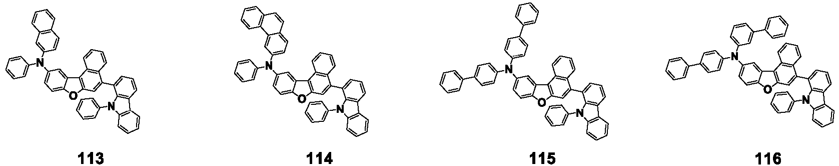

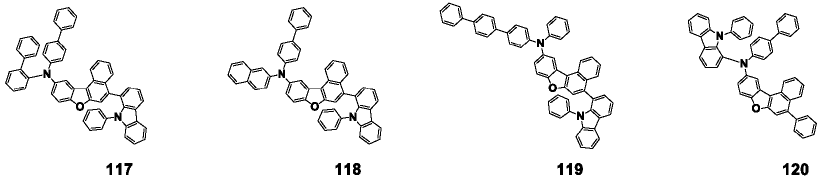

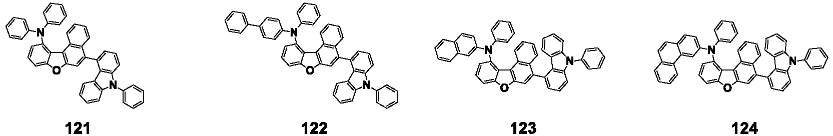

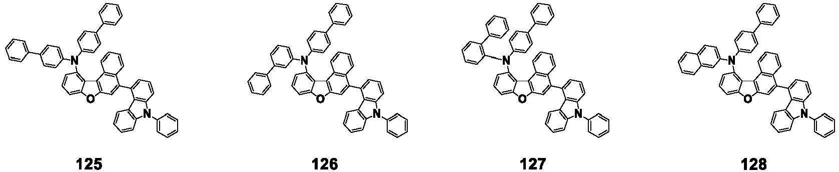

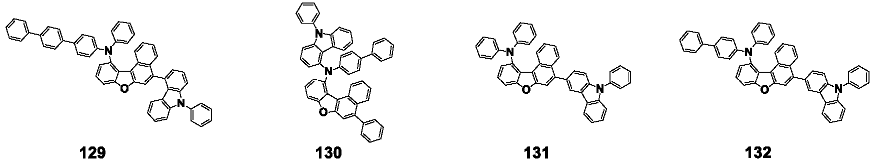

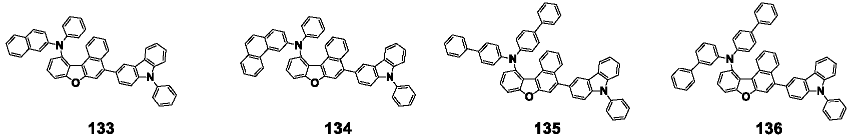

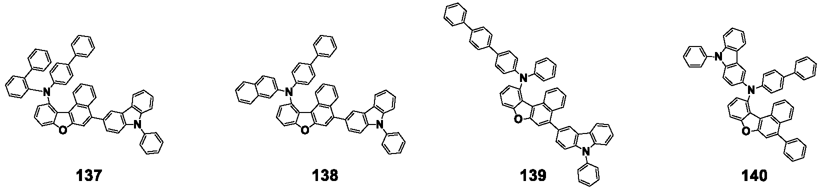

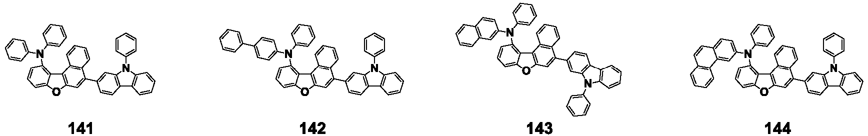

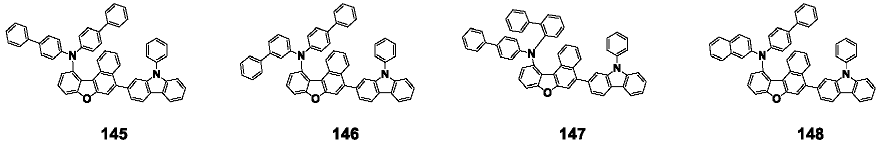

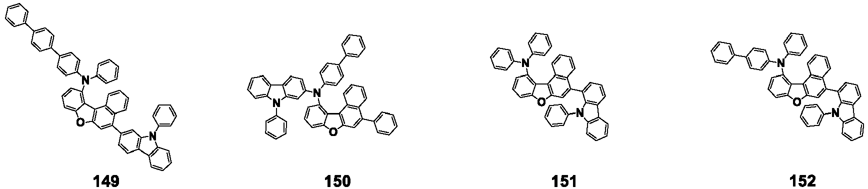

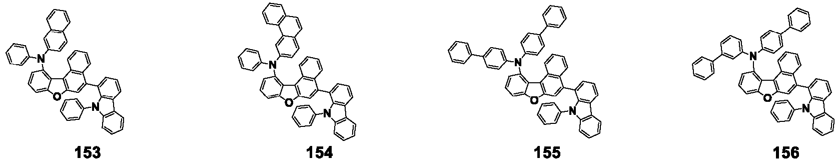

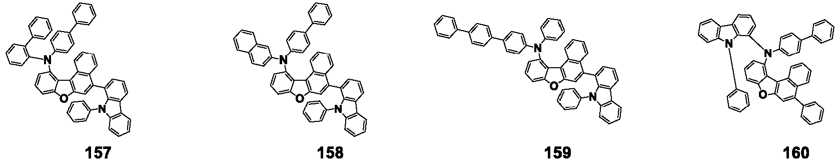

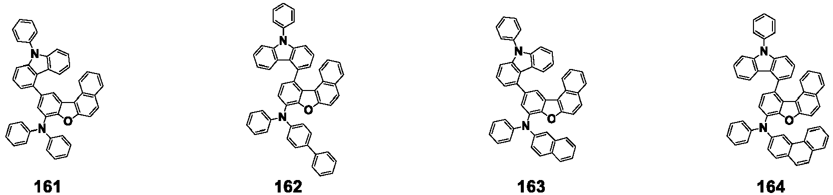

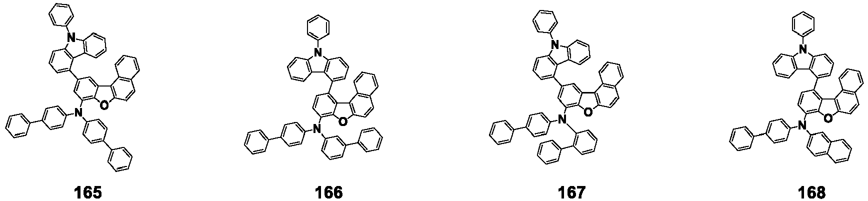

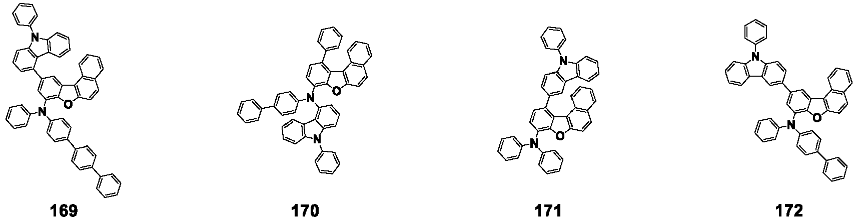

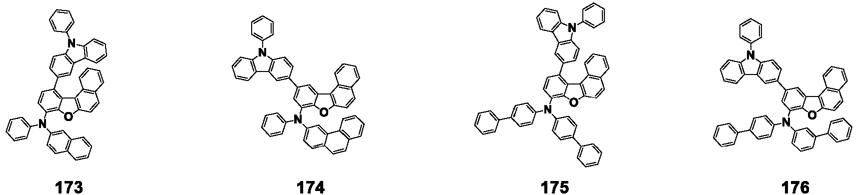

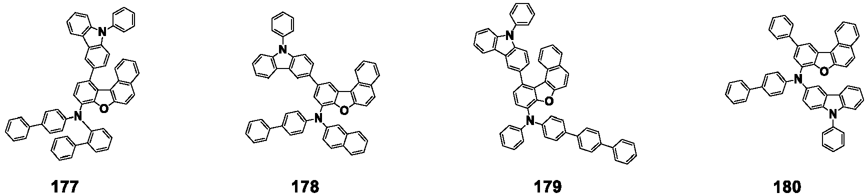

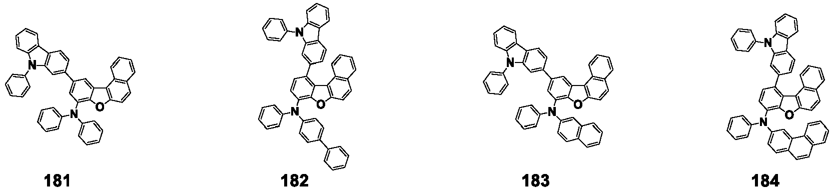

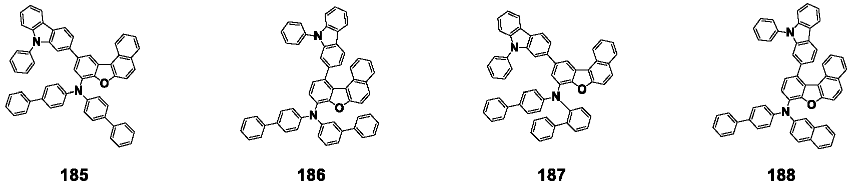

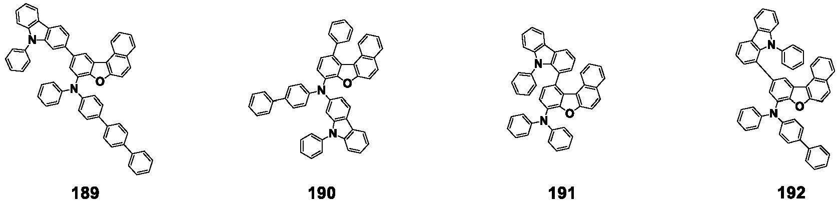

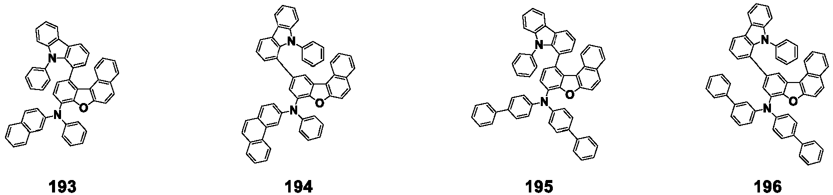

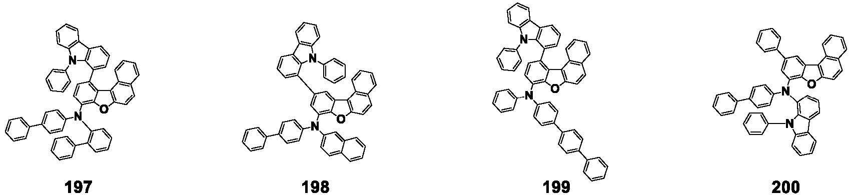

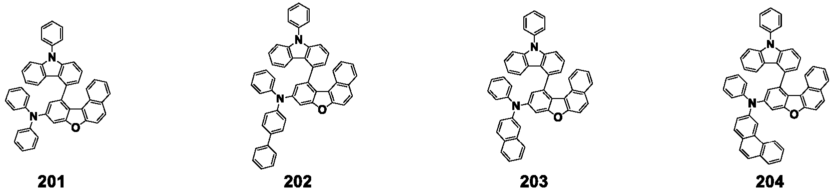

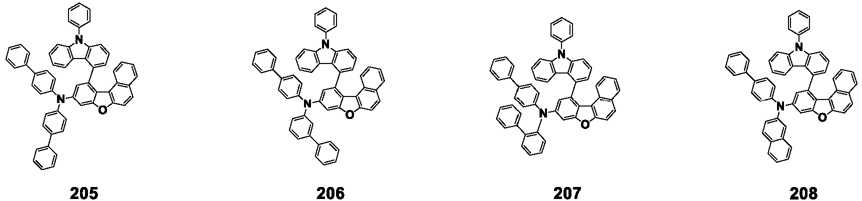

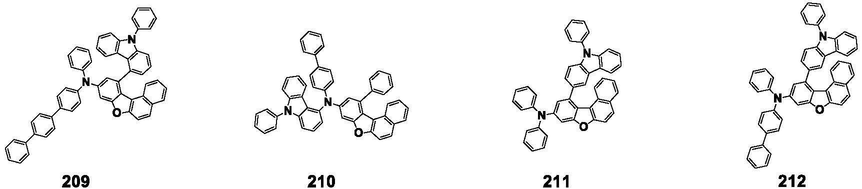

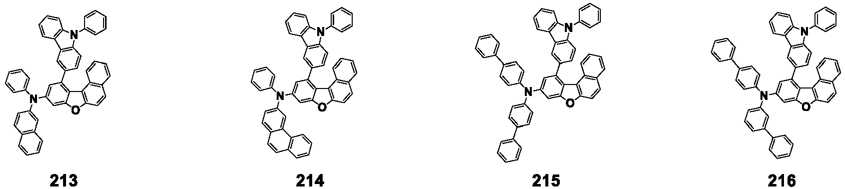

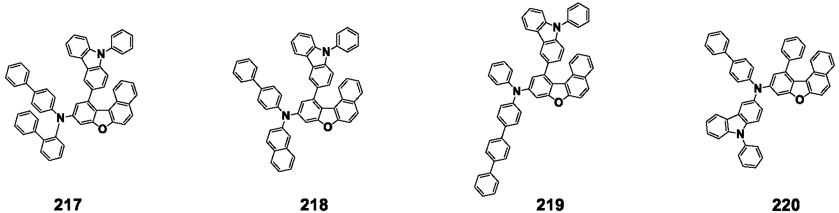

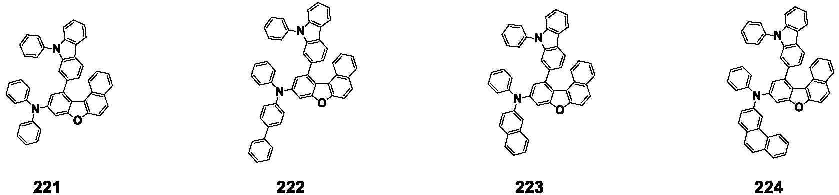

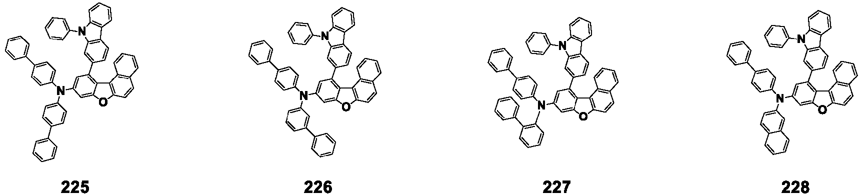

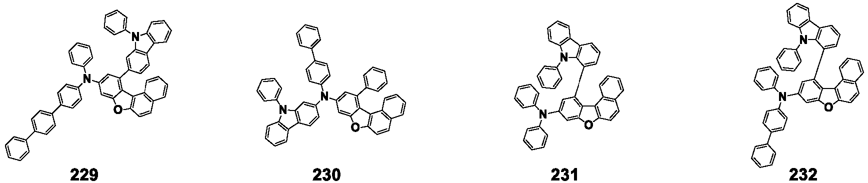

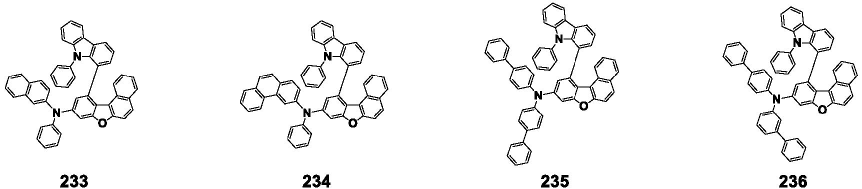

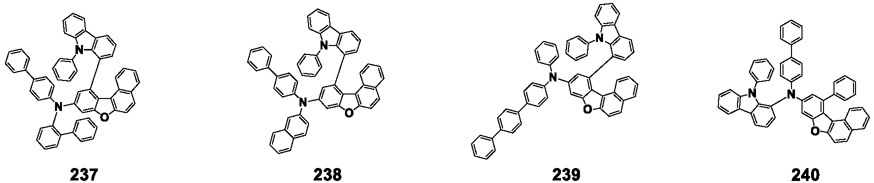

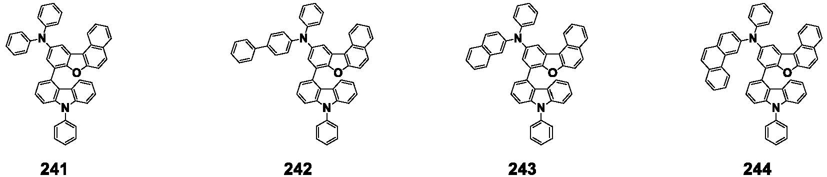

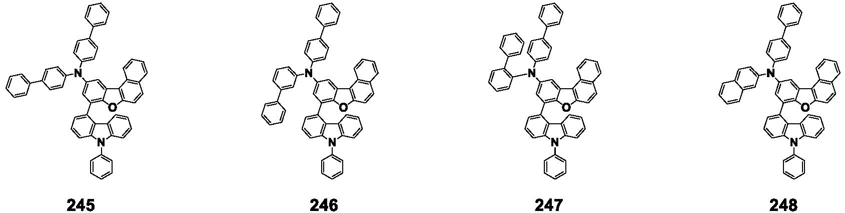

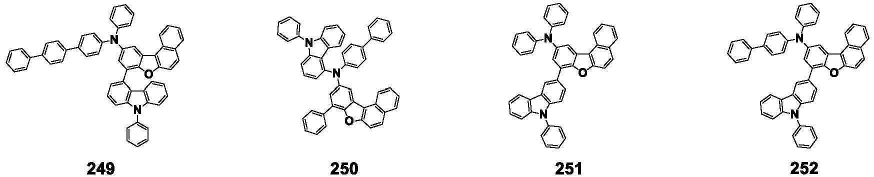

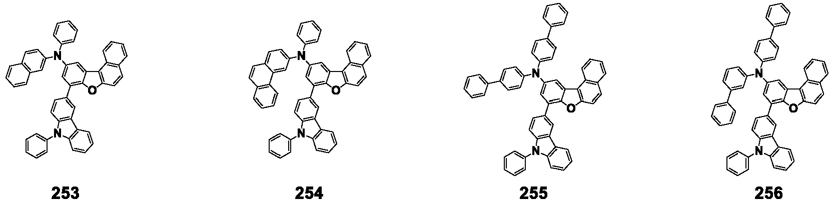

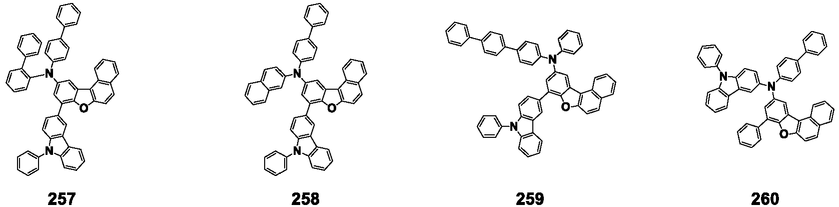

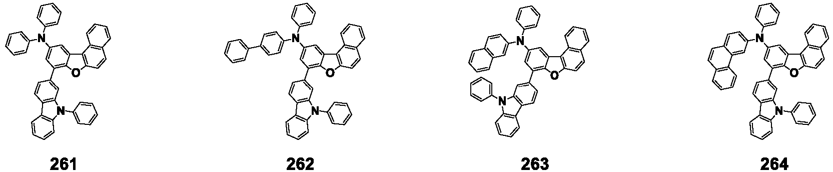

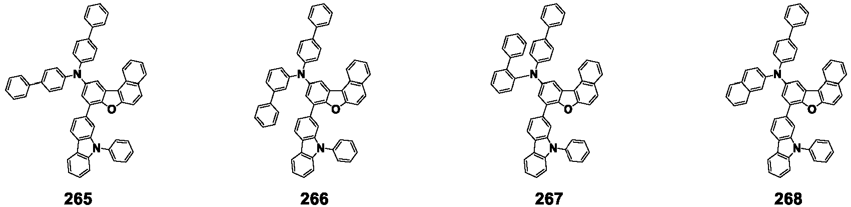

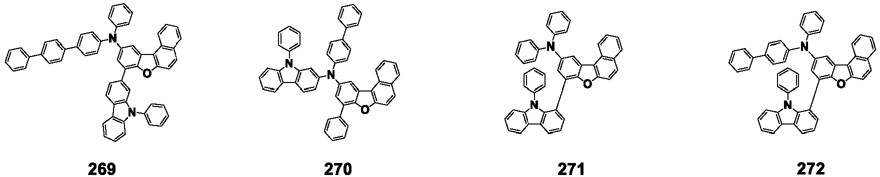

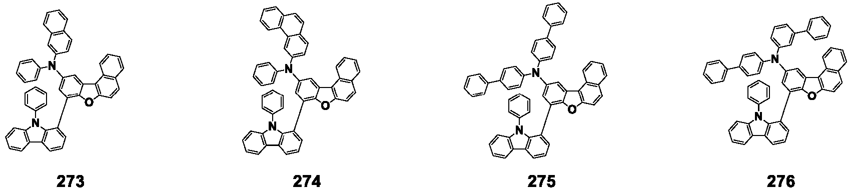

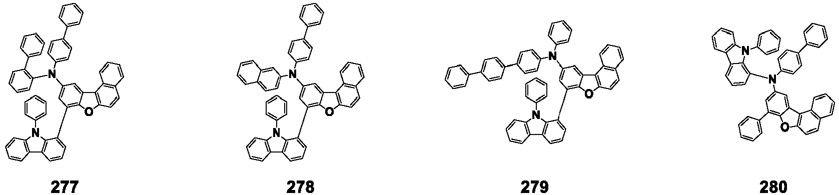

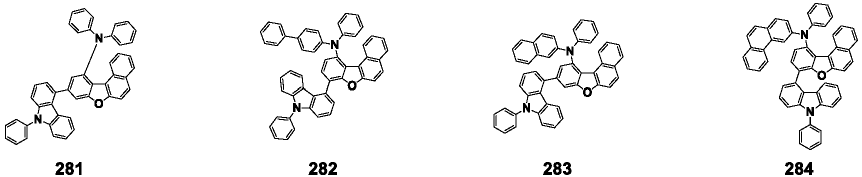

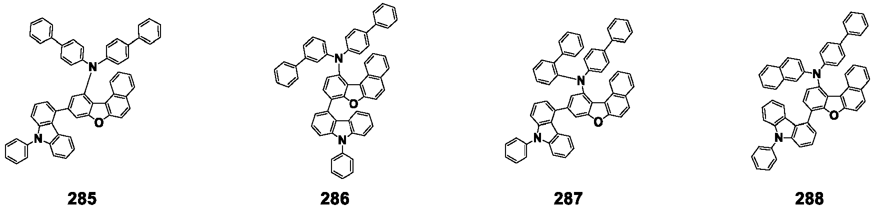

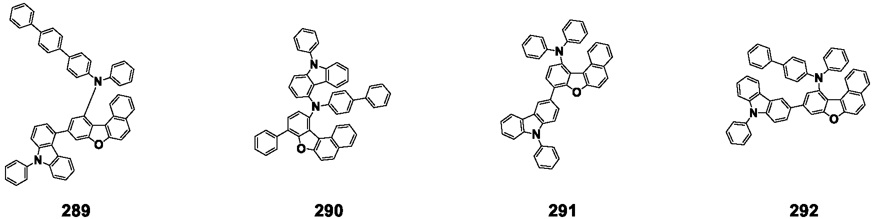

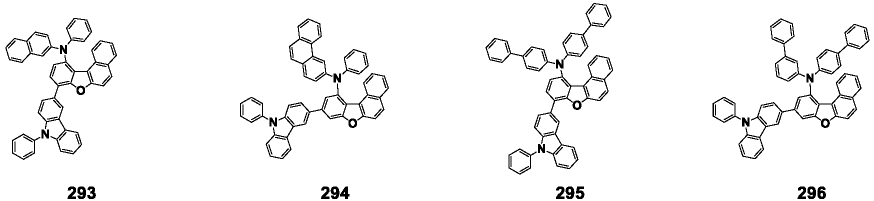

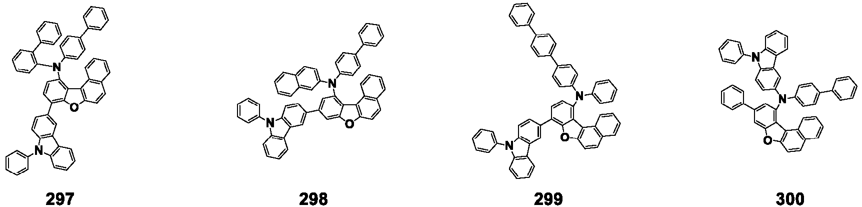

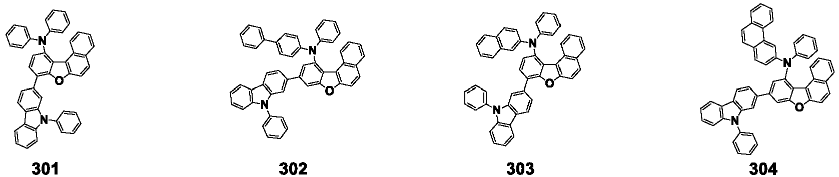

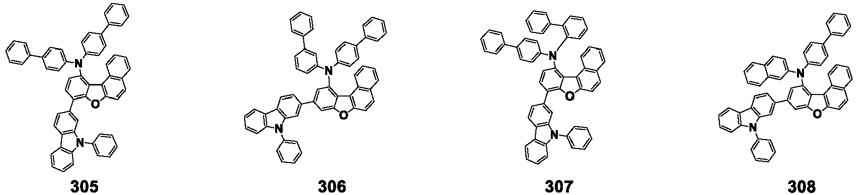

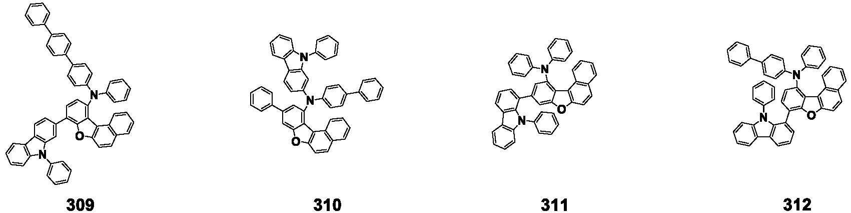

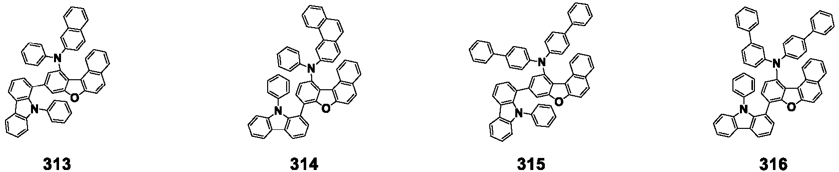

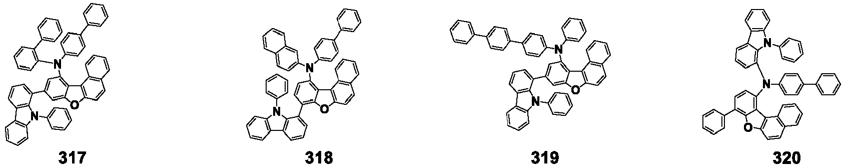

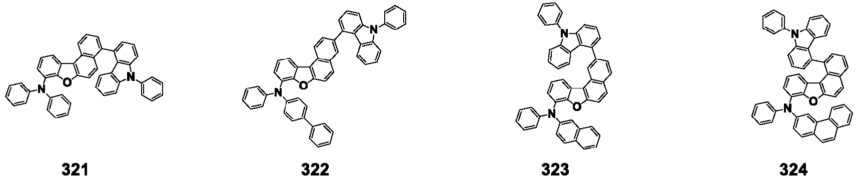

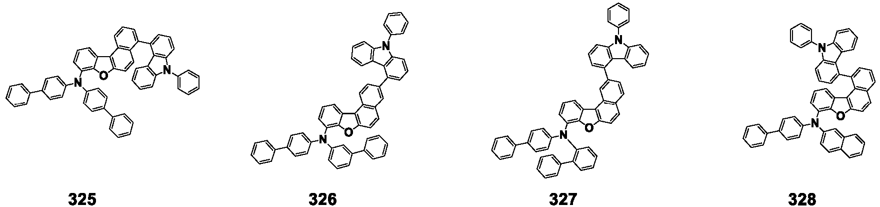

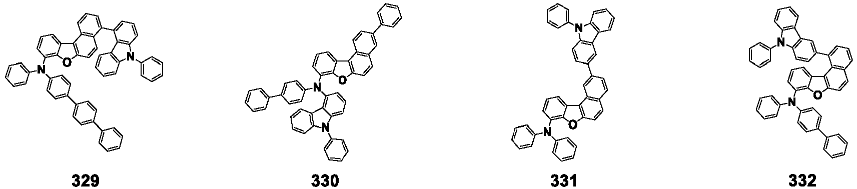

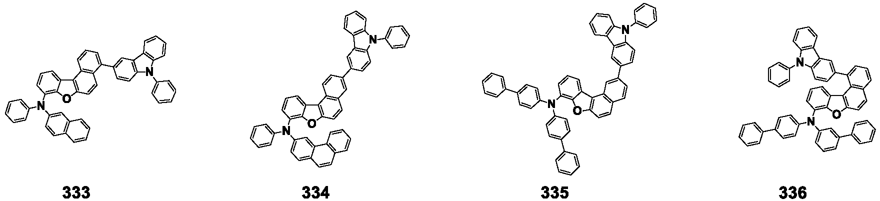

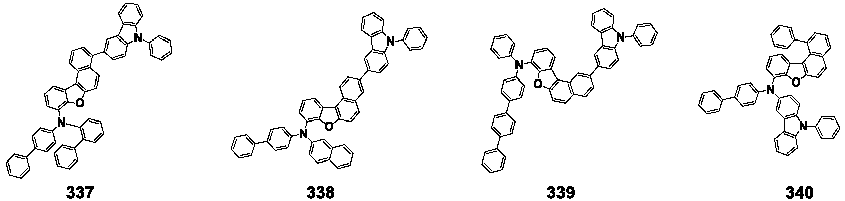

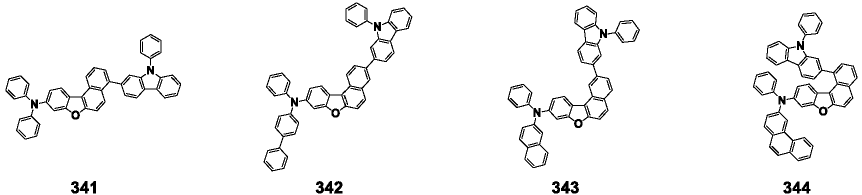

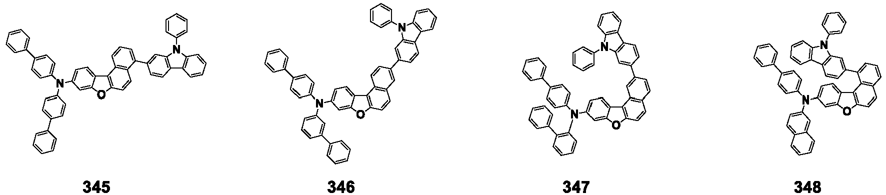

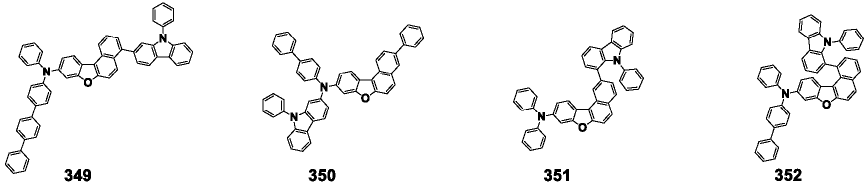

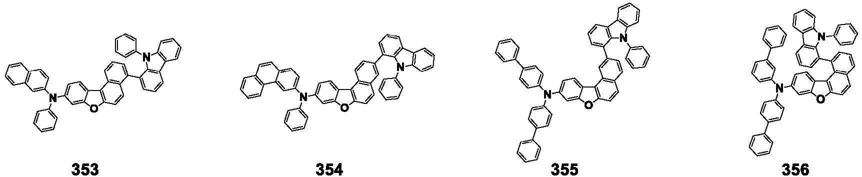

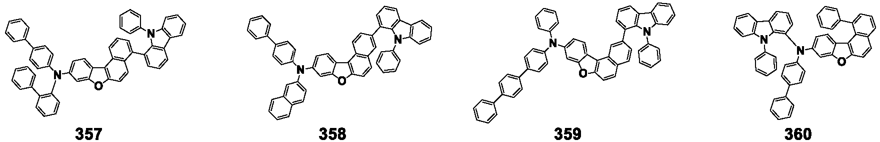

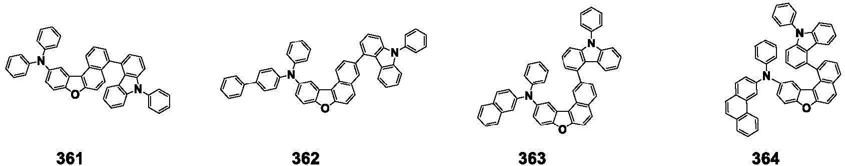

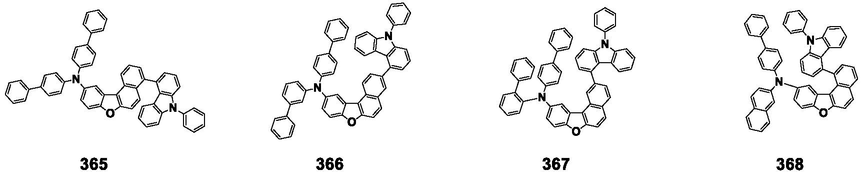

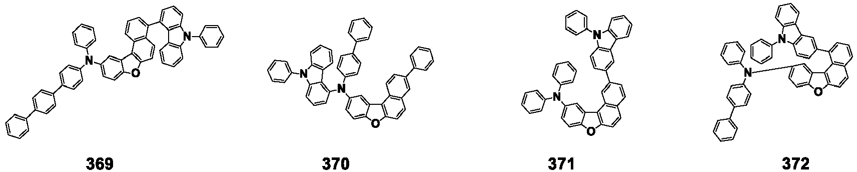

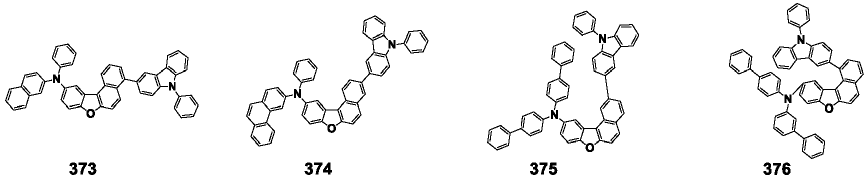

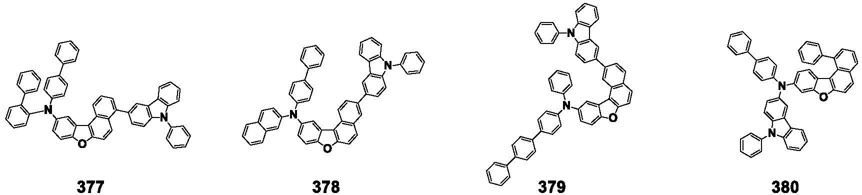

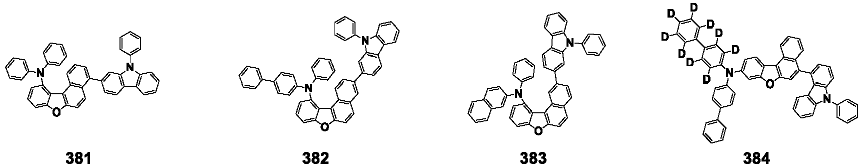

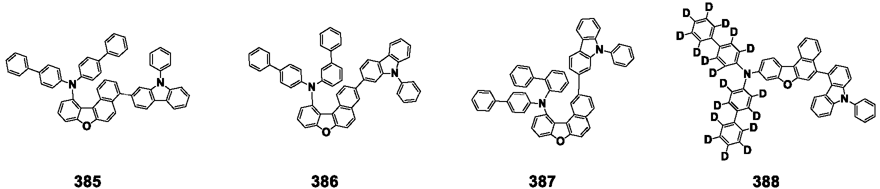

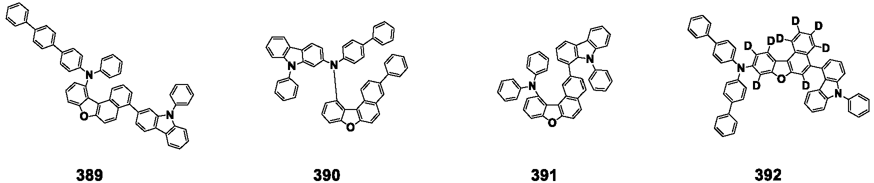

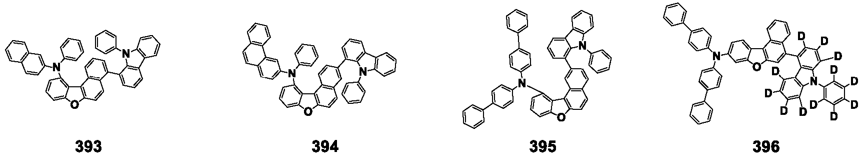

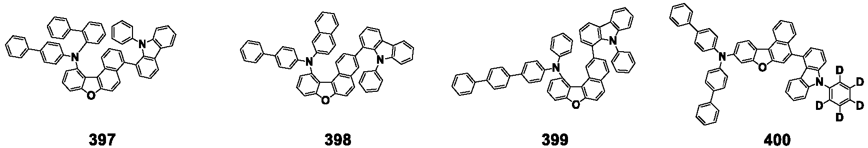

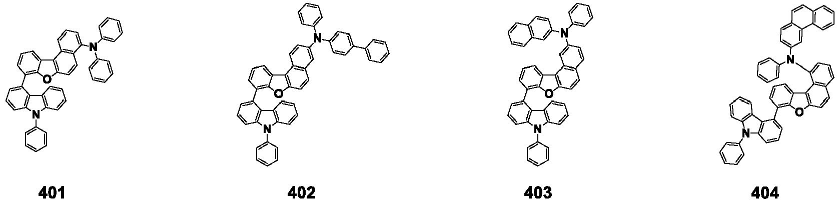

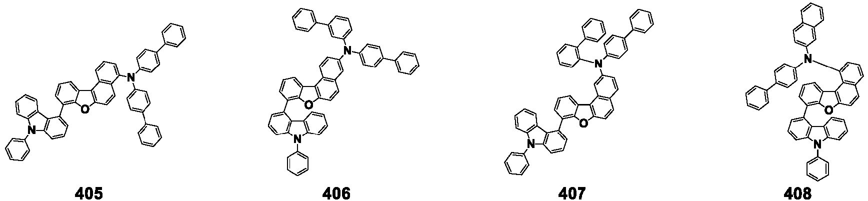

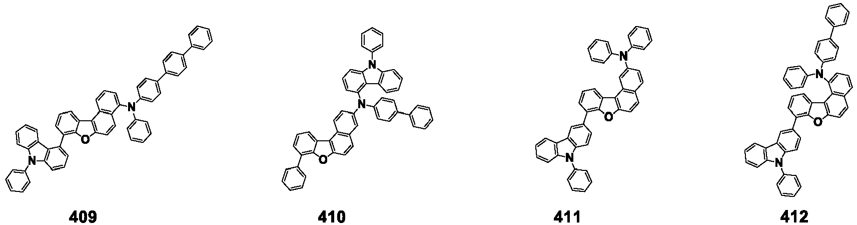

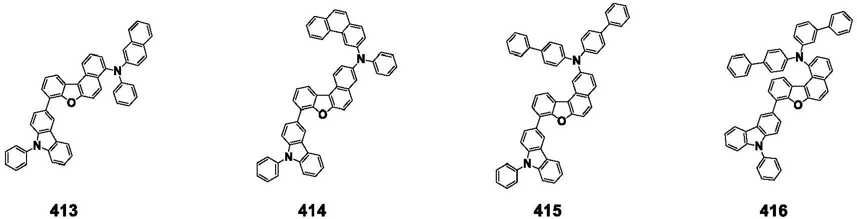

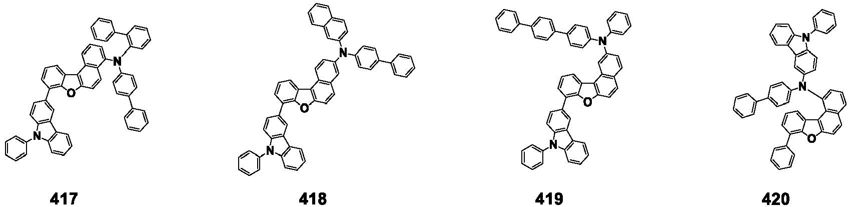

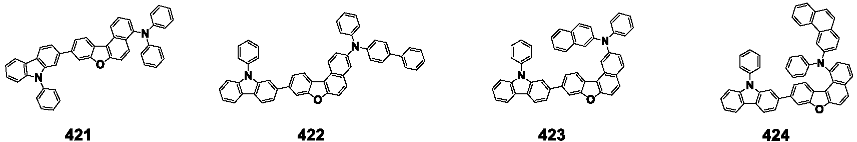

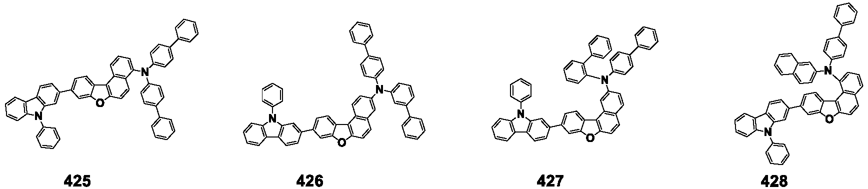

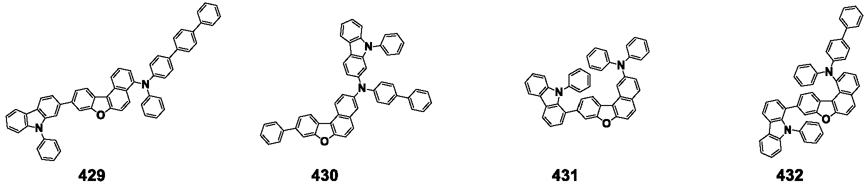

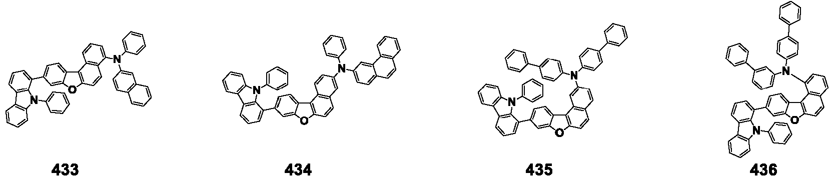

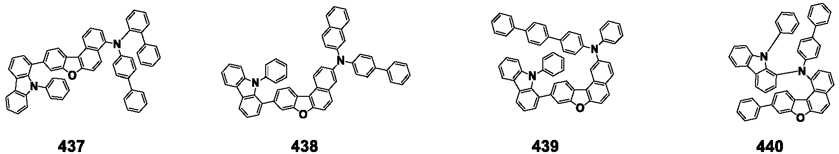

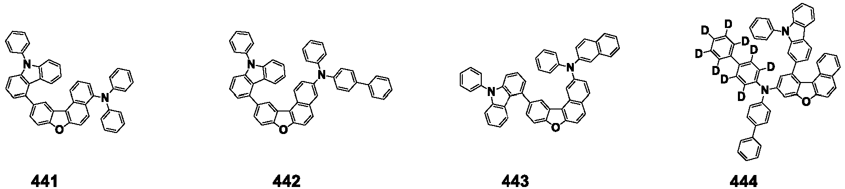

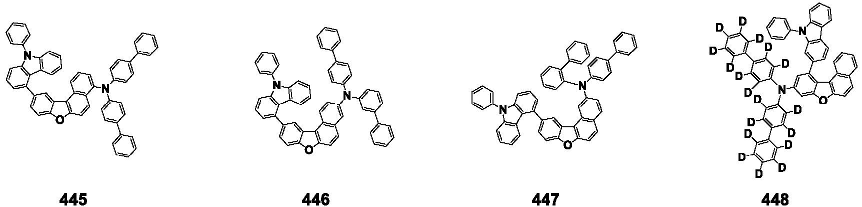

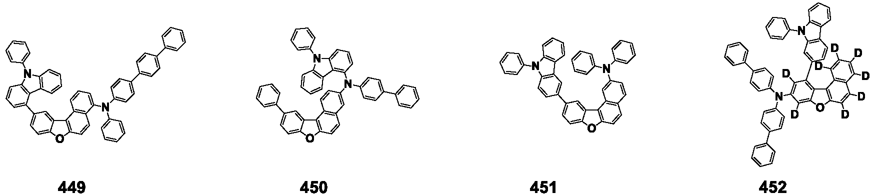

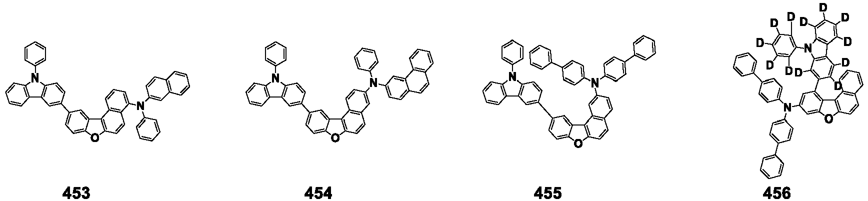

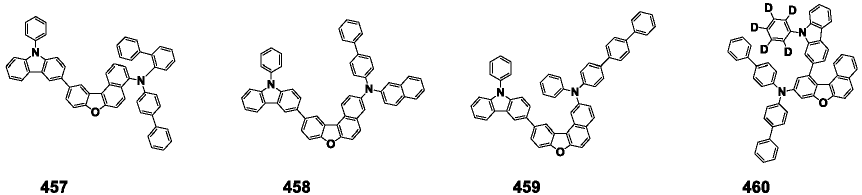

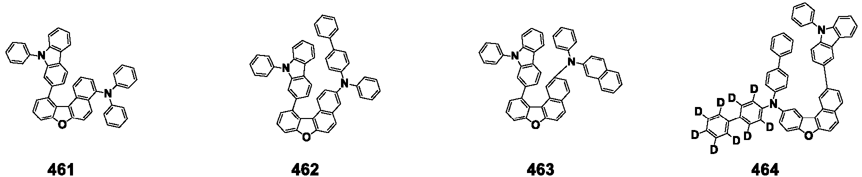

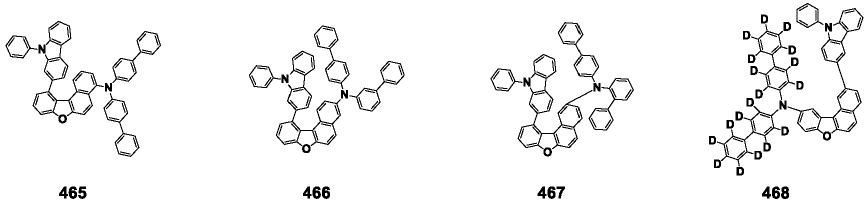

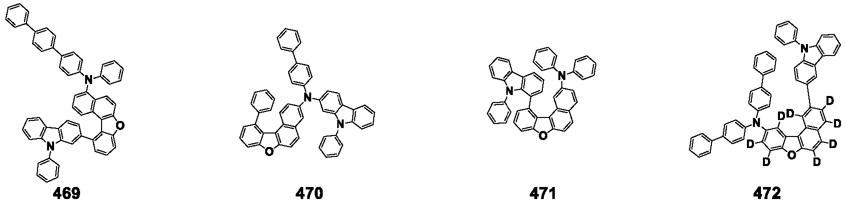

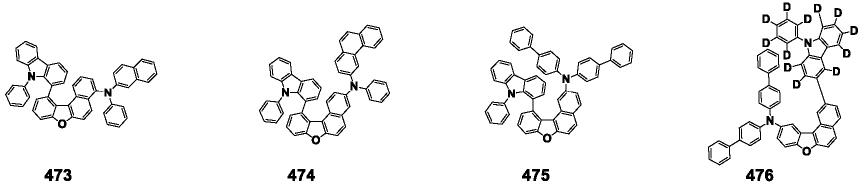

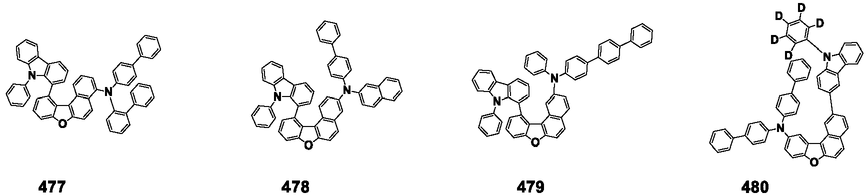

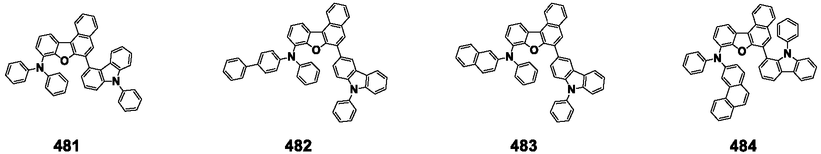

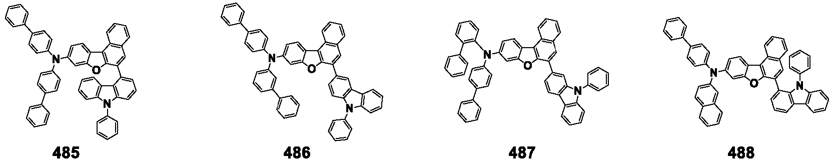

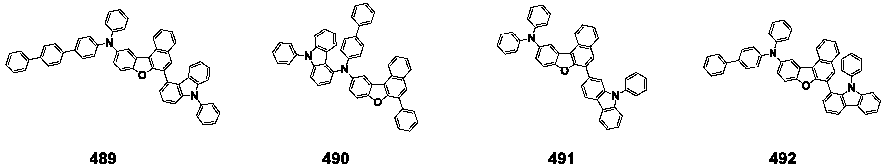

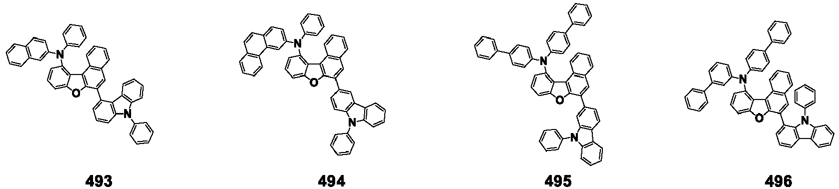

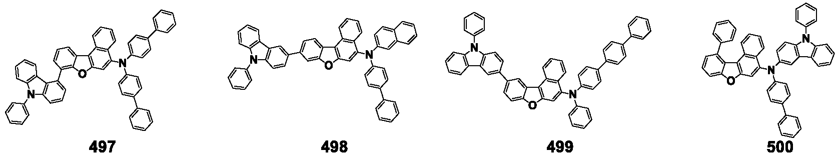

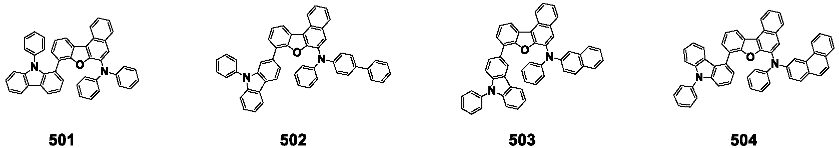

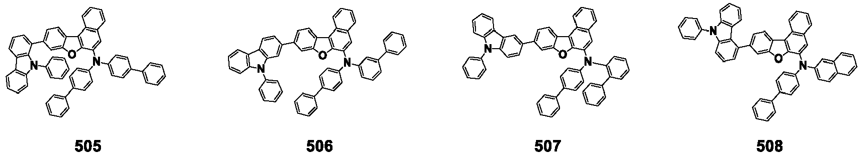

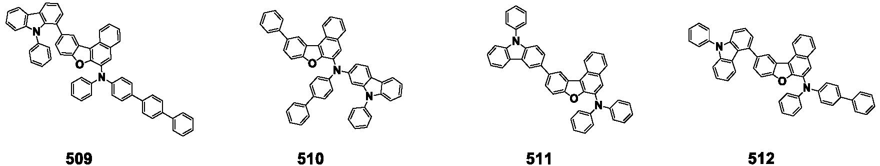

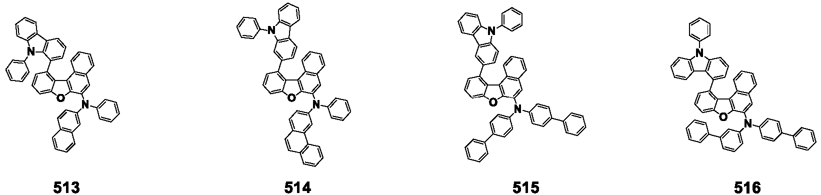

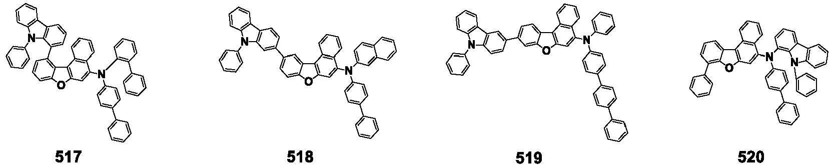

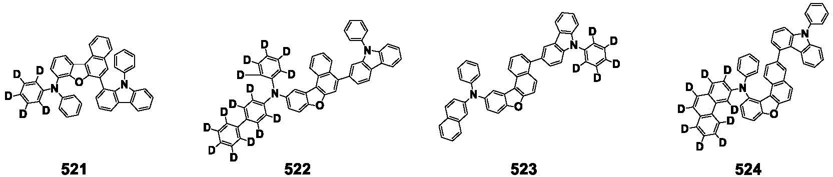

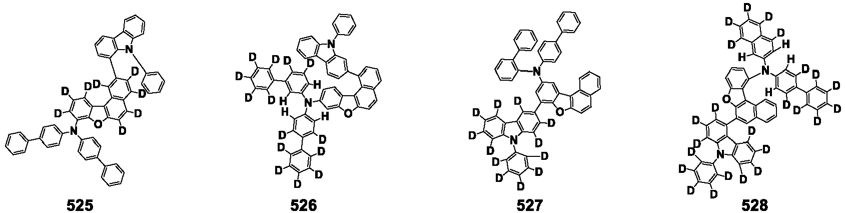

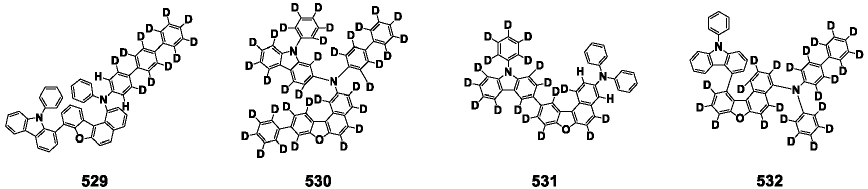

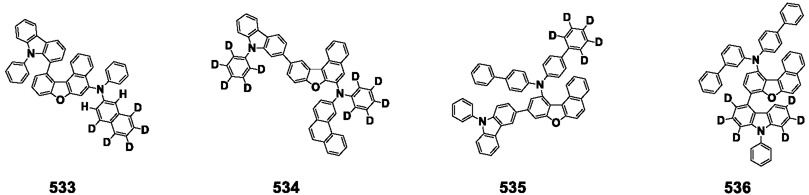

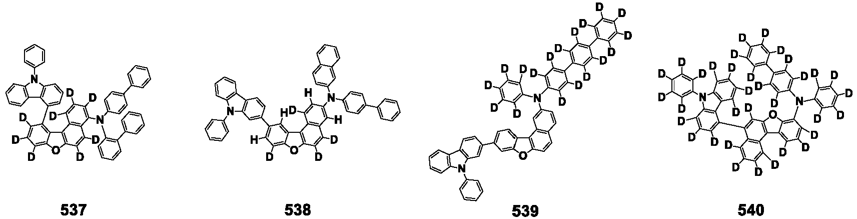

In addition, by introducing various substituents to the heterocyclic compound represented by Chemical Formula 1, compounds having unique properties of the introduced substituents may be synthesized. For example, by introducing substituents normally used for a hole injection layer material, a hole transport layer material, a light emitting layer material, an electron transport layer material, an electron blocking layer material and a charge generation layer material used for manufacturing an organic light emitting device to the core structure, materials satisfying conditions required for each organic material layer may be synthesized.

In addition, by introducing various substituents to the heterocyclic compound represented by Chemical Formula 1, the energy bandgap may be finely controlled, and meanwhile, properties at interfaces between organic materials may be enhanced, and material applications may become diverse.

Meanwhile, the heterocyclic compound has a high glass transition temperature (Tg), and thereby has excellent thermal stability. Such an increase in the thermal stability becomes an important factor providing driving stability to a device.

The heterocyclic compound according to one embodiment of the present disclosure may be prepared using a multi-step chemical reaction. Some intermediate compounds are prepared first, and from the intermediate compounds, the heterocyclic compound represented by Chemical Formula 1 may be prepared. More specifically, the heterocyclic compound according to one embodiment of the present disclosure may be prepared based on preparation examples to be described later.

Another embodiment of the present disclosure provides an organic light emitting device including the heterocyclic compound represented by Chemical Formula 1. The "organic light emitting device" may be expressed in terms such as an "organic light emitting diode", an "OLED", an "OLED device" and an "organic electroluminescent device".

In addition, one embodiment of the present disclosure relates to an organic light emitting device comprising:
a first electrode;
a second electrode provided opposite to the first electrode; and
one or more organic material layers provided between the first electrode and the second electrode,
wherein at least one of the one or more organic material layers comprise the heterocyclic compound represented by Chemical Formula 1.

In one embodiment of the present disclosure, the first electrode may be a positive electrode, and the second electrode may be a negative electrode.

In another embodiment, the first electrode may be a negative electrode, and the second electrode may be a positive electrode.

In one embodiment of the present disclosure, the organic light emitting device may be a red organic light emitting device, and the heterocyclic compound represented by Chemical Formula 1 may be used as a material of the red organic light emitting device.

In another embodiment of the present disclosure, the organic light emitting device may be a blue organic light emitting device, and the heterocyclic compound represented by Chemical Formula 1 may be used as a material of the blue organic light emitting device.

In another embodiment of the present disclosure, the organic light emitting device may be a green organic light emitting device, and the heterocyclic compound represented by Chemical Formula 1 may be used as a material of the green organic light emitting device.

In one embodiment of the present disclosure, the organic light emitting device may be a red organic light emitting device, and the heterocyclic compound represented by Chemical Formula 1 may be used as a light emitting layer material of the red organic light emitting device.

In another embodiment of the present disclosure, the organic light emitting device may be a blue organic light emitting device, and the heterocyclic compound represented by Chemical Formula 1 may be used as a light emitting layer material of the blue organic light emitting device.

In another embodiment of the present disclosure, the organic light emitting device may be a green organic light emitting device, and the heterocyclic compound represented by Chemical Formula 1 may be used as a light emitting layer material of the green organic light emitting device.

Specific descriptions on the heterocyclic compound represented by Chemical Formula 1 are the same as the descriptions provided above.

The organic light emitting device of the present disclosure may be manufactured using common organic light emitting device manufacturing methods and materials except that one or more organic material layers are formed using the heterocyclic compound described above.

The heterocyclic compound may be formed into the organic material layer using a solution coating method as well as a vacuum deposition method when the organic light emitting device is manufactured. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, but may also be formed in a multilayer structure in which two or more organic material layers are laminated. For example, the organic light emitting device of the present disclosure may have a structure including as a hole injection layer, an electron blocking layer, a hole transport layer, a light emitting layer, an electron transport layer, a hole blocking layer, an electron injection layer and the like the organic material layer. However, the structure of the organic light emitting device is not limited thereto, and may include a smaller number of organic material layers.

In the organic light emitting device of the present disclosure, the organic material layer includes a light emitting layer, and the light emitting layer may include the heterocyclic compound represented by Chemical Formula 1. When the heterocyclic compound is used in the light emitting layer, HOMO (Highest Occupied Molecular Orbital) and LUMO (Lowest Unoccupied Molecular Orbital) are spatially separated, enabling strong charge transfer, and therefore, driving efficiency and lifetime of the organic light emitting device may become superior.

One embodiment of the present disclosure provides an organic light emitting device, wherein the organic material layer including the heterocyclic compound represented by Chemical Formula 1 further includes a heterocyclic compound represented by the following Chemical Formula 4.

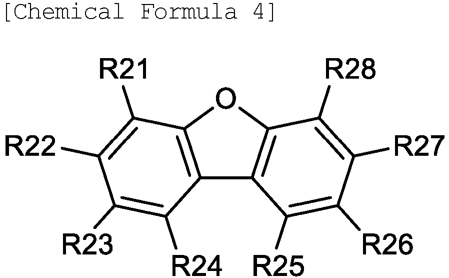

In Chemical Formula 4,
R21 to R28 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; -P(=O)R201R202; -SiR201R202R203; the following Chemical Formula 5; the following Chemical Formula 6; and the following Chemical Formula 7, or two or more groups adjacent to each other bond to each other to form a substituted or unsubstituted C6 to C60 aromatic hydrocarbon ring; or a substituted or unsubstituted C2 to C60 heteroring, and R201, R202 and R203 are the same as or different from each other and each independently a substituted or unsubstituted C1 to C60 alkyl group; a substituted or unsubstituted C6 to C60 aryl group; or a substituted or unsubstituted C2 to C60 heteroaryl group, and
any one of R21 to R28 is the following Chemical Formula 5, and another one is the following Chemical Formula 6; or the following Chemical Formula 7, 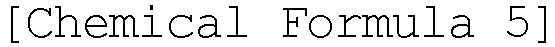 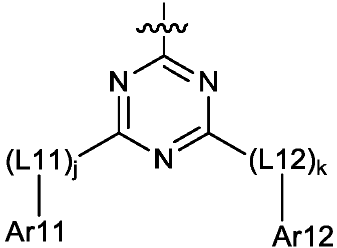 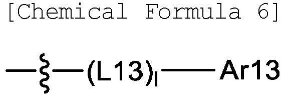 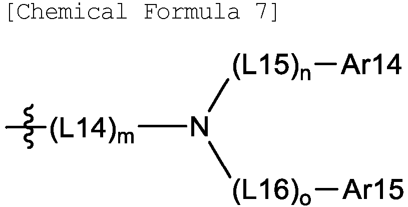
In Chemical Formulae 5 to 7,
L11 to L16 are the same as or different from each other, and each independently a direct bond; a substituted or unsubstituted C6 to C60 arylene group; or a substituted or unsubstituted C2 to C60 heteroarylene group,
j is an integer of 0 to 5, and when j is 2 or greater, L11s are the same as or different from each other,
k is an integer of 0 to 5, and when k is 2 or greater, L12s are the same as or different from each other,
1 is an integer of 0 to 5, and when l is 2 or greater, L13s are the same as or different from each other,
m is an integer of 0 to 5, and when m is 2 or greater, L14s are the same as or different from each other,
n is an integer of 0 to 5, and when n is 2 or greater, L15s are the same as or different from each other,
o is an integer of 0 to 5, and when o is 2 or greater, L16s are the same as or different from each other, and
Ar11 to Ar15 are the same as or different from each other, and each independently a substituted or unsubstituted C6 to C60 aryl group; or a substituted or unsubstituted C2 to C60 heteroaryl group.

In one embodiment of the present disclosure, R21 to R28 are the same as or different from each other, and each independently hydrogen; deuterium; halogen; a cyano group; a substituted or unsubstituted C1 to C30 alkyl group; a substituted or unsubstituted C2 to C30 alkenyl group; a substituted or unsubstituted C2 to C30 alkynyl group; a substituted or unsubstituted C1 to C30 alkoxy group; a substituted or unsubstituted C3 to C30 cycloalkyl group; a substituted or unsubstituted C2 to C30 heterocycloalkyl group; -P(=O)R201R202; -SiR201R202R203; Chemical Formula 5; Chemical Formula 6; or Chemical Formula 7, or two or more groups adjacent to each other bond to each other to form a substituted or unsubstituted C6 to C30 aromatic hydrocarbon ring; or a substituted or unsubstituted C2 to C30 heteroring, and R201, R202 and R203 are the same as or different from each other and may be each independently a substituted or unsubstituted C1 to C30 alkyl group; a substituted or unsubstituted C6 to C30 aryl group; or a substituted or unsubstituted C2 to C30 heteroaryl group, and any one of R21 to R28 is Chemical Formula 5 and another one may be Chemical Formula 6; or Chemical Formula 7.

In another embodiment of the present disclosure, R21 to R28 are the same as or different from each other, and each independently hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; -P(=O)R201R202; -SiR201R202R203; Chemical Formula 5; Chemical Formula 6; or Chemical Formula 7, or two or more groups adjacent to each other bond to each other to form a substituted or unsubstituted C6 to C20 aromatic hydrocarbon ring; or a substituted or unsubstituted C2 to C20 heteroring, and R201, R202 and R203 are the same as or different from each other and may be each independently a substituted or unsubstituted C1 to C20 alkyl group; a substituted or unsubstituted C6 to C20 aryl group; or a substituted or unsubstituted C2 to C20 heteroaryl group, and any one of R21 to R28 is Chemical Formula 5 and another one may be Chemical Formula 6; or Chemical Formula 7.

In another embodiment of the present disclosure, R21 to R28 are the same as or different from each other, and each independently hydrogen; deuterium; Chemical Formula 5; Chemical Formula 6; or Chemical Formula 7, or two or more groups adjacent to each other may bond to each other to form a substituted or unsubstituted C6 to C20 aromatic hydrocarbon ring; or a substituted or unsubstituted C2 to C20 heteroring, and any one of R21 to R28 is Chemical Formula 5 and another one may be Chemical Formula 6; or Chemical Formula 7.

In one embodiment of the present disclosure, Ar11 to Ar15 are the same as or different from each other, and may be each independently a substituted or unsubstituted C6 to C30 aryl group; or a substituted or unsubstituted C2 to C30 heteroaryl group.

In another embodiment of the present disclosure, Ar11 to Ar15 are the same as or different from each other, and may be each independently a substituted or unsubstituted C6 to C20 aryl group; or a substituted or unsubstituted C2 to C20 heteroaryl group.

In another embodiment of the present disclosure, Ar11 and Ar12 are the same as or different from each other, and may be each independently a substituted or unsubstituted phenyl group; a substituted or unsubstituted biphenyl group; a substituted or unsubstituted naphthyl group; a substituted or unsubstituted phenanthrenyl group; or a substituted or unsubstituted dibenzofuranyl group.

In another embodiment of the present disclosure, Ar13 may be a substituted or unsubstituted phenyl group; a substituted or unsubstituted biphenyl group; a substituted or unsubstituted naphthyl group; a substituted or unsubstituted dibenzofuranyl group; a substituted or unsubstituted dibenzothiophenyl group; or a substituted or unsubstituted benzocarbazole group.

In another embodiment of the present disclosure, Ar14 and Ar15 are the same as or different from each other, and may be each independently a substituted or unsubstituted phenyl group; or a substituted or unsubstituted biphenyl group.

In one embodiment of the present disclosure, L11 to L16 are the same as or different from each other, and may be each independently a direct bond; a substituted or unsubstituted C6 to C30 arylene group; or a substituted or unsubstituted C2 to C30 heteroarylene group.

In another embodiment of the present disclosure, L11 to L16 are the same as or different from each other, and may be each independently a direct bond; a substituted or unsubstituted C6 to C20 arylene group; or a substituted or unsubstituted C2 to C20 heteroarylene group.

In another embodiment of the present disclosure, L11 and L12 are the same as or different from each other, and may be each independently a direct bond; or a substituted or unsubstituted phenylene group; or a substituted or unsubstituted naphthylene group.

In another embodiment of the present disclosure, L13 may be a direct bond; a substituted or unsubstituted phenylene group; a substituted or unsubstituted naphthylene group; or a substituted or unsubstituted benzocarbazolene group.

In another embodiment of the present disclosure, L14 to L16 are the same as or different from each other, and may be each independently a direct bond; or a substituted or unsubstituted phenylene group.

In one embodiment of the present disclosure, the heterocyclic compound represented by Chemical Formula 4 may be represented by any one of the following Chemical Formulae 4-1 to 4-4.

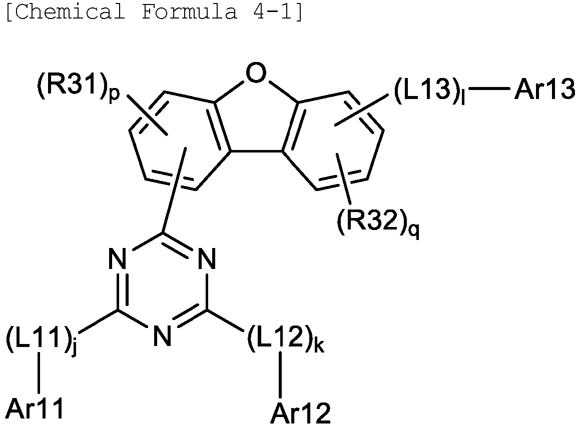

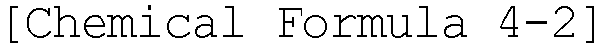

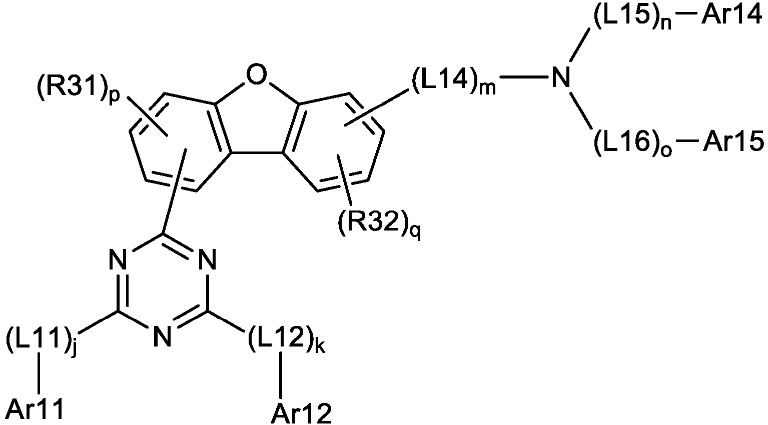

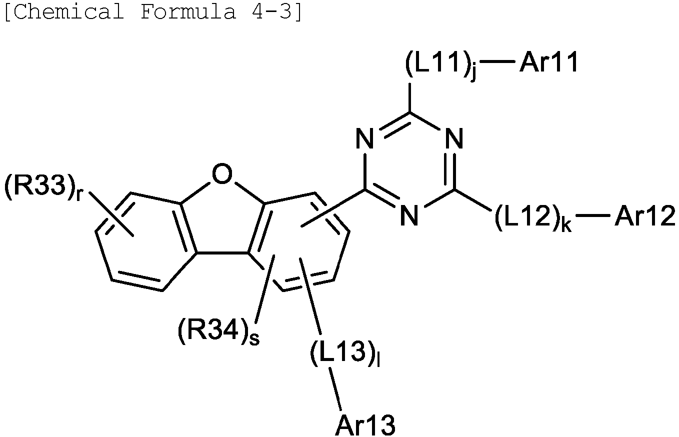

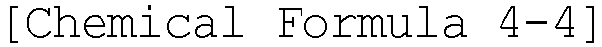

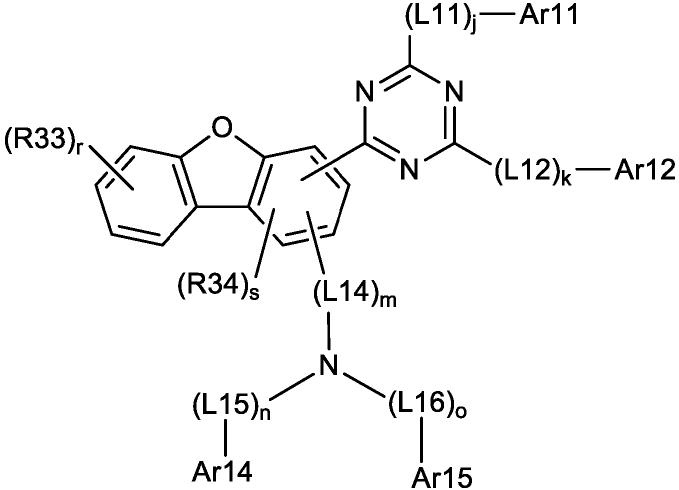

In Chemical Formulae 4-1 to 4-4,
R31 to R34 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R201R202; -SiR201R202R203; and -NR201R202, or two or more groups adjacent to each other bond to each other to form a substituted or unsubstituted C6 to C60 aromatic hydrocarbon ring; or a substituted or unsubstituted C2 to C60 heteroring, and R201, R202 and R203 are the same as or different from each other and each independently a substituted or unsubstituted C1 to C60 alkyl group; a substituted or unsubstituted C6 to C60 aryl group; or a substituted or unsubstituted C2 to C60 heteroaryl group,
p is an integer of 0 to 3, and when p is 2 or greater, R31s are the same as or different from each other,
q is an integer of 0 to 3, and when q is 2 or greater, R32s are the same as or different from each other,
r is an integer of 0 to 4, and when r is 2 or greater, R33s are the same as or different from each other,
s is an integer of 0 to 2, and when s is 2 or greater, R34s are the same as or different from each other,
L11, L12, Ar11, Ar12, j and k have the same definitions as in Chemical Formula 5,
L13, Ar13 and l have the same definitions as in Chemical Formula 6, and
L14 to L16, Ar14, Ar15, m, n and o have the same definitions as in Chemical Formula 7.

In one embodiment of the present disclosure, R31 to R34 are the same as or different from each other, and each independently hydrogen; deuterium; halogen; a cyano group; a substituted or unsubstituted C1 to C30 alkyl group; a substituted or unsubstituted C2 to C30 alkenyl group; a substituted or unsubstituted C2 to C30 alkynyl group; a substituted or unsubstituted C1 to C30 alkoxy group; a substituted or unsubstituted C3 to C30 cycloalkyl group; a substituted or unsubstituted C2 to C30 heterocycloalkyl group; a substituted or unsubstituted C6 to C30 aryl group; a substituted or unsubstituted C2 to C30 heteroaryl group; -P(=O)R201R202; -SiR201R202R203; or -NR201R202, or two or more groups adjacent to each other bond to each other to form a substituted or unsubstituted C6 to C30 aromatic hydrocarbon ring; or a substituted or unsubstituted C2 to C30 heteroring, and R201, R202 and R203 are the same as or different from each other and may be each independently a substituted or unsubstituted C1 to C30 alkyl group; a substituted or unsubstituted C6 to C30 aryl group; or a substituted or unsubstituted C2 to C30 heteroaryl group.

In another embodiment of the present disclosure, R21 to R28 are the same as or different from each other, and each independently hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; -P(=O)R201R202; -SiR201R202R203; or -NR201R202, or two or more groups adjacent to each other bond to each other to form a substituted or unsubstituted C6 to C20 aromatic hydrocarbon ring; or a substituted or unsubstituted C2 to C20 heteroring, and R201, R202 and R203 are the same as or different from each other and may be each independently a substituted or unsubstituted C1 to C20 alkyl group; a substituted or unsubstituted C6 to C20 aryl group; or a substituted or unsubstituted C2 to C20 heteroaryl group.

In another embodiment of the present disclosure, R21 to R28 are the same as or different from each other, and each independently hydrogen; or deuterium, or two or more groups adjacent to each other may bond to each other to form a substituted or unsubstituted C6 to C20 aromatic hydrocarbon ring; or a substituted or unsubstituted C2 to C20 heteroring.

In one embodiment of the present disclosure, R21 to R28, R31 to R34, L11 to L16 and Ar11 to Ar15 may all include hydrogen (H) that is not deuterated.

In another embodiment of the present disclosure, at least one of R21 to R28, R31 to R34, L11 to L16 and Ar11 to Ar15 includes deuterium (D), and at least one of R21 to R28, R31 to R34, L11 to L16 and Ar11 to Ar15 may include hydrogen that is not deuterated.

In another embodiment of the present disclosure, R21 to R28, R31 to R34, L11 to L16 and Ar11 to Ar15 may all include deuterium.

In one embodiment of the present disclosure, the heterocyclic compound represented by Chemical Formula 4 may not include deuterium as a substituent, or may have a deuterium content of, for example, greater than 0%, 1% or greater, 10% or greater, 20% or greater, 30% or greater, 40% or greater or 50% or greater, and 100% or less, 90% or less, 80% or less, 70% or less or 60% or less with respect to the total number of hydrogen atoms and deuterium atoms.

In another embodiment of the present disclosure, the heterocyclic compound represented by Chemical Formula 4 may not include deuterium as a substituent, or may have a deuterium content of 1% to 100% with respect to the total number of hydrogen atoms and deuterium atoms.

In another embodiment of the present disclosure, the heterocyclic compound represented by Chemical Formula 4 may not include deuterium as a substituent, or may have a deuterium content of 20% to 90% with respect to the total number of hydrogen atoms and deuterium atoms.

In another embodiment of the present disclosure, the heterocyclic compound represented by Chemical Formula 4 may not include deuterium as a substituent, or may have a deuterium content of 30% to 80% with respect to the total number of hydrogen atoms and deuterium atoms.

In another embodiment of the present disclosure, the heterocyclic compound represented by Chemical Formula 4 may not include deuterium as a substituent, or may have a deuterium content of 50% to 70% with respect to the total number of hydrogen atoms and deuterium atoms.

When the heterocyclic compound represented by Chemical Formula 1 and the heterocyclic compound represented by Chemical Formula 4 are included at the same time, effects of more superior efficiency and lifetime are obtained. From this, it may be expected that an exciplex phenomenon occurs when the two compounds are included at the same time.

The exciplex phenomenon is a phenomenon of releasing energy having sizes of a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transport ability and an acceptor (n-host) having a favorable electron transport ability are used as a host of a light emitting layer, holes are injected to the p-host and electrons are injected to the n-host, and thus a driving voltage may be lowered, which resultantly helps with enhancement in the lifetime. In other words, when the compound represented by Chemical Formula 1 is used as the donor and the compound represented by Chemical Formula 4 is used as the acceptor, excellent device properties are obtained.

In one embodiment of the present disclosure, when the heterocyclic compound represented by Chemical Formula 1 and the heterocyclic compound represented by Chemical Formula 4 are included at the same time, at least one of the compounds may not include deuterium as a substituent, or may have a deuterium content of greater than 0%, 1% or greater, 10% or greater, 20% or greater, 30% or greater, 40% or greater or 50% or greater, and 100% or less, 90% or less, 80% or less, 70% or less or 60% or less with respect to the total number of hydrogen atoms and deuterium atoms.

In another embodiment of the present disclosure, at least one of the compounds may not include deuterium as a substituent, or may have a deuterium content of 1% to 100% with respect to the total number of hydrogen atoms and deuterium atoms.

In another embodiment of the present disclosure, at least one of the compounds may not include deuterium as a substituent, or may have a deuterium content of 20% to 90% with respect to the total number of hydrogen atoms and deuterium atoms.

In another embodiment of the present disclosure, at least one of the compounds may not include deuterium as a substituent, or may have a deuterium content of 30% to 80% with respect to the total number of hydrogen atoms and deuterium atoms.

In another embodiment of the present disclosure, at least one of the compounds may not include deuterium as a substituent, or may have a deuterium content of 50% to 70% with respect to the total number of hydrogen atoms and deuterium atoms.

In one embodiment of the present disclosure, the heterocyclic compound represented by Chemical Formula 4 may be represented by any one of the following compounds.

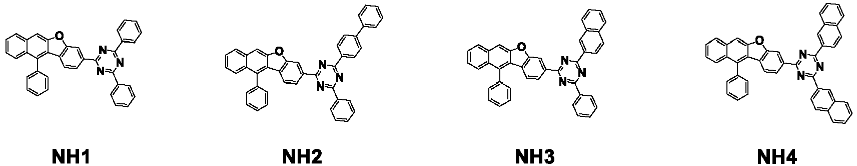

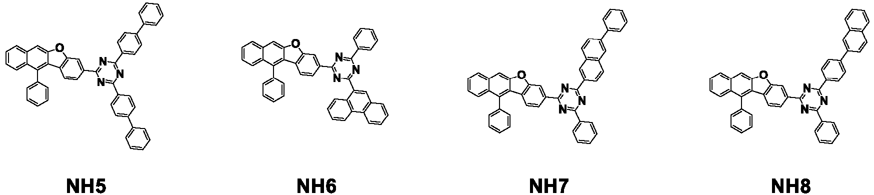

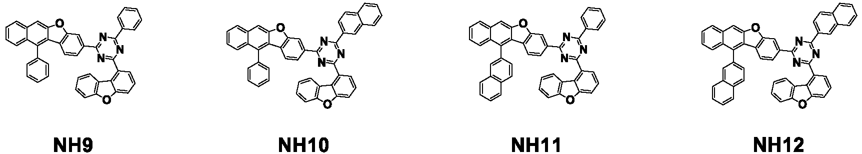

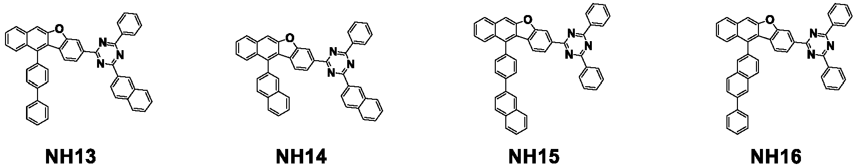

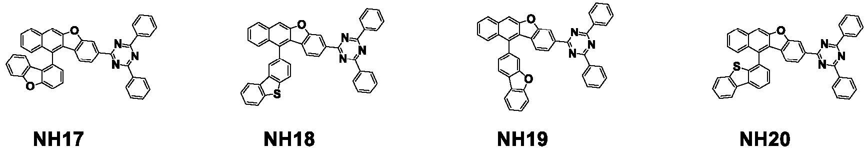

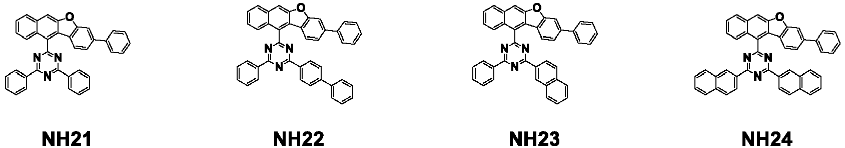

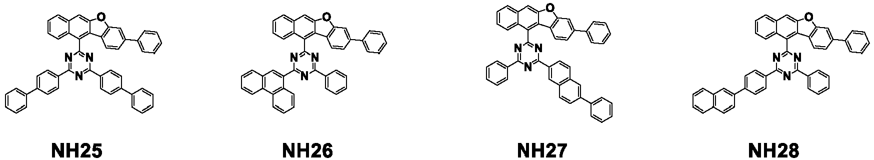

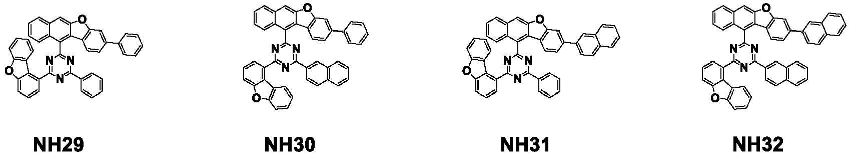

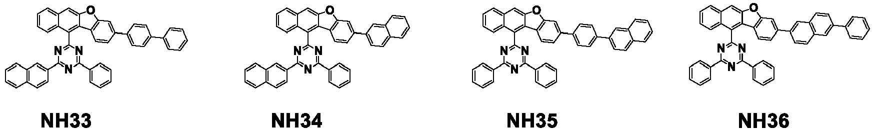

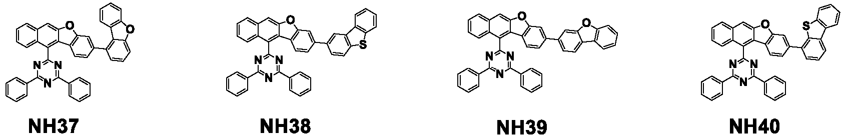

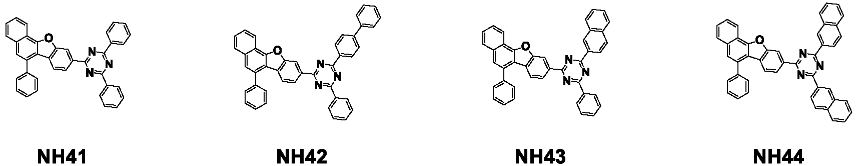

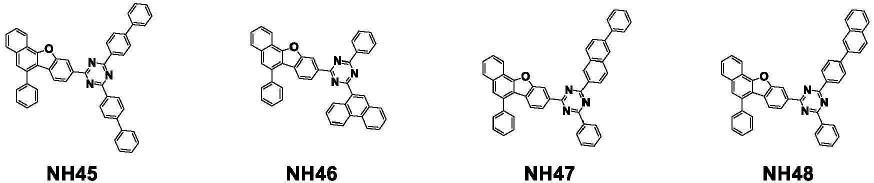

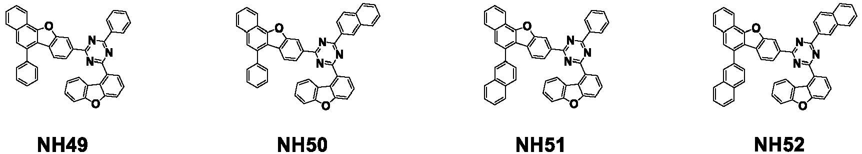

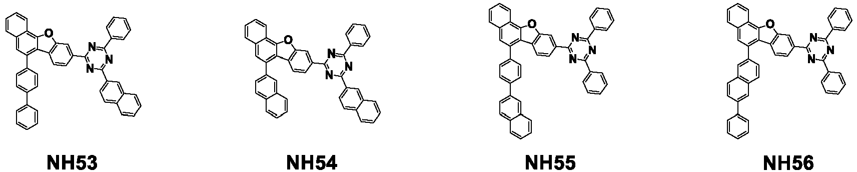

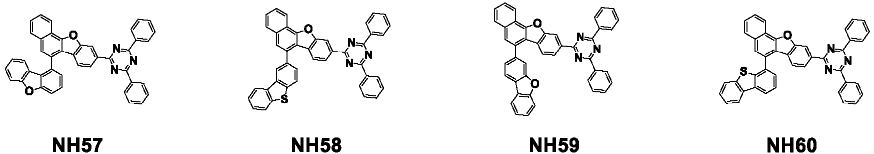

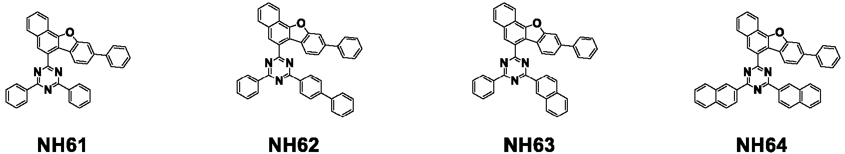

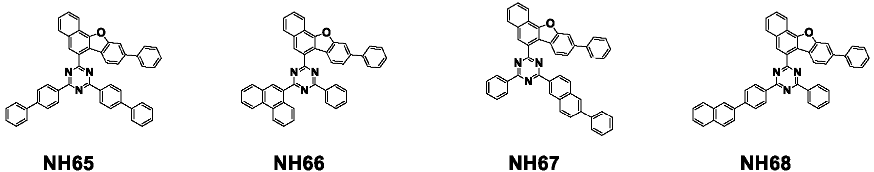

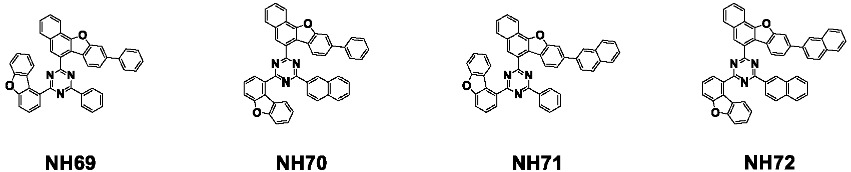

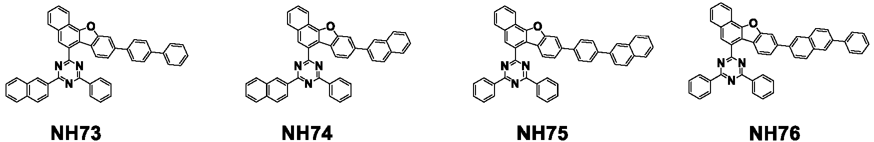

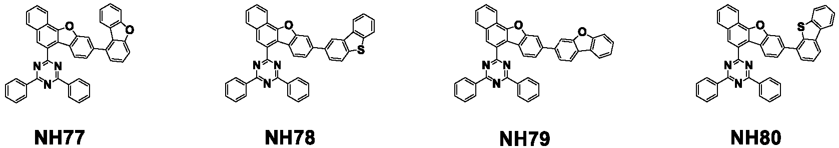

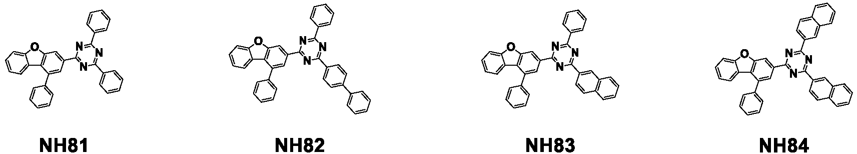

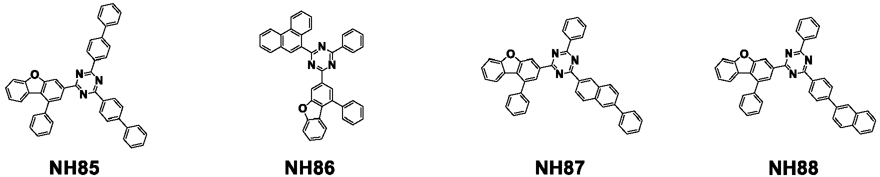

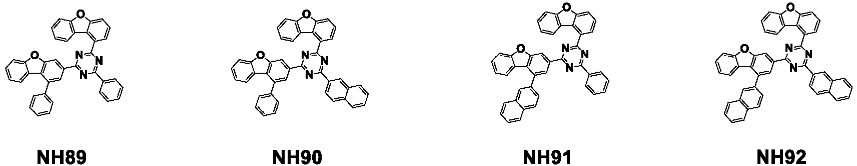

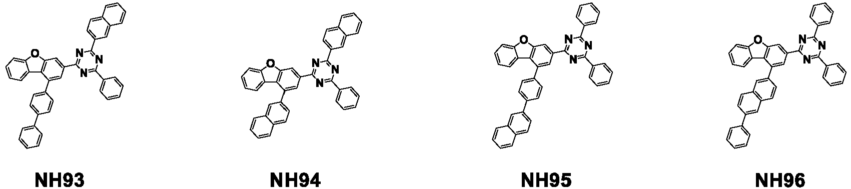

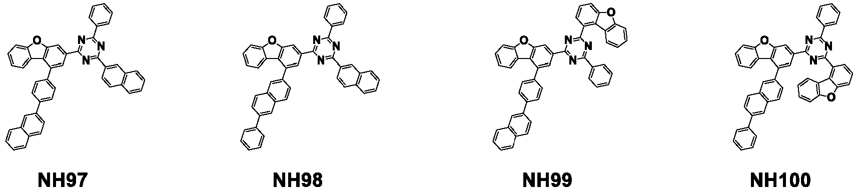

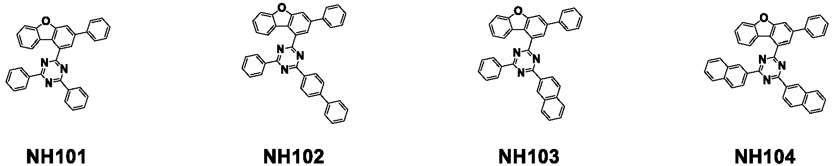

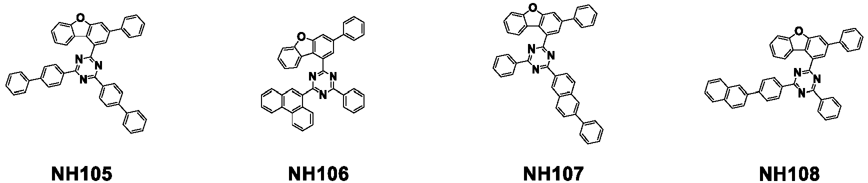

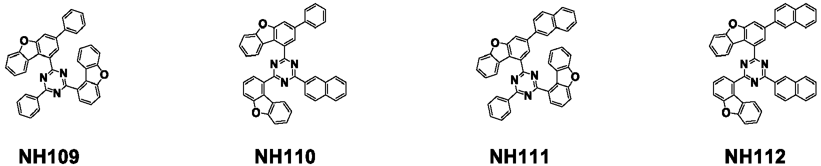

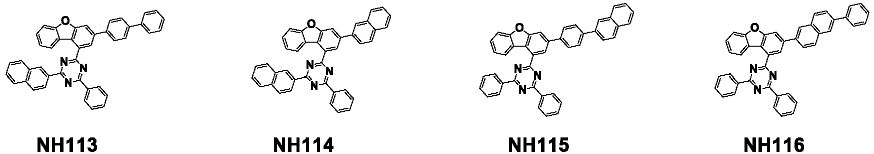

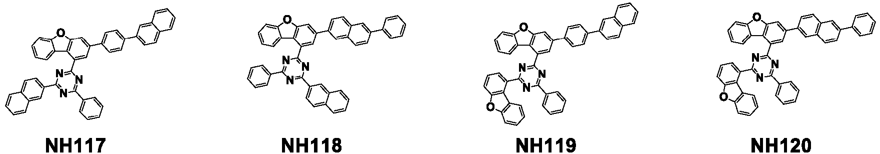

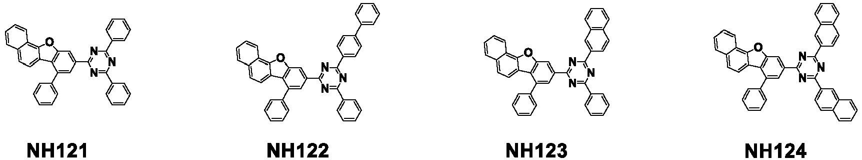

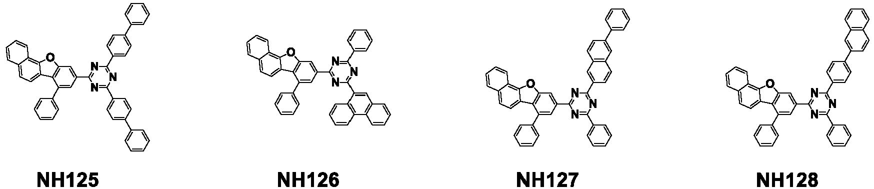

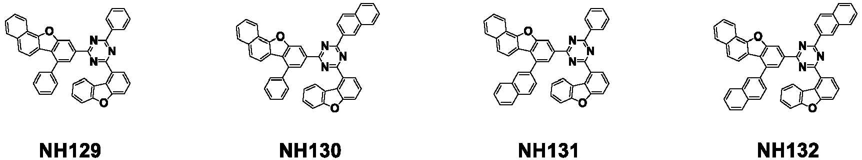

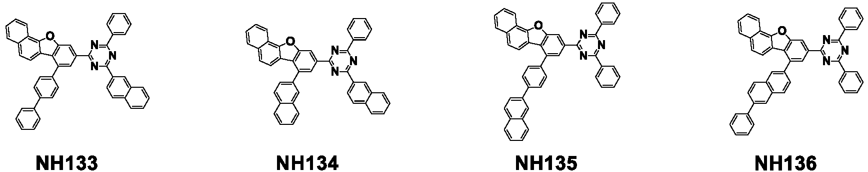

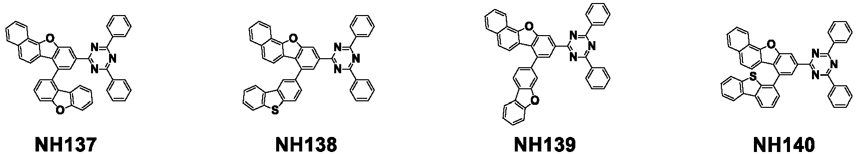

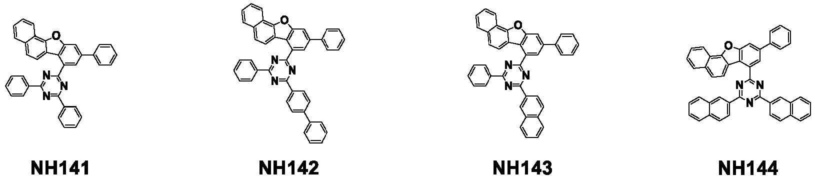

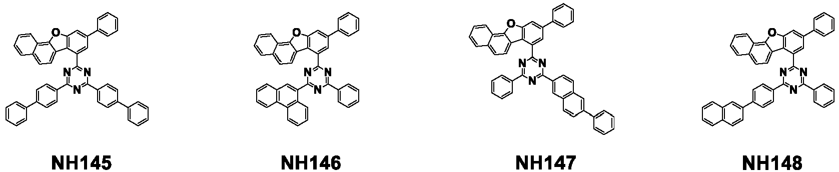

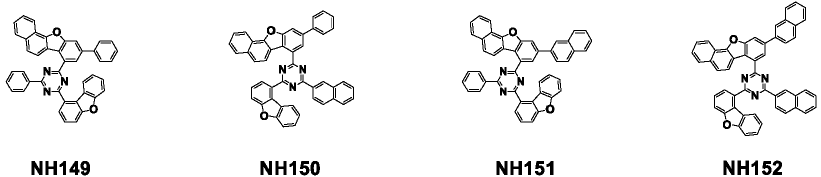

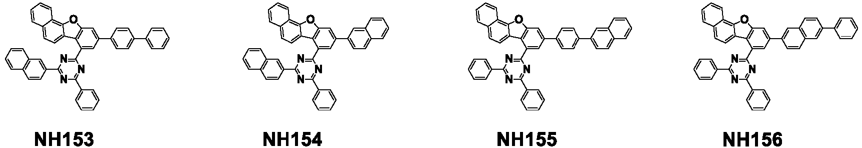

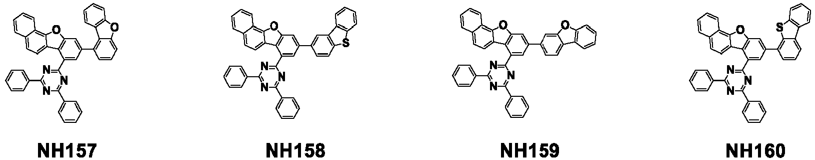

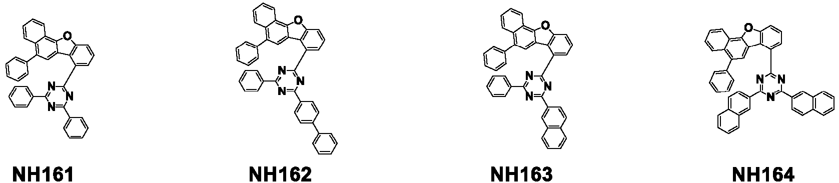

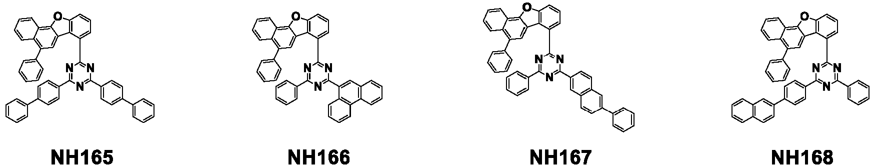

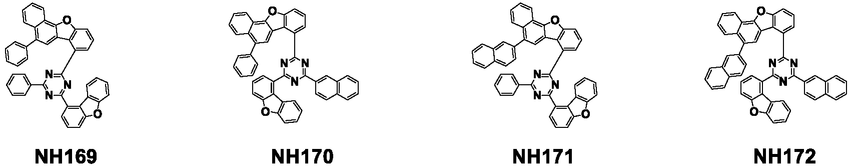

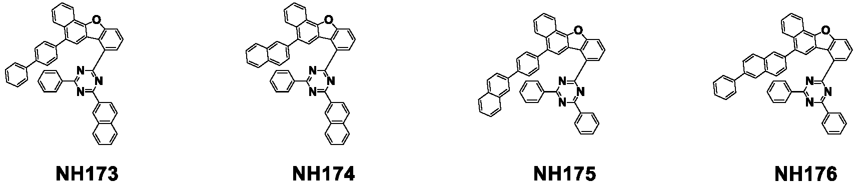

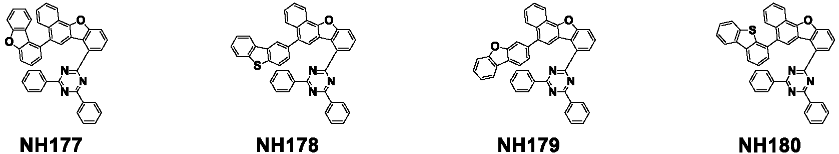

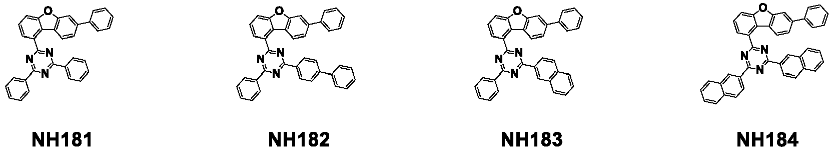

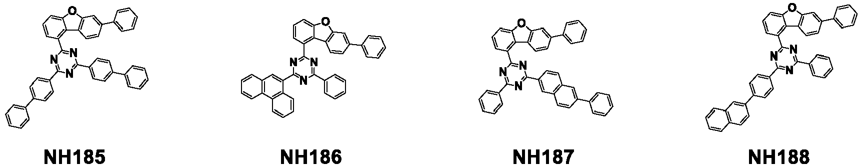

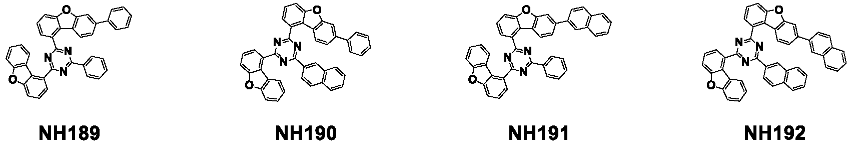

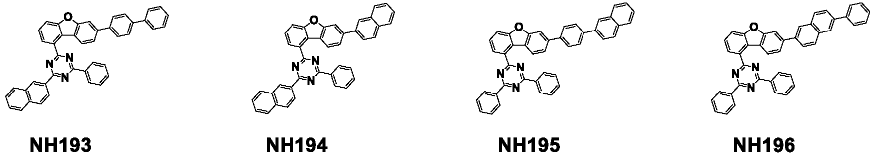

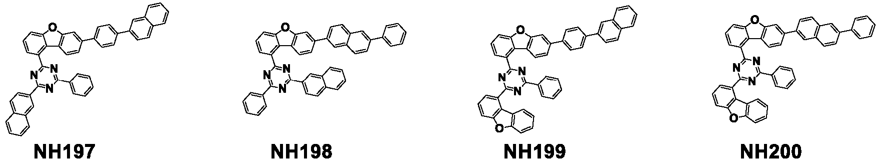

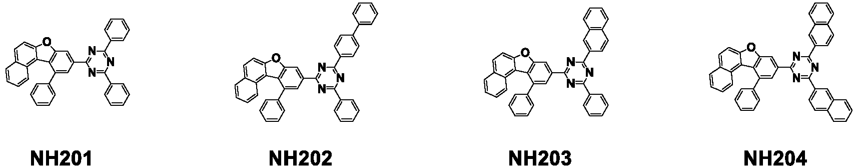

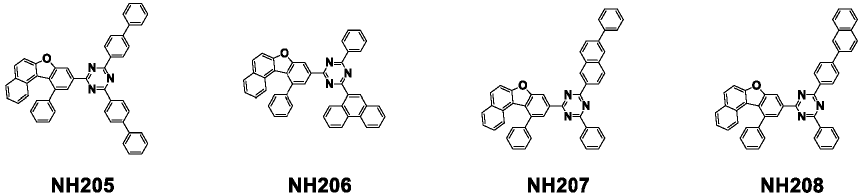

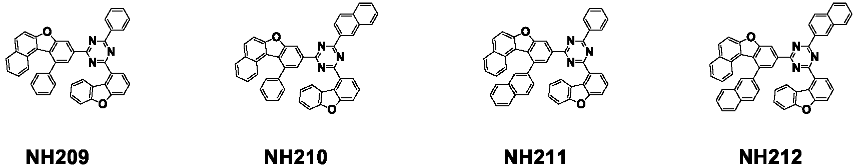

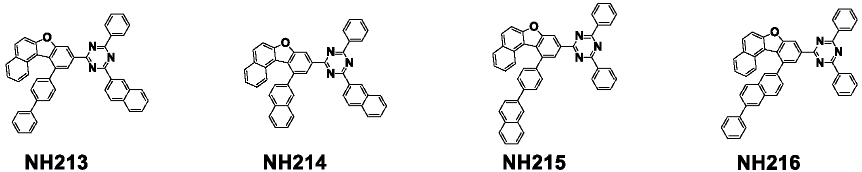

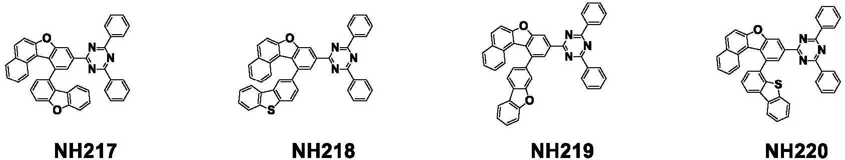

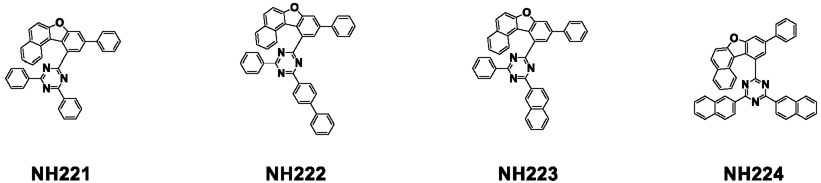

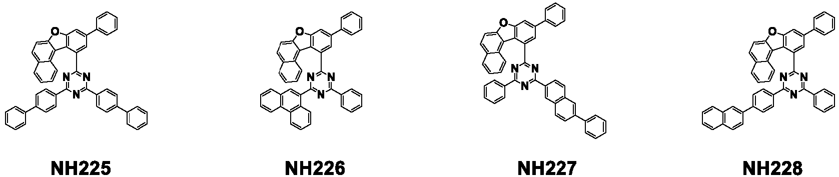

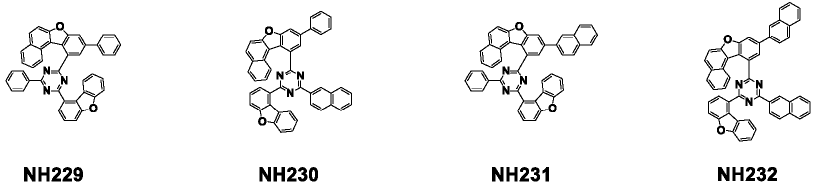

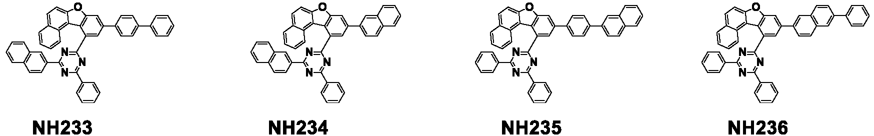

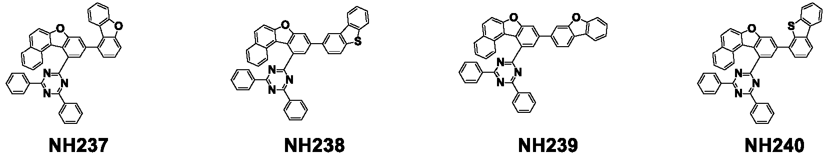

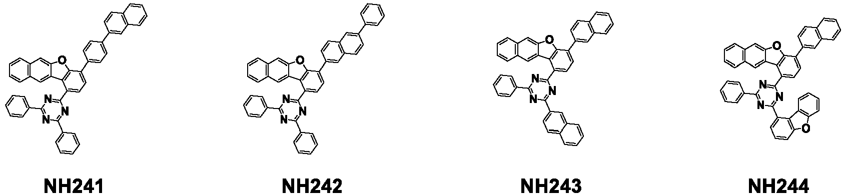

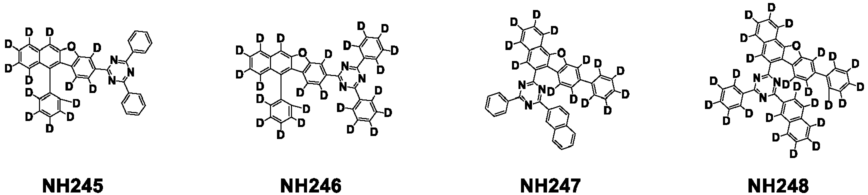

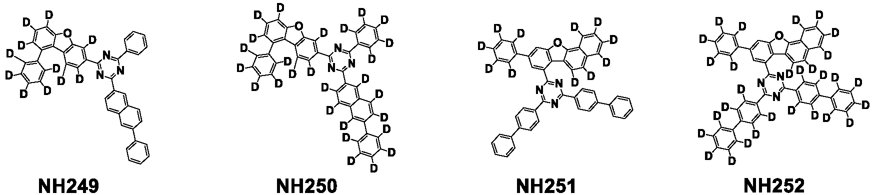

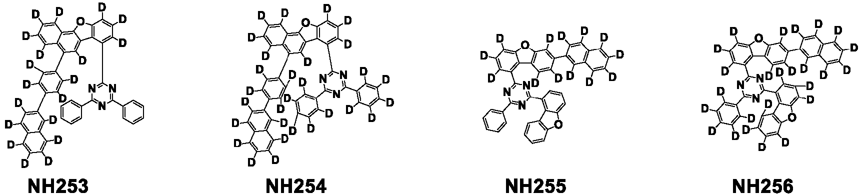

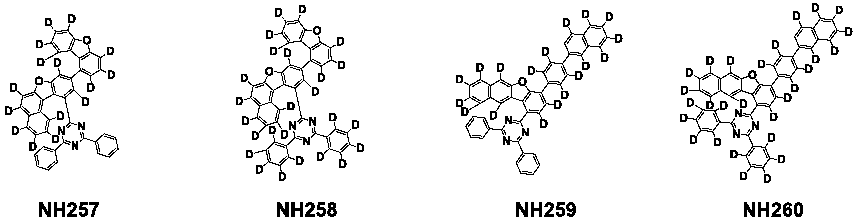

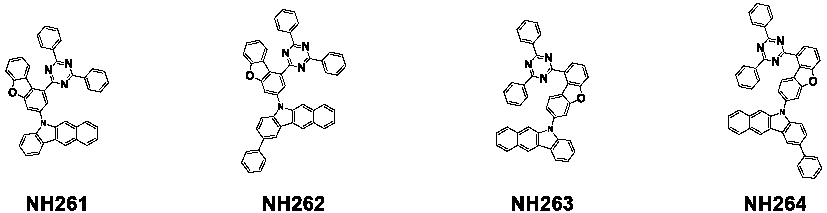

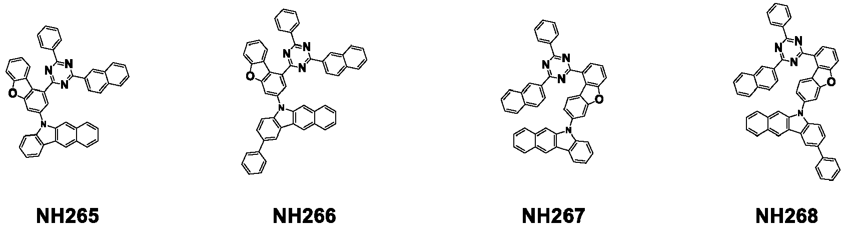

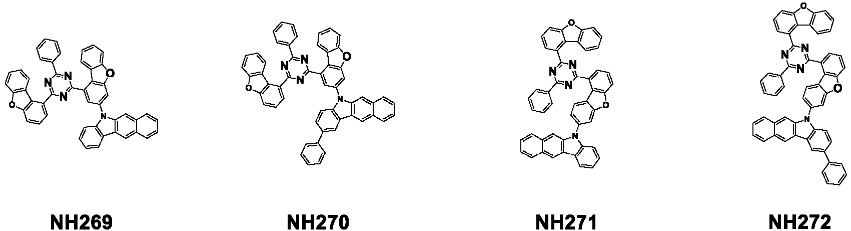

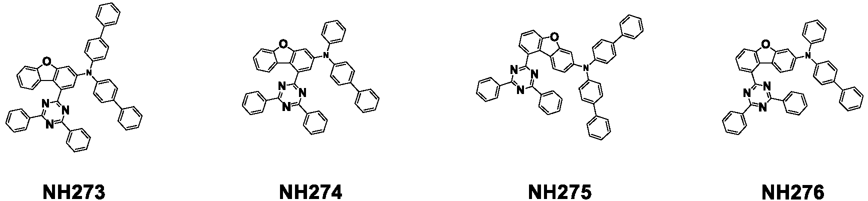

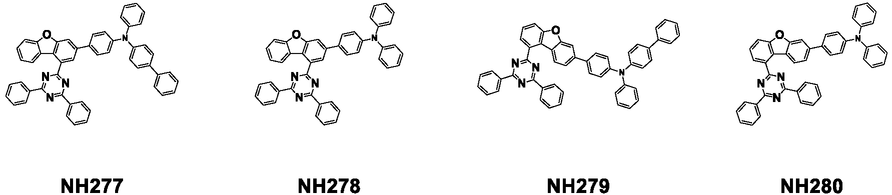

In addition, one embodiment of the present disclosure provides a composition for an organic material layer, the composition comprising: the heterocyclic compound represented by Chemical Formula 1; and the heterocyclic compound represented by Chemical Formula 4.

Specific descriptions on the heterocyclic compound represented by Chemical Formula 1 and the heterocyclic compound represented by Chemical Formula 4 are the same as the descriptions provided above.

In one embodiment of the present disclosure, the heterocyclic compound represented by Chemical Formula 1 and the heterocyclic compound represented by Chemical Formula 4 may have a weight ratio of 1:9 to 9:1, 1:9 to 5:5 or 2:8 to 5:5 in the composition for an organic material layer, however, the ratio is not limited thereto.

The composition for an organic material layer may be used when forming an organic material of an organic light emitting device, and particularly, may be more preferably used when forming a host of a light emitting layer.

In one embodiment of the present disclosure, the organic material layer includes the heterocyclic compound represented by Chemical Formula 1 and the heterocyclic compound represented by Chemical Formula 4, and a phosphorescent dopant may be used therewith.

As the phosphorescent dopant material, those known in the art may be used. For example, phosphorescent dopant materials represented by LL'MX', LL'L"M, LMX'X", L₂MX' and L₃M may be used, however, the scope of the present disclosure is not limited by these examples.

M may be iridium, platinum, osmium or the like.

L is an anionic bidentate ligand coordinated to M by sp² carbon and heteroatom, and X may function to trap electrons or holes. Nonlimiting examples of L may include 2-(1-naphthyl)benzoxazole, 2-phenylbenzoxazole, 2-phenylbenzothiazole, 7,8-benzoquinoline, phenylpyridine, benzothiophenylpyridine, 3-methoxy-2-phenylpyridine, thiophenylpyridine, tolylpyridine and the like. Nonlimiting examples of X' and X" may include acetylacetonate (acac), hexafluoroacetylacetonate, salicylidene, picolinate, 8-hydroxyquinolinate and the like.

Specific examples of the phosphorescent dopant are shown below, however, the phosphorescent dopant is not limited to these examples.

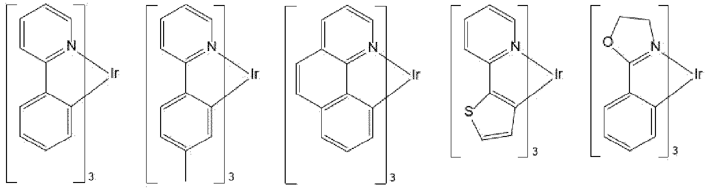

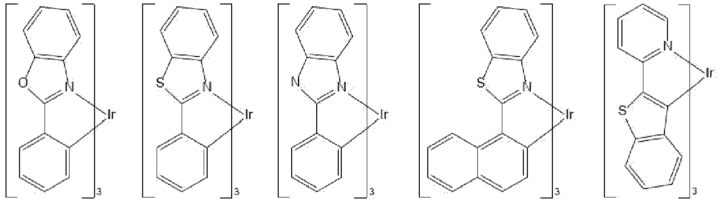

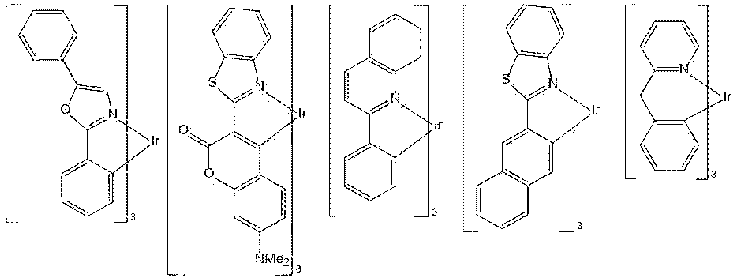

In one embodiment of the present disclosure, the organic material layer includes the heterocyclic compound represented by Chemical Formula 1 and the heterocyclic compound represented by Chemical Formula 4, and an iridium-based dopant may be used therewith.

In one embodiment of the present disclosure, as the iridium-based dopant, (piq)₂(Ir) (acac) may be used as a red phosphorescent dopant, or Ir(ppy)₃ may be used as a green phosphorescent dopant.

In one embodiment of the present disclosure, the content of the dopant may be from 1% to 15%, preferably from 2% to 10% and more preferably from 3% to 7% based on the total weight of the light emitting layer.

In one embodiment of the present disclosure, the organic material layer may include one or more selected from the group consisting of an electron injection layer, an electron transport layer, a hole blocking layer, a light emitting layer, a light emitting auxiliary layer, an electron blocking layer, a hole transport layer and a hole injection layer, and the one or more layers selected from the group consisting of an electron injection layer, an electron transport layer, a hole blocking layer, a light emitting layer, a light emitting auxiliary layer, an electron blocking layer, a hole transport layer and a hole injection layer may include the heterocyclic compound represented by Chemical Formula 1.

In another embodiment of the present disclosure, the organic material layer may include an electron injection layer or an electron transport layer, and the electron injection layer or the electron transport layer may include the heterocyclic compound represented by Chemical Formula 1.

In another embodiment of the present disclosure, the organic material layer may include an electron blocking layer or a hole blocking layer, and the electron blocking layer or the hole blocking layer may include the heterocyclic compound represented by Chemical Formula 1.

In another embodiment of the present disclosure, the organic material layer may include an electron blocking layer, a light emitting layer or a hole transport layer, and the electron blocking layer, the light emitting layer or the hole transport layer may include the heterocyclic compound represented by Chemical Formula 1.

In another embodiment of the present disclosure, the organic material layer may include a hole transport layer, and the hole transport layer may include the heterocyclic compound represented by Chemical Formula 1. The heterocyclic compound represented by Chemical Formula 1 has high hole mobility and proper HOMO level, and accordingly, when the heterocyclic compound represented by Chemical Formula 1 is used in the hole transport layer, holes are readily transported to the light emitting layer, and driving voltage of an organic light emitting device may be lowered, and driving efficiency and lifetime may be improved.

In another embodiment of the present disclosure, the organic material layer may include an electron blocking layer, and the electron blocking layer may include the heterocyclic compound represented by Chemical Formula 1. The heterocyclic compound represented by Chemical Formula 1 has a high LUMO level, and therefore, when the heterocyclic compound represented by Chemical Formula 1 is used in the electron blocking layer, probability of forming excitons by holes and electrons may increase, and possibility of them being emitted as light in the light emitting layer may increase. Accordingly, an electron blocking ability is improved, and charge balance is obtained between holes and electrons, and as a result, driving efficiency and lifetime of an organic light emitting device may become superior.

In another embodiment of the present disclosure, the organic material layer may include a light emitting layer, and the light emitting layer may include the heterocyclic compound represented by Chemical Formula 1.

In another embodiment of the present disclosure, the organic material layer includes a light emitting layer, the light emitting layer includes a host material, and the host material may include the heterocyclic compound represented by Chemical Formula 1.

In another embodiment of the present disclosure, the organic material layer includes a light emitting layer, and the light emitting layer may include the heterocyclic compound represented by Chemical Formula 1 and the heterocyclic compound represented by Chemical Formula 4.

In another embodiment of the present disclosure, the organic material layer includes a light emitting layer, the light emitting layer may include two or more host materials. At least one of the host materials may include the heterocyclic compound represented by Chemical Formula 1, and another one may include the heterocyclic compound represented by Chemical Formula 4.

In another embodiment of the present disclosure, the organic material layer includes a light emitting layer, and two or more host materials may be pre-mixed and used in the light emitting layer. At least one of the two or more host materials may include the heterocyclic compound represented by Chemical Formula 1, and another one may include the heterocyclic compound represented by Chemical Formula 4.

The pre-mixing means, before depositing the two or more host materials on the organic material layer, mixing the materials first and placing them in one source of supply for mixing.

The organic light emitting device according to one embodiment of the present disclosure may further include one, or two or more layers selected from the group consisting of a light emitting layer, a hole injection layer, a hole transport layer, an electron injection layer, an electron transport layer, an electron blocking layer and a hole blocking layer.

FIGS. 1 to 3 illustrate a lamination order of electrodes and organic material layers of the organic light emitting device according to one embodiment of the present disclosure. However, it is not intended that the scope of the present application be limited by these drawings, and structures of organic light emitting devices known in the art may also be applied to the present application.

FIG. 1 illustrates an organic light emitting device in which a positive electrode 200, an organic material layer 300 and a negative electrode 400 are sequentially laminated on a substrate 100. However, the structure is not limited only to such a structure, and as illustrated in FIG. 2, an organic light emitting device in which a negative electrode, an organic material layer and a positive electrode are sequentially laminated on a substrate may also be obtained.

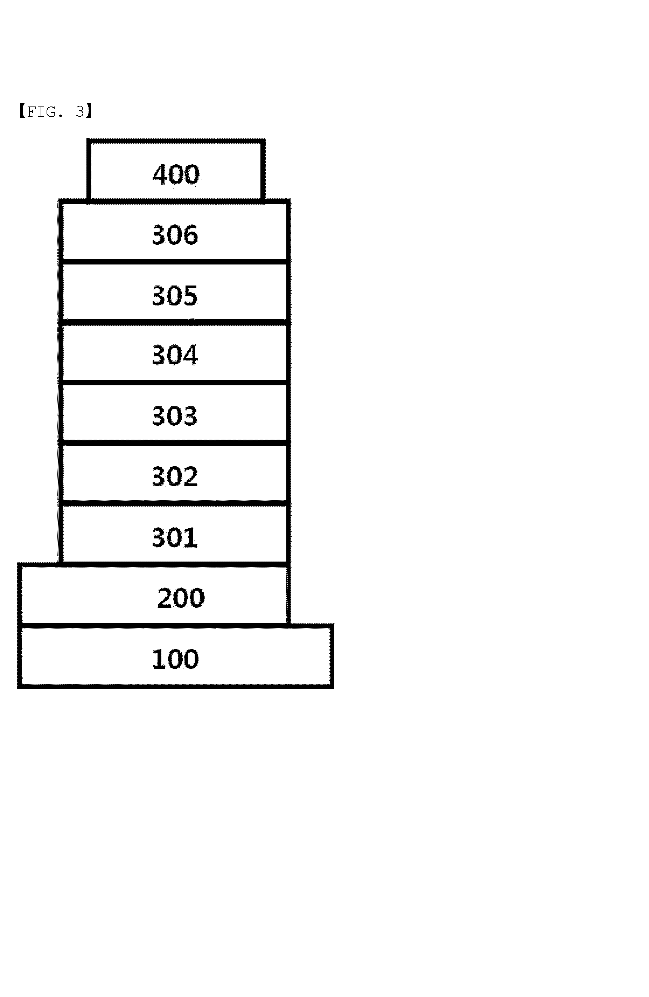

FIG. 3 illustrates a case of the organic material layer being a multilayer. An organic light emitting device according to FIG. 3 includes a hole injection layer 301, a hole transport layer 302, a light emitting layer 303, a hole blocking layer 304, an electron transport layer 305 and an electron injection layer 306. However, the scope of the present application is not limited by such a lamination structure, and as necessary, the layers other than the light emitting layer may not be included, and other necessary functional layers may be further added.

One embodiment of the present disclosure provides a method for manufacturing an organic light emitting device, the method including: preparing a substrate; forming a first electrode on the substrate; forming one or more organic material layers on the first electrode; and forming a second electrode on the one or more organic material layers, wherein the forming of one or more organic material layers includes forming the one or more organic material layers using the composition for an organic material layer according to one embodiment of the present disclosure.

In one embodiment of the present disclosure, the forming of organic material layers may be forming the organic material layers using a thermal vacuum deposition method after pre-mixing the heterocyclic compound represented by Chemical Formula 1 and the heterocyclic compound represented by Chemical Formula 4.

The pre-mixing means, before depositing the heterocyclic compound represented by Chemical Formula 1 and the heterocyclic compound represented by Chemical Formula 4 on the organic material layer, mixing the materials first and placing them in one source of supply for mixing.

The pre-mixed material may be referred to as the composition for an organic material layer according to one embodiment of the present application.

The organic material layer including the heterocyclic compound represented by Chemical Formula 1 may further include other materials as necessary.

The organic material layer including the heterocyclic compound represented by Chemical Formula 1 and the heterocyclic compound represented by Chemical Formula 4 at the same time may further include other materials as necessary.

In the organic light emitting device according to one embodiment of the present disclosure, materials other than the heterocyclic compound represented by Chemical Formula 1 and the heterocyclic compound represented by Chemical Formula 4 are illustrated below, however, these are for illustrative purposes only and not for limiting the scope of the present application, and these materials may be replaced by materials known in the art.

As the positive electrode material, materials each having a relatively large work function may be used, and transparent conductive oxides, metals, conductive polymers or the like may be used. Specific examples of the positive electrode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the negative electrode material, materials each having a relatively small work function may be used, and metals, metal oxides, conductive polymers or the like may be used. Specific examples of the negative electrode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection layer material, known hole injection layer materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tris[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], conductive polymers having solubility such as polyaniline/dodecylbenzenesulfonic acid or poly(3,4-ethylenedioxythiophene)/poly(4-styrenesulfonate), polyaniline/camphor sulfonic acid or polyaniline/poly(4-styrenesulfonate), and the like, may be used.

As the hole transport layer material, pyrazoline derivatives, arylamine-based derivatives, stilbene derivatives, triphenyldiamine derivatives and the like may be used, and low molecular or high molecular materials may also be used.

As the electron transport layer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials as well as low molecular materials may also be used.

As examples of the electron injection layer material, LiF is typically used in the art, however, the present application is not limited thereto.

As the light emitting layer material, red, green or blue light emitting materials may be used, and as necessary, two or more light emitting materials may be mixed and used. Herein, the two or more light emitting materials may be deposited as individual sources of supply or pre-mixed and deposited as one source of supply when used. In addition, fluorescent materials may also be used as the light emitting layer material, however, phosphorescent materials may also be used. As the light emitting layer material, materials emitting light alone by binding holes and electrons injected from a positive electrode and a negative electrode, respectively, may be used, however, materials having a host material and a dopant material involved in light emission together may also be used.

When hosts of the light emitting layer material are mixed and used, same series hosts may be mixed and used, or different series hosts may be mixed and used. For example, any two or more types of materials among n-type host materials and p-type host materials may be selected and used as a host material of a light emitting layer.

The organic light emitting device according to one embodiment of the present disclosure may be a top-emission type, a bottom-emission type or a dual-emission type depending on the materials used.

The heterocyclic compound according to one embodiment of the present disclosure may also be used in an organic electronic device including an organic solar cell, an organic photo conductor, an organic transistor and the like under a principle similar to that in the organic light emitting device.

Hereinafter, preferred examples are provided to help to understand the present disclosure, however, the following examples are only provided to more readily understand the present disclosure, and the present disclosure is not limited thereto.

### <Preparation Example>

### Preparation Example 1. Preparation of Compound 2

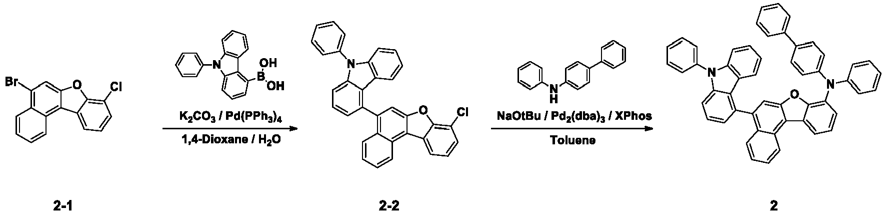

### Preparation Example 1-1. Preparation of Compound 2-2

Compound 2-1 (15 g, 0.045 mol, 1 eq.), (9-phenyl-9H-carbazol-4-yl)boronic acid (14.3 g, 0.049 mol, 1.1 eq.), K₂CO₃ (15.6 g, 0.113 mol, 2.5 eq.) and Pd(PPh₃)₄ (2.6 g, 0.002 mol, 0.05 eq.) were introduced to 1,4-dioxane (150 mL) and distilled water (30 mL), and the mixture was stirred for 8 hours at 100°C.

After terminating the reaction by introducing distilled water thereto, the result was extracted using methylene chloride and water, and moisture was removed using MgSO₄. After that, the result was separated using a silica gel column to obtain Compound 2-2 (16 g, yield 72%) .

### Preparation Example 1-2. Preparation of Compound 2

Compound 2-2 (8 g, 0.016 mol, 1 eq.), N-phenyl-[1,1'-biphenyl]-4-amine (4.2 g, 0.017 mol, 1.05 eq.), NaOt-Bu (2.3 g, 0.024 mol, 1.5 eq.), Pd₂(dba)₃ (0.7 g, 0.0008 mol, 0.05 eq.) and Xphos (0.8 g, 0.0016 mol, 0.1 eq.) were introduced to toluene (120 mL), and the mixture was stirred for 5 hours at 90°C.

After terminating the reaction by introducing distilled water thereto, the result was extracted using methylene chloride and water, and moisture was removed using MgSO₄. After that, the result was separated using a silica gel column to obtain Compound 2 (9 g, yield 76%).

Target compounds were prepared as shown in the following Table 1 in the same manner as in Preparation Example 1, except that Compound A of the following Table 1 was used instead of Compound 2-1, Compound B of the following Table 1 was used instead of (9-phenyl-9H-carbazol-4-yl)boronic acid, and Compound C of the following Table 1 was used instead of N-phenyl-[1,1'-biphenyl]-4-amine.

**[Table 1]**

| Compo und No. | Compound A | Compound B | Compound C | Target Compound |
|---|---|---|---|---|
| 30 | 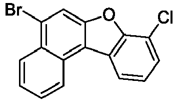 | 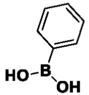 | 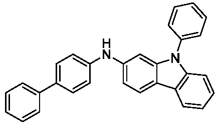 | 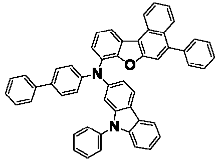 |
| 43 | 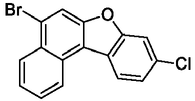 | 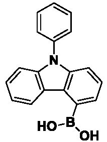 | 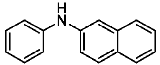 | 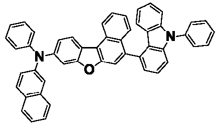 |
| 72 | 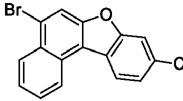 | 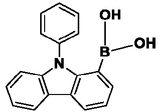 | 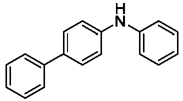 | 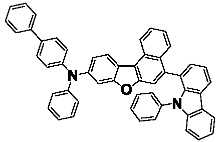 |
| 97 | 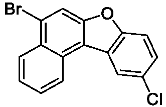 | 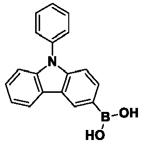 | 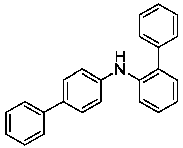 | 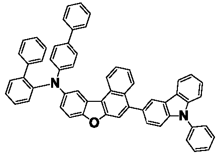 |
| 104 | 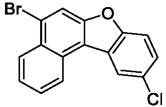 | 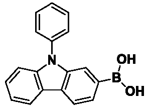 | 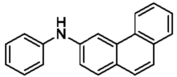 | 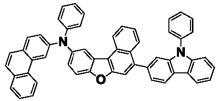 |
| 130 | 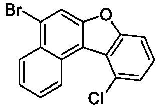 | 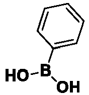 | 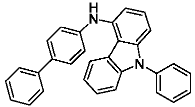 | 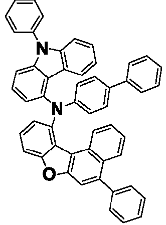 |
| 155 | 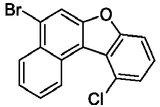 | 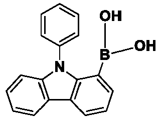 | 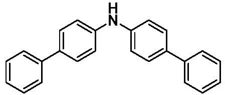 | 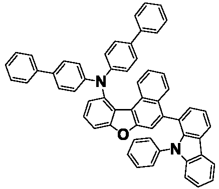 |
| 176 | 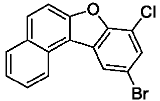 | 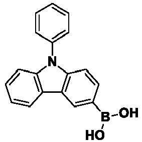 | 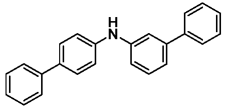 | 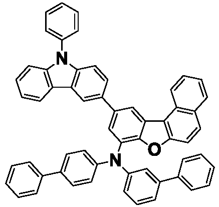 |
| 189 | 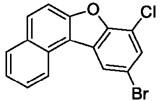 | 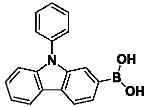 | 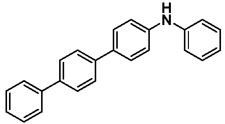 | 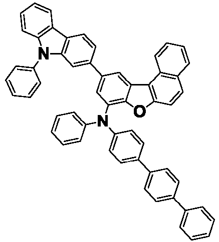 |
| 220 | 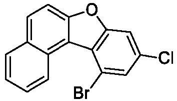 | 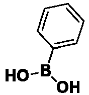 | 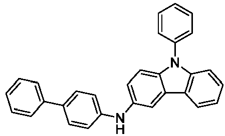 | 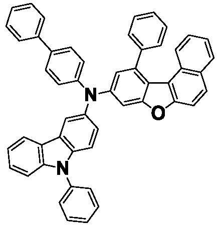 |
| 221 | 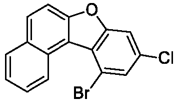 | 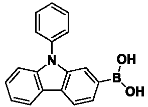 | 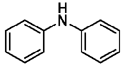 | 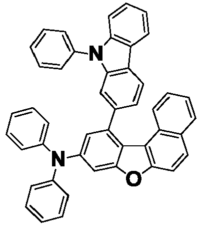 |
| 242 | 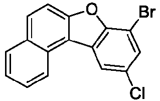 | 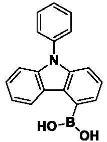 | 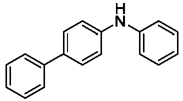 | 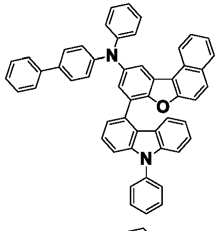 |
| 275 | 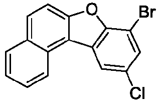 | 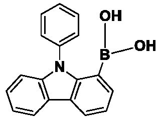 | 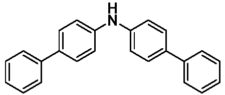 | 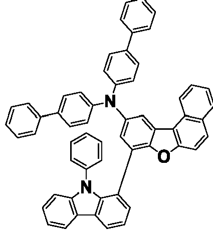 |
| 285 | 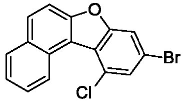 | 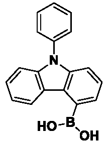 | 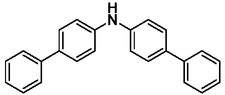 | 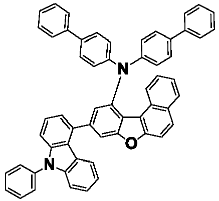 |
| 318 | 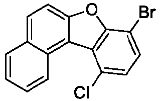 | 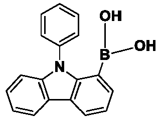 | 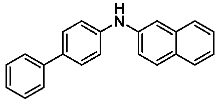 | 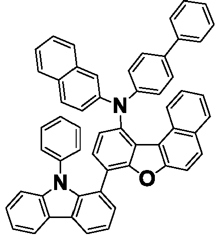 |
| 322 | 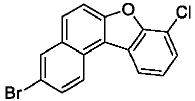 | 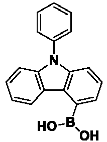 | 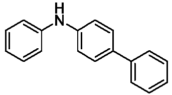 | 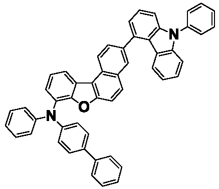 |
| 355 | 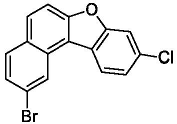 | 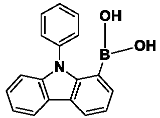 | 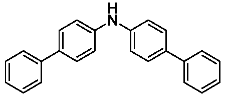 | 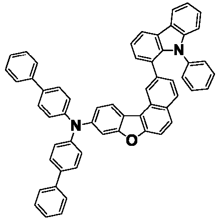 |
| 380 | 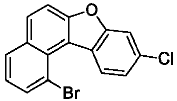 | 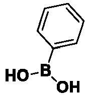 | 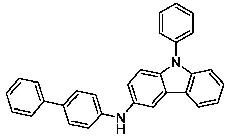 | 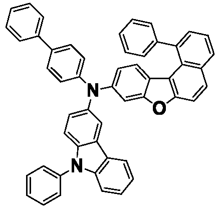 |
| 388 | 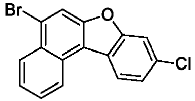 | 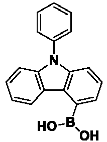 | 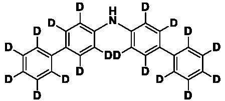 | 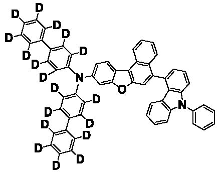 |
| 414 | 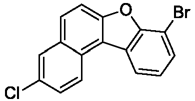 | 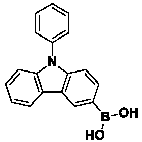 | 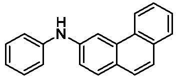 | 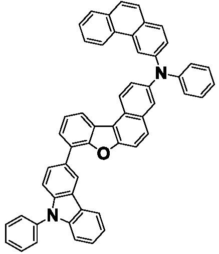 |
| 430 | 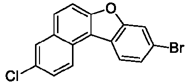 | 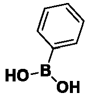 | 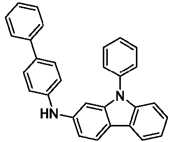 | 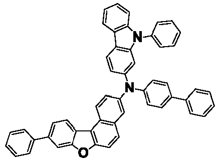 |
| 445 | 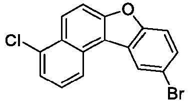 | 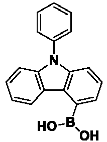 | 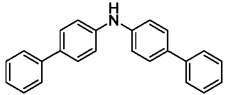 | 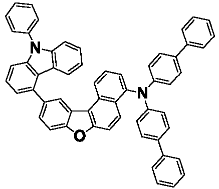 |
| 475 | 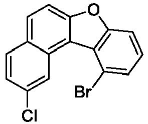 | 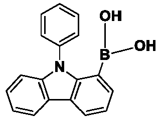 | 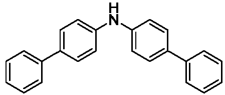 | 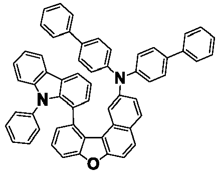 |
| 481 | 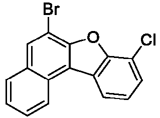 | 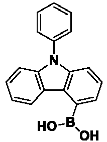 | 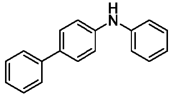 | 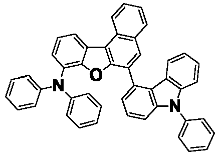 |
| 517 | 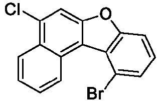 | 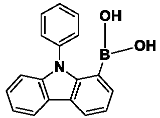 | 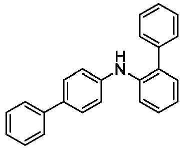 | 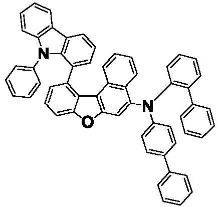 |

### Preparation Example 2. Preparation of Compound 540

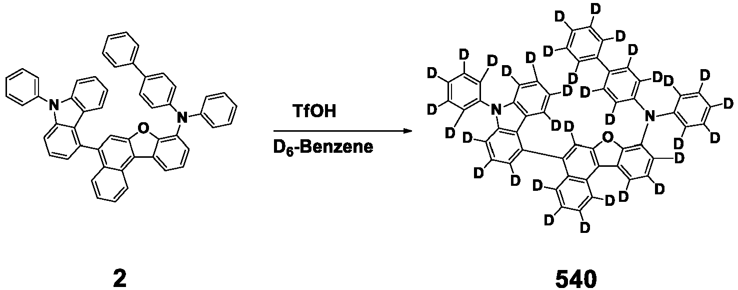

After introducing Compound 2 (8 g, 0.011 mol, 1 eq.), TfOH (2.7 g, 0.018 mol, 1.5 eq.) and D₆-benzene (70 mL), the mixture was stirred for 6 hours at 80°C.

After terminating the reaction by introducing distilled water thereto, the result was extracted using methylene chloride and water, and moisture was removed using MgSO₄. After that, the result was separated using a silica gel column to obtain Compound 540 (7 g, yield 83%).

### Preparation Example 3. Preparation of Compound NH7

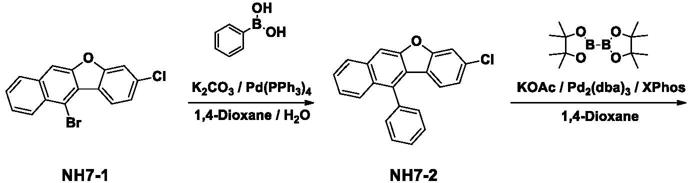

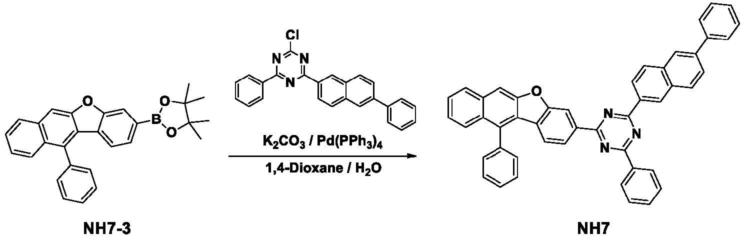

### Preparation Example 3-1. Preparation of Compound NH7-2

Compound NH7-1 (15 g, 0.045 mol, 1 eq.), phenylboronic acid (6.1 g, 0.050 mol, 1.1 eq.), K₂CO₃ (18.8 g, 0.136 mol, 3 eq.) and Pd(PPh₃)₄ (2.6 g, 0.002 mol, 0.05 eq.) were introduced to 1,4-dioxane (150 mL) and distilled water (30 mL), and the mixture was stirred for 8 hours at 100°C.

After terminating the reaction by introducing distilled water thereto, the result was extracted using methylene chloride and water, and moisture was removed using MgSO₄. After that, the result was separated using a silica gel column to obtain Compound NH7-2 (12 g, yield 81%).

### Preparation Example 3-2. Preparation of Compound NH7-3

Compound NH7-2 (12 g, 0.036 mol, 1 eq.), 4,4,4',4',5,5,5',5'-octamethyl-2,2'-bi(1,3,2-dioxaborolane (13.7 g, 0.054 mol, 1.5 eq.), KOAc (10.6 g, 0.108 mol, 3 eq.), Pd₂(dba)₃ (1.6 g, 0.0018 mol, 0.05 eq.) and XPhos (1.7 g, 0.0036 mol, 0.1 eq.) were introduced to 1,4-dioxane (100 mL), and the mixture was stirred for 8 hours at 100°C.

After terminating the reaction by introducing distilled water thereto, the result was extracted using methylene chloride and water, and moisture was removed using MgSO₄. After that, the result was separated using a silica gel column to obtain Compound NH7-3 (10 g, yield 65%) .

### Preparation Example 3-3. Preparation of Compound NH7

Compound NH7-3 (10 g, 0.024 mol, 1 eq.), 2-chloro-4-phenyl-6-(6-phenylnaphthalen-2-yl)-1,3,5-triazine (10.3 g, 0.026 mol, 1.1 eq.), K₂CO₃ (9.9 g, 0.071 mol, 3 eq.) and Pd(PPh₃)₄ (1.4 g, 0.001 mol, 0.05 eq.) were introduced to 1,4-dioxane (100 mL) and distilled water (20 mL), and the mixture was stirred for 6 hours at 100°C.

After terminating the reaction by introducing distilled water thereto, the result was extracted using methylene chloride and water, and moisture was removed using MgSO₄. After that, the result was separated using a silica gel column to obtain Compound NH7 (13 g, yield 84%).

Target compounds were prepared as shown in the following Table 2 in the same manner as in Preparation Example 3, except that Compound D of the following Table 2 was used instead of Compound NH7-1, Compound E of the following Table 2 was used instead of phenylboronic acid, and Compound F of the following Table 2 was used instead of 2-chloro-4-phenyl-6-(6-phenylnaphthalen-2-yl)-1,3,5-triazine.

**[Table 2]**

| Compo und No. | Compound D | Compound E | Compound F | Target Compound |
|---|---|---|---|---|
| NH71 | 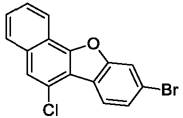 | 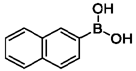 | 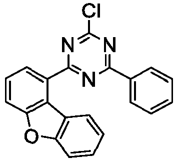 | 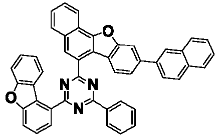 |
| NH96 | 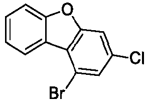 | 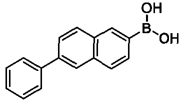 | 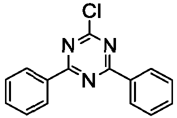 | 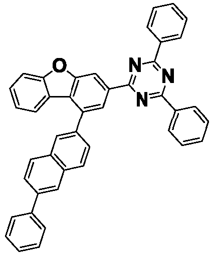 |
| NH157 | 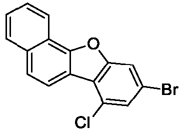 | 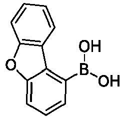 | 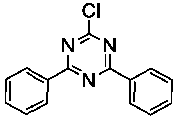 | 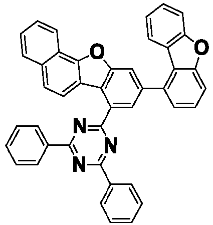 |
| NH163 | 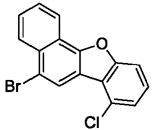 | 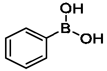 | 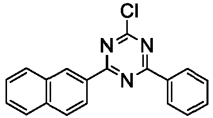 | 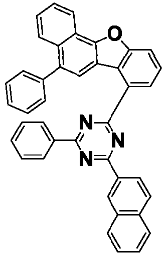 |
| NH225 | 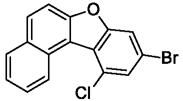 | 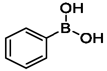 | 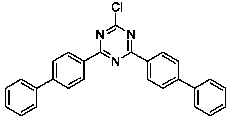 | 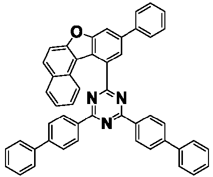 |
| NH241 | 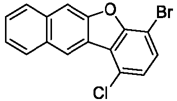 | 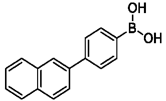 | 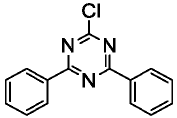 | 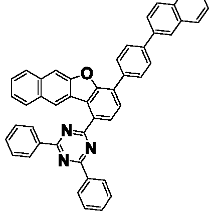 |

Synthesis results for the compounds described in Preparation Examples 1 to 3 and Tables 1 and 2 are shown in the following Tables 3 and 4.

The following Table 3 shows measurement values of ¹H NMR (CDCl₃, 200 MHz), and the following Table 4 shows measurement values of field desorption mass spectrometry (FD-MS).

**[Table 3]**

| Compoun d No. | ¹H NMR (CDCl₃, 200 MHz) |
|---|---|
| 2 | δ=8.55(3H, m), 7.94(1H, d), 7.79(1H, d), 7.59-7.20(22H, m), 7.07(1H, t), 6.81(1H, t), 6.63(4H, m), 6.39(1H, d) |
| 30 | δ=8.55(3H, m), 8.03(1H, d), 7.94(1H, d), 7.79(2H, m), 7.64-7.25(21H, m), 7.07(1H, t), 6.69(4H, m), 6.39(1H, d) |
| 43 | δ=8.55(3H, m), 7.88(6H, m), 7.64-7.20(19H, m), 6.81(1H, t), 6.63(2H, m), 6.33(1H, d) |
| 72 | δ=8.55(3H, m), 8.08(1H, d), 7.87(2H, m), 7.64-7.20(22H, m), 6.81(1H, t), 6.69(4H, m), 6.33(1H, d) |
| 97 | δ=8.55(3H, m), 7.94(2H, m), 7.77(1H, s), 7.69-7.16(27H, m), 6.87(1H, t), 6.69(3H, m), 6.33(1H, d) |
| 104 | δ=8.93(1H, d), 8.55(3H, m), 8.12(3H, m), 7.88-7.20(22H, m), 7.02(1H, d), 6.81(1H, t), 6.63(2H, m), 6.39(1H, d) |
| 130 | δ=8.55(2H, m), 8.12(1H, d), 7.79(2H, m), 7.64-7.41(20H, m), 7.30(2H, m), 7.08(3H, m), 6.69(2H, m), 6.33(1H, d), 5.85(1H, d) |
| 155 | δ=8.55(3H, m), 8.08(1H, d), 7.94(1H, d), 7.87(1H, d), 7.64-7.35(25H, m), 7.13(1H, t), 7.02(1H, d), 6.69(4H, m), 6.33(1H, d) |
| 176 | δ=8.55(2H, m), 8.16(1H, d), 7.94(1H, d), 7.87(1H, d), 7.77(1H, s), 7.69-7.25(26H, m), 7.07(1H, s), 6.88(2H, m), 6.69(2H, m), 6.59(1H, d) |
| 189 | δ=8.54(2H, m), 8.18(2H, m), 7.94(1H, d), 7.79(1H, d), 7.67-7.20(26H, m), 7.07(1H, s), 6.81(1H, t), 6.63(4H, m) |
| 220 | δ=8.55(2H, m), 8.16(1H, d), 7.94(1H, d), 7.79(2H, m), 7.67-7.25(24H, m), 6.69(4H, m) |
| 221 | δ=8.54(2H, m), 8.16(2H, m), 7.94(1H, d), 7.79(1H, d), 7.67-7.25(18H, m), 6.81(2H, m), 6.63(4H, m) |
| 242 | δ=8.55(2H, m), 8.16(1H, d), 7.94(1H, d), 7.79(1H, d), 7.67-7.25(24H, m), 6.81(1H, t), 6.63(4H, m) |
| 275 | δ=8.55(2H, m), 8.16(1H, d), 8.08(1H, d), 7.94(1H, d), 7.87(1H, d), 7.67-7.25(28H, m), 6.69(4H, m) |
| 285 | δ=8.55(2H, m), 8.16(1H, d), 7.94(1H, d), 7.79(1H, d), 7.67-7.25(28H, m), 7.00(1H, s), 6.69(4H, m) |
| 318 | δ=8.55(2H, m), 8.16(1H, d), 8.08(1H, d), 7.94-7.25(29H, m), 6.69(2H, m), 6.39(1H, d) |
| 322 | δ=8.55(1H, d), 8.06(1H, d), 7.94(1H, d), 7.79-7.20(24H, m), 7.07(1H, t), 6.81(1H, t), 6.63(4H, m), 6.39(1H, d) |
| 355 | δ=8.55(1H, d), 8.34(1H, s), 8.08(1H, d), 7.87(3H, m), 7.73(1H, d), 7.73-7.25(26H, m), 6.69(4H, m), 6.33(1H, d) |
| 380 | δ=8.42(1H, d), 8.12(1H, d), 8.04(1H, d), 7.79(2H, m), 7.79-7.41(24H, m), 7.29(1H, t), 6.69(2H, m), 6.33(1H, d) 5.93(1H. d) |
| 388 | δ=8.55(3H, m), 7.94(1H, d), 7.79(1H, d), 7.64-7.25(14H, m), 6.33(1H, d) |
| 414 | δ=8.93(1H, d), 8.55(1H, d), 8.13(2H, m), 7.94-7.20(26H, m), 7.02(1H, d), 6.81(1H, t), 6.63(2H, m) |
| 430 | δ=8.55(1H, d), 8.03(1H, d), 7.95(2H, m), 7.84(1H, d), 7.75(2H, m), 7.66-7.25(23H, m), 6.69(4H, m) |
| 445 | δ=8.55(1H, d), 8.04(1H, d), 7.94(1H, d), 7.79(2H, m, 7.72-7.33(28H, m), 6.98(1H, d), 6.69(4H, m) |
| 475 | δ=8.51(1H, d), 8.30(1H, d), 8.12(1H, d), 7.84(1H, d), 7.74(2H, m), 7.66-7.41(26H, m), 7.29(2H, m), 6.69(4H, m) |
| 481 | δ=8.55(1H, d), 8.00(3H, m), 7.79(1H, d), 7.59-7.20(17H, m), 7.07(1H, t), 6.81(2H, m), 6.63(4H, m), 6.39(1H, d) |
| 517 | δ=8.55(2H, d), 8.08(2H, m), 7.94(2H, m), 7.75(1H, d), 7.62-7.08(27H, m), 6.87(1H, t), 6.69(3H, m) |
| 540 | Fully substituted with D |
| NH7 | δ=9.09(1H, s), 8.55(2H, m), 8.28(2H, m), 8.08(1H, d), 7.92(3H, m), 7.75(2H, m), 7.64(1H, s) 7.55(17H, m) |
| NH71 | δ=8.28(2H, m), 8.00(7H, m), 7.75-7.32(18H, m) |
| NH96 | δ=8.28(4H, m), 7.92(3H, m), 7.73-7.32(20H, m) |
| NH157 | δ=8.28(4H, m), 8.16(2H, m), 7.89(2H, m), 7.75-7.32(17H, m) |
| NH163 | δ=9.09(1H, s), 8.49(2H, m), 8.28(2H, m), 8.18(1H, d), 7.92(3H, m), 7.79(4H, m), 7.62-7.41(12H, m) |
| NH225 | δ=8.54(1H, d), 8.16(1H, d), 7.85(4H, m), 7.67-7.41(21H, m), 7.25(4H, m) |
| NH241 | δ=8.28(4H, m), 8.16(2H, m), 7.87(5H, m), 7.73-7.41(14H, m), 7.25(4H, m) |

**[Table 4]**

| Compound No. | FD-MS | Compound No. | FD-MS |
|---|---|---|---|
| 2 | m/z=702.27 | 355 | m/z=778.30 |
| 30 | m/z=702.27 | 380 | m/z=702.27 |
| 43 | m/z=676.25 | 388 | m/z=796.41 |
| 72 | m/z=702.27 | 414 | m/z=726.27 |
| 97 | m/z=778.30 | 430 | m/z=702.27 |
| 104 | m/z=726.27 | 445 | m/z=778.30 |
| 130 | m/z=702.27 | 475 | m/z=778.30 |
| 155 | m/z=778.30 | 481 | m/z=626.24 |
| 176 | m/z=778.30 | 517 | m/z=778.30 |
| 189 | m/z=778.30 | 540 | m/z=736.48 |
| 220 | m/z=702.27 | NH7 | m/z=651.23 |
| 221 | m/z=626.24 | NH71 | m/z=665.21 |
| 242 | m/z=702.27 | NH96 | m/z=601.22 |
| 275 | m/z=778.30 | NH157 | m/z=615.19 |
| 285 | m/z=778.30 | NH163 | m/z=575.20 |
| 318 | m/z=752.28 | NH225 | m/z=677.25 |
| 322 | m/z=702.27 | NH241 | m/z=651.23 |

### Experimental Example 1.

### Experimental Example 1-1. Manufacture of Organic Light Emitting Device

A transparent electrode ITO thin film obtained from glass for an OLED (manufactured by Samsung Corning Advanced Glass) was ultrasonic cleaned using trichloroethylene, acetone, ethanol and distilled water sequentially for 5 minutes each, and then stored in isopropanol before use. Then, the ITO substrate was installed in a substrate folder of a vacuum deposition apparatus.

To a cell in the vacuum deposition apparatus, 4,4',4"-tris(N,N-(2-naphthyl)-phenylamino)triphenyl amine (2-TNATA) was introduced.

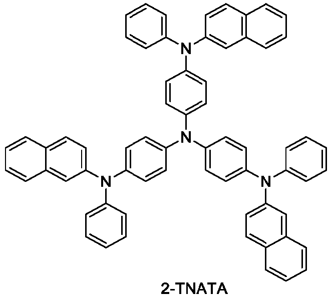

Subsequently, the chamber was evacuated until the degree of vacuum therein reached 10⁻⁶ torr, and then the 2-TNATA was evaporated by applying a current to the cell to deposit a hole injection layer having a thickness of 600 Å on the ITO substrate. To another cell in the vacuum deposition apparatus, the compound represented by Chemical Formula 1, a comparative example compound or N,N'-bis(α-naphthyl)-N,N'-diphenyl-4,4'-diamine (NPB) described in the following Table 5 was introduced, and evaporated by applying a current to the cell to deposit a hole transport layer having a thickness of 300 Å on the hole injection layer.

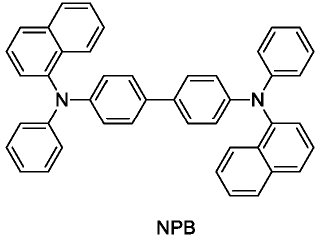

After forming the hole injection layer and the hole transport layer as above, a blue light emitting material having the following structure was deposited thereon as a light emitting layer. Specifically, on one cell in the vacuum deposition apparatus, BH1 that is a blue light emitting host material was vacuum deposited to a thickness of 200 Å, and D1 that is a blue light emitting dopant material was vacuum deposited thereon by 5% with respect to the host material. After that, E1 was deposited to 300 Å as an electron transport layer.

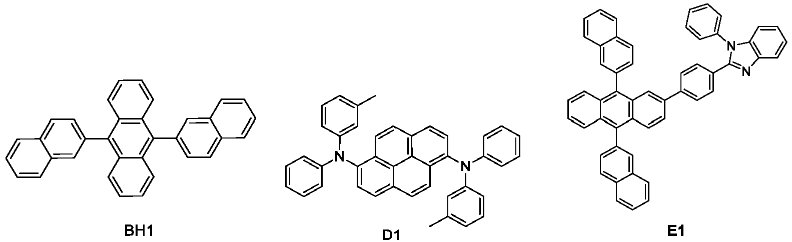

After that, lithium fluoride (LiF) was deposited to a thickness of 10 Å as an electron injection layer, and Al was deposited to a thickness of 1000 Å to form a negative electrode, and as a result, an organic light emitting device was manufactured.

Meanwhile, all the organic compounds required to manufacture the organic light emitting device were vacuum sublimation purified under 10⁻⁸ torr to 10⁻⁶ torr for each material to be used in the manufacture of the organic light emitting device.

### Experimental Example 1-2. Driving Voltage and Light Emission Efficiency of Organic Light Emitting Device

For each of the organic electroluminescent devices manufactured as above, electroluminescent (EL) properties were measured using M7000 manufactured by McScience Inc., and with the measurement results, T₉₅, a lifetime that is a time taken for luminance to become 95% with respect to initial luminance, was measured when standard luminance was 700 cd/m² through a lifetime measurement system (M6000) manufactured by McScience Inc.

Results of measuring driving voltage, light emission efficiency and lifetime (T₉₅) of the blue organic light emitting devices manufactured according to the above-described manufacturing method are shown in Table 5.

**[Table 5]**

| | Compound | Driving Voltage (V) | Light Emission Efficienc y (cd/A) | CIE (x, y) | Lifetime (T95) |
|---|---|---|---|---|---|
| Example 1 | 2 | 4.2 | 6.5 | (0.14, 0.05) | 43 |
| Example 2 | 30 | 4.3 | 6.6 | (0.14, 0.05) | 47 |
| Example 3 | 43 | 4.2 | 6.4 | (0.14, 0.05) | 45 |
| Example 4 | 72 | 4.2 | 6.5 | (0.14, 0.05) | 44 |
| Example 5 | 97 | 4.3 | 6.4 | (0.14, 0.05) | 48 |
| Example 6 | 104 | 4.2 | 6.5 | (0.14, 0.05) | 46 |
| Example 7 | 130 | 4.2 | 6.6 | (0.14, 0.05) | 43 |
| Example 8 | 155 | 4.2 | 6.4 | (0.14, 0.05) | 44 |
| Example 9 | 176 | 4.3 | 6.5 | (0.14, 0.05) | 46 |
| Example 10 | 189 | 4.3 | 6.4 | (0.14, 0.05) | 45 |
| Example 11 | 220 | 4.3 | 6.6 | (0.14, 0.05) | 47 |
| Example 12 | 221 | 4.2 | 6.4 | (0.14, 0.05) | 43 |
| Example 13 | 242 | 4.3 | 6.4 | (0.14, 0.05) | 45 |
| Example 14 | 275 | 4.3 | 6.5 | (0.14, 0.05) | 48 |
| Example 15 | 285 | 4.2 | 6.5 | (0.14, 0.05) | 46 |
| Example 16 | 318 | 4.3 | 6.6 | (0.14, 0.05) | 47 |
| Example 17 | 322 | 4.2 | 6.6 | (0.14, 0.05) | 43 |
| Example 18 | 355 | 4.3 | 6.4 | (0.14, 0.05) | 44 |
| Example 19 | 380 | 4.2 | 6.4 | (0.14, 0.05) | 45 |
| Example 20 | 388 | 4.3 | 6.5 | (0.14, 0.05) | 47 |
| Example 21 | 414 | 4.2 | 6.5 | (0.14, 0.05) | 48 |
| Example 22 | 430 | 4.3 | 6.6 | (0.14, 0.05) | 43 |
| Example 23 | 445 | 4.2 | 6.5 | (0.14, 0.05) | 47 |
| Example 24 | 475 | 4.3 | 6.4 | (0.14, 0.05) | 45 |
| Example 25 | 481 | 4.3 | 6.4 | (0.14, 0.05) | 46 |
| Example 26 | 517 | 4.3 | 6.5 | (0.14, 0.05) | 43 |
| Example 27 | 540 | 4.2 | 6.4 | (0.14, 0.05) | 44 |
| Comparative Example 1 | NPB | 5.3 | 5.3 | (0.14, 0.05) | 29 |
| Comparative Example 2 | H1 | 5.0 | 5.5 | (0.14, 0.05) | 1 |
| Comparative Example 3 | H2 | 5.1 | 5.4 | (0.14, 0.05) | 31 |
| Comparative Example 4 | H3 | 5.2 | 5.5 | (0.14, 0.05) | 33 |
| Comparative Example 5 | H4 | 5.2 | 5.4 | (0.14, 0.05) | 30 |
| Comparative Example 6 | H5 | 5.1 | 5.3 | (0.14, 0.05) | 32 |

### [Comparative Example Compound]

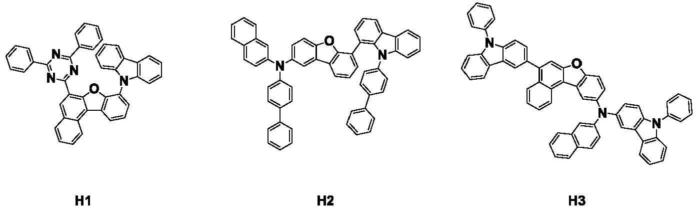

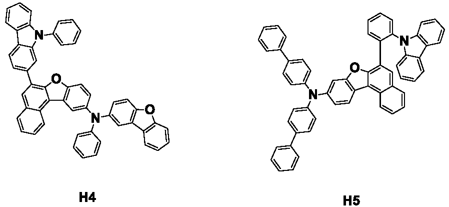

As seen from the results of Table 5, the blue organic light emitting devices of Examples 1 to 27 using the heterocyclic compound represented by Chemical Formula 1 of the present disclosure as a hole transport layer showed results of lower driving voltage, and significantly superior light emission efficiency and lifetime compared to the organic light emitting devices of Comparative Examples 1 to 6. Specifically, the heterocyclic compound represented by Chemical Formula 1 of the present disclosure suppresses pi-pi stacking of the aromatic ring, thereby preventing a phenomenon of property decline in the organic light emitting device. Accordingly, the organic light emitting device using the heterocyclic compound represented by Chemical Formula 1 of the present disclosure as a hole transport layer may obtain low driving voltage, high light emission efficiency and long lifetime properties.

Compound H1 of Comparative Example 2 does not include an arylamine group corresponding to Chemical Formula 2 and includes a triazine group instead of an arylamine group, and Compound H2 of Comparative Example 3 has a core structure different from the heterocyclic compound represented by Chemical Formula 1 of the present disclosure. Compound H3 of Comparative Example 4 includes two carbazole groups corresponding to Chemical Formula 3, Compound H4 of Comparative Example 5 has a structure in which Ar2 or Ar3 of the arylamine group corresponding to Chemical Formula 2 includes a heteroaryl group, and Compound H5 of Comparative Example 6 has a structure in which a carbazole group corresponding to Chemical Formula 3 is linked by a phenylene linker.

NPB used as a compound of an existing hole transport layer and the compounds of Comparative Examples 2 to 6 have core structures or substituents different from the heterocyclic compound represented by Chemical Formula 1 of the present disclosure, and accordingly, showed results of higher driving voltage and lower light emission efficiency and shorter lifetime compared to the present disclosure.

From the above-described results, it may be seen that the organic light emitting device including the heterocyclic compound represented by Chemical Formula 1 of the present disclosure as a hole transport layer has low driving voltage, high light emission efficiency and long lifetime properties.

### Experimental Example 2.

### Experimental Example 2-1. Manufacture of Organic Light Emitting Device

A transparent electrode ITO thin film obtained from glass for an OLED (manufactured by Samsung Corning Advanced Glass) was ultrasonic cleaned using trichloroethylene, acetone, ethanol and distilled water sequentially for 5 minutes each, and then stored in isopropanol before use.

The ITO substrate was installed in a substrate folder of a vacuum deposition apparatus, and 4,4',4"-tris(N,N-(2-naphthyl)-phenylamino)triphenyl amine (2-TNATA) was introduced thereto.

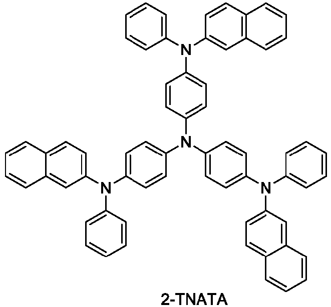

Subsequently, the chamber was evacuated until the degree of vacuum therein reached 10⁻⁶ torr, and then the 2-TNATA was evaporated by applying a current to the cell to deposit a hole injection layer having a thickness of 600 Å on the ITO substrate. To another cell in the vacuum deposition apparatus, N,N'-bis(α-naphthyl)-N,N'-diphenyl-4,4'-diamine (NPB) was introduced, and evaporated by applying a current to the cell to deposit a hole transport layer having a thickness of 250 Å on the hole injection layer.

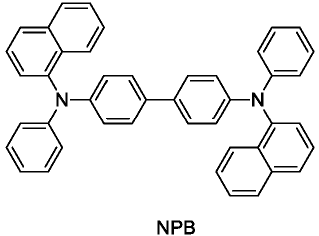

Subsequently, the compound represented by Chemical Formula 1 or a comparative example compound described in Table 6 was deposited to a thickness of 50 Å as an electron blocking layer.

A blue light emitting material having the following structure was deposited thereon as a light emitting layer. Specifically, on one cell in the vacuum deposition apparatus, BH1 that is a blue light emitting host material was vacuum deposited to a thickness of 200 Å, and D1 that is a blue light emitting dopant material was vacuum deposited thereon by 5% with respect to the host material.

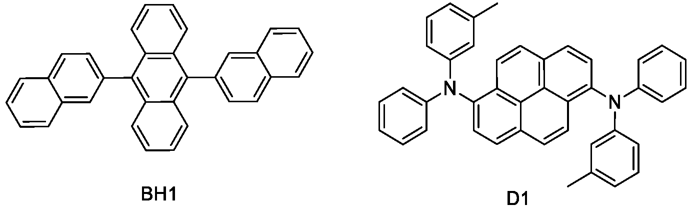

After that, a compound of the following Structural Formula E1 was deposited to a thickness of 300 Å as an electron transport layer.

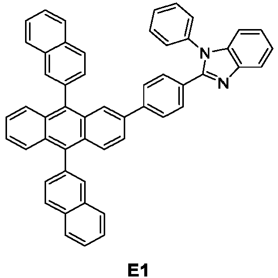

After that, lithium fluoride (LiF) was deposited to a thickness of 10 Å as an electron injection layer, and Al was deposited to a thickness of 1000 Å to form a negative electrode, and as a result, an organic light emitting device was manufactured.

Meanwhile, all the organic compounds required to manufacture the organic light emitting device were vacuum sublimation purified under 10⁻⁸ torr to 10⁻⁶ torr for each material to be used in the manufacture of the organic light emitting device.

### Experimental Example 2-2. Driving Voltage and Light Emission Efficiency of Organic Light Emitting Device

For each of the organic electroluminescent devices manufactured as above, electroluminescent (EL) properties were measured using M7000 manufactured by McScience Inc., and with the measurement results, T₉₅, a lifetime that is time taken for luminance to become 95% with respect to initial luminance, was measured when standard luminance was 700 cd/m² through a lifetime measurement system (M6000) manufactured by McScience Inc.

Results of measuring driving voltage, light emission efficiency and lifetime (T₉₅) of the blue organic light emitting devices manufactured according to the above-described manufacturing method are shown in Table 6.

**[Table 6]**

| | Compound | Driving Voltage (V) | Light Emission Efficiency (cd/A) | CIE (x, y) | Lifetime (T95) |
|---|---|---|---|---|---|
| Example 28 | 2 | 4.1 | 6.5 | (0.14, 0.05) | 44 |
| Example 29 | 30 | 4.2 | 6.6 | (0.14, 0.05) | 48 |
| Example 30 | 43 | 4.3 | 6.5 | (0.14, 0.05) | 46 |
| Example 31 | 72 | 4.2 | 6.4 | (0.14, 0.05) | 45 |
| Example 32 | 97 | 4.1 | 6.5 | (0.14, 0.05) | 49 |
| Example 33 | 104 | 4.2 | 6.4 | (0.14, 0.05) | 47 |
| Example 34 | 130 | 4.2 | 6.5 | (0.14, 0.05) | 44 |
| Example 35 | 155 | 4.3 | 6.6 | (0.14, 0.05) | 45 |
| Example 36 | 176 | 4.2 | 6.4 | (0.14, 0.05) | 46 |
| Example 37 | 189 | 4.1 | 6.5 | (0.14, 0.05) | 45 |
| Example 38 | 220 | 4.1 | 6.4 | (0.14, 0.05) | 47 |
| Example 39 | 221 | 4.2 | 6.6 | (0.14, 0.05) | 44 |
| Example 40 | 242 | 4.2 | 6.5 | (0.14, 0.05) | 46 |
| Example 41 | 275 | 4.1 | 6.6 | (0.14, 0.05) | 48 |
| Example 42 | 285 | 4.3 | 6.4 | (0.14, 0.05) | 46 |
| Example 43 | 318 | 4.2 | 6.5 | (0.14, 0.05) | 48 |
| Example 44 | 322 | 4.2 | 6.5 | (0.14, 0.05) | 44 |
| Example 45 | 355 | 4.1 | 6.4 | (0.14, 0.05) | 45 |
| Example 46 | 380 | 4.2 | 6.5 | (0.14, 0.05) | 45 |
| Example 47 | 388 | 4.1 | 6.6 | (0.14, 0.05) | 48 |
| Example 48 | 414 | 4.2 | 6.5 | (0.14, 0.05) | 48 |
| Example 49 | 430 | 4.2 | 6.5 | (0.14, 0.05) | 44 |
| Example 50 | 445 | 4.3 | 6.4 | (0.14, 0.05) | 47 |
| Example 51 | 475 | 4.1 | 6.6 | (0.14, 0.05) | 46 |
| Example 52 | 481 | 4.2 | 6.5 | (0.14, 0.05) | 47 |
| Example 53 | 517 | 4.3 | 6.4 | (0.14, 0.05) | 44 |
| Example 54 | 540 | 4.1 | 6.5 | (0.14, 0.05) | 45 |
| Comparative Example 7 | H1 | 5.1 | 5.4 | (0.14, 0.05) | 2 |
| Comparative Example 8 | H2 | 4.9 | 5.5 | (0.14, 0.05) | 32 |
| Comparative Example 9 | H3 | 5.0 | 5.4 | (0.14, 0.05) | 34 |
| Comparative Example 10 | H4 | 5.0 | 5.5 | (0.14, 0.05) | 31 |
| Comparative Example 11 | H5 | 4.9 | 5.4 | (0.14, 0.05) | 33 |

### [Comparative Example Compound]

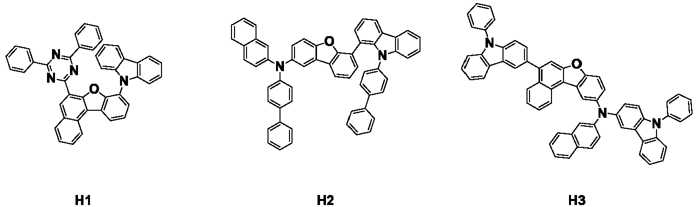

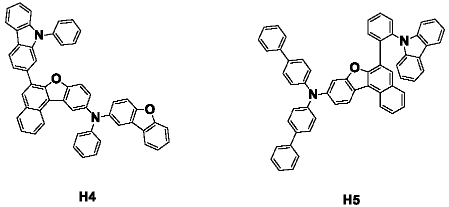

As seen from the results of Table 6, the blue organic light emitting devices of Examples 28 to 54 using the heterocyclic compound represented by Chemical Formula 1 of the present disclosure as an electron blocking layer showed results of lower driving voltage, and significantly superior light emission efficiency and lifetime compared to the organic light emitting devices of Comparative Examples 7 to 11. When electrons pass through a hole transport layer and migrate to a positive electrode without binding in a light emitting layer, efficiency and lifetime of an organic light emitting device are reduced. When a compound having a high LUMO level is used as an electron blocking layer, electrons attempting to pass through a light emitting layer and migrate to a positive electrode are blocked by an energy barrier of the electron blocking layer, and the above-described phenomenon may be prevented. In other words, probability of forming excitons by holes and electrons increases and possibility of them being emitted as light in the light emitting layer increases. Since the heterocyclic compound represented by Chemical Formula 1 of the present disclosure is a compound having a high LUMO level, the above-described phenomenon may be prevented. Accordingly, the organic light emitting device using the heterocyclic compound represented by Chemical Formula 1 of the present disclosure as an electron blocking layer may obtain low driving voltage, high light emission efficiency and long lifetime properties.

As described in Experimental Example 1, Comparative Example Compounds H1 to H5 have core structures or substituents different from the heterocyclic compound represented by Chemical Formula 1 of the present disclosure. Accordingly, Comparative Example Compounds H1 to H5 exhibited a lower LUMO level compared to the heterocyclic compound represented by Chemical Formula 1 of the present disclosure, and showed results of higher driving voltage and lower light emission efficiency and shorter lifetime compared to the present disclosure.

From the above-described results, it may be seen that the organic light emitting device including the heterocyclic compound represented by Chemical Formula 1 of the present disclosure as an electron blocking layer has low driving voltage, high light emission efficiency and long lifetime properties.

### Experimental Example 3.

### Experimental Example 3-1. Manufacture of Organic Light Emitting Device

A glass substrate on which ITO was coated as a thin film to a thickness of 1,500 Å was ultrasonic cleaned with distilled water. When the cleaning with distilled water was finished, the substrate was ultrasonic cleaned with solvents such as acetone, methanol and isopropyl alcohol, then dried, and then subjected to ultraviolet ozone (UVO) treatment for 5 minutes using UV in a UV cleaner. After that, the substrate was transferred to a plasma cleaner (PT), then subjected to plasma treatment under vacuum for ITO work function and residual film removal, and transferred to a thermal deposition apparatus for organic deposition.

On the transparent ITO electrode (positive electrode), as common layers, 4,4',4"-tris[2-naphthyl(phenyl)amino]triphenylamine (2-TNATA) was formed as a hole injection layer, N,N'-bis(α-naphthyl)-N,N'-diphenyl-4,4'-diamine (NPB) was formed as a hole transport layer, and cyclohexylidenebis[N,N-bis(4-methylphenyl)benzenamine] (TAPC) was formed as an electron blocking layer.

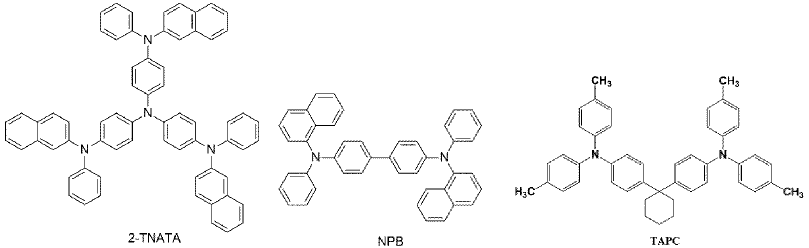

A light emitting layer was thermal vacuum deposited thereon as follows. The light emitting layer was deposited to 400 Å by depositing two types of compounds described in the following Table 7 as a red host in one source of supply and, using (piq)₂(Ir)(acac) as a red phosphorescent dopant, doping the Ir compound to the host by 3 wt%.

After that, Bphen was deposited to a thickness of 30 Å as a hole blocking layer, and TPBI was deposited to a thickness of 250 Å thereon as an electron transport layer. Lastly, lithium fluoride (LiF) was deposited on the electron transport layer to a thickness of 10 Å to form an electron injection layer, and then aluminum (Al) was deposited on the electron injection layer to a thickness of 1,200 Å to form a negative electrode, and as a result, an organic electroluminescent device was manufactured.

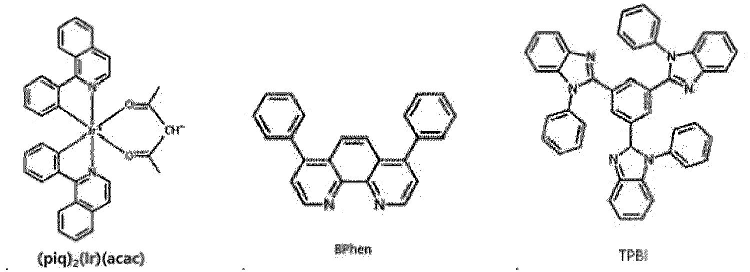

Meanwhile, all the organic compounds required to manufacture the OLED were vacuum sublimation purified under 10⁻⁸ torr to 10⁻⁶ torr for each material to be used in the manufacture of the organic light emitting device (OLED).

### Experimental Example 3-2. Driving Voltage and Light Emission Efficiency of Organic Light Emitting Device

For each of the organic light emitting devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc. Results of measuring driving voltage, light emission efficiency, color coordinate (CIE) and lifetime of the organic light emitting devices manufactured according to the present disclosure are shown in the following Table 7.

T₉₀ means a lifetime (unit: hour), a time taken for luminance to become 90% with respect to initial luminance.

**[Table 7]**

| | Compound | Ratio (P/N) | Driving Voltage (V) | Light Emission Efficienc y (cd/A) | CIE (x, y) | Lifet ime (T90) |
|---|---|---|---|---|---|---|
| Example 55 | 2:NH7 | 1:3 | 4.3 | 54.8 | (0.68, 0.32) | 188 |
| Example 56 | 2:NH7 | 1:2 | 4.2 | 55.7 | (0.68, 0.32) | 193 |
| Example 57 | 2:NH7 | 1:1 | 4.1 | 56.0 | (0.68, 0.32) | 200 |
| Example 58 | 2:NH7 | 2:1 | 4.2 | 55.3 | (0.68, 0.32) | 186 |
| Example 59 | 2:NH7 | 3:1 | 4.3 | 54.7 | (0.68, 0.32) | 178 |
| Example 60 | 30:NH7 | 1:1 | 4.1 | 56.3 | (0.68, 0.32) | 213 |
| Example 61 | 43:NH7 | 1:1 | 4.2 | 57.6 | (0.68, 0.32) | 208 |
| Example 62 | 72:NH7 | 1:1 | 4.0 | 56.8 | (0.68, 0.32) | 221 |
| Example 63 | 97:NH71 | 1:1 | 4.1 | 57.5 | (0.68, 0.32) | 203 |
| Example 64 | 104:NH71 | 1:1 | 4.2 | 57.1 | (0.68, 0.32) | 219 |
| Example 65 | 130;NH71 | 1:1 | 4.0 | 56.3 | (0.68, 0.32) | 225 |
| Example 66 | 155:NH71 | 1:1 | 4.1 | 57.8 | (0.68, 0.32) | 206 |
| Example 67 | 176:NH96 | 1:1 | 4.1 | 58.1 | (0.68, 0.32) | 196 |
| Example 68 | 189:NH96 | 1:1 | 4.0 | 56.6 | (0.68, 0.32) | 195 |
| Example 69 | 220:NH96 | 1:1 | 4.2 | 56.9 | (0.68, 0.32) | 203 |
| Example 70 | 221:NH96 | 1:1 | 4.2 | 57.3 | (0.68, 0.32) | 218 |
| Example 71 | 242:NH157 | 1:1 | 4.0 | 56.6 | (0.68, 0.32) | 225 |
| Example 72 | 275:NH157 | 1:1 | 4.0 | 57.7 | (0.68, 0.32) | 213 |
| Example 73 | 285:NH157 | 1:1 | 4.1 | 56.4 | (0.68, 0.32) | 225 |
| Example 74 | 318:NH157 | 1:1 | 4.2 | 56.8 | (0.68, 0.32) | 217 |
| Example 75 | 322:NH163 | 1:1 | 4.1 | 57.5 | (0.68, 0.32) | 197 |
| Example 76 | 355:NH163 | 1:1 | 4.0 | 56.3 | (0.68, 0.32) | 221 |
| Example 77 | 380:NH163 | 1:1 | 4.2 | 57.1 | (0.68, 0.32) | 202 |
| Example 78 | 388:NH163 | 1:1 | 4.1 | 56.5 | (0.68, 0.32) | 196 |
| Example 79 | 414:NH225 | 1:1 | 4.2 | 57.4 | (0.68, 0.32) | 219 |
| Example 80 | 430:NH225 | 1:1 | 4.0 | 58.0 | (0.68, 0.32) | 224 |
| Example 81 | 445:NH225 | 1:1 | 4.0 | 57.2 | (0.68, 0.32) | 220 |
| Example 82 | 475:NH225 | 1:1 | 4.1 | 57.8 | (0.68, 0.32) | 211 |
| Example 83 | 481:NH241 | 1:1 | 4.2 | 56.6 | (0.68, 0.32) | 218 |
| Example 84 | 517:NH241 | 1:1 | 4.1 | 57.0 | (0.68, 0.32) | 206 |
| Example 85 | 540:NH241 | 1:1 | 4.2 | 56.1 | (0.68, 0.32) | 200 |
| Comparative Example 12 | H1:NH7 | 1:1 | 4.9 | 47.3 | (0.68, 0.32) | 122 |
| Comparative Example 13 | H2:NH96 | 1:1 | 4.9 | 48.2 | (0.68, 0.32) | 123 |
| Comparative Example 14 | H3:NH163 | 1:1 | 4.8 | 47.7 | (0.68, 0.32) | 135 |
| Comparative Example 15 | H4:NH225 | 1:1 | 4.9 | 48.8 | (0.68, 0.32) | 117 |
| Comparative Example 16 | H5:NH241 | 1:1 | 4.8 | 46.4 | (0.68, 0.32) | 131 |

### [Comparative Example Compound]

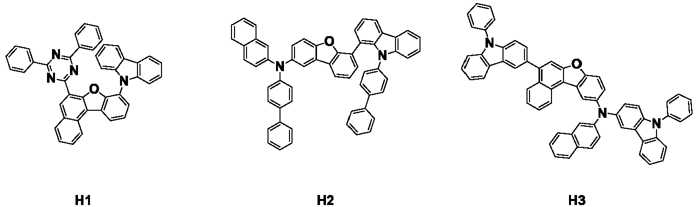

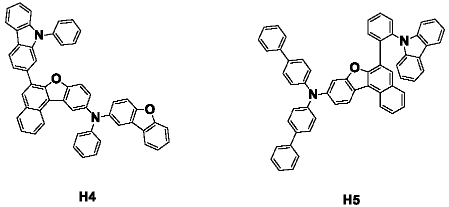

As seen from the results of Table 7, the red organic light emitting devices of Examples 55 to 85 using the heterocyclic compound represented by Chemical Formula 1 of the present disclosure as a p-type host and the heterocyclic compound represented by Chemical Formula 4 as an n-type host and using these as a light emitting layer after mixing showed results of lower driving voltage, and significantly superior light emission efficiency and lifetime compared to the organic light emitting devices of Comparative Examples 12 to 16.

When a donor (p-host) having a favorable hole transport ability and an acceptor (n-host) having a favorable electron transport ability are used as a host of a light emitting layer, holes are injected to the p-host and electrons are injected to the n-host due to an exciplex phenomenon of the two compounds, and thus a charge balance in the device may be obtained. Accordingly, it was able to be seen that combining the n-type host compound having proper electron transfer properties and the p-type host compound having proper hole transfer properties in a proper ratio was able to help with improvement in driving efficiency and lifetime.

As described in Experimental Example 1, Comparative Example Compounds H1 to H5 have core structures or substituents different from the heterocyclic compound represented by Chemical Formula 1 of the present disclosure. Accordingly, Comparative Example Compounds H1 to H5 exhibited a lower hole transport ability compared to the heterocyclic compound represented by Chemical Formula 1 of the present disclosure, and showed results of higher driving voltage and lower light emission efficiency and shorter lifetime compared to the present disclosure.

From the above-described results, it may be seen that the organic light emitting device including the heterocyclic compound represented by Chemical Formula 1 and the heterocyclic compound represented by Chemical Formula 4 of the present disclosure at the same time as a light emitting layer has low driving voltage, high light emission efficiency and long lifetime properties.

### [Reference Numeral]

100: Substrate
200: Positive Electrode
300: Organic Material Layer
301: Hole Injection Layer
302: Hole Transport Layer
303: Light Emitting Layer
304: Hole Blocking Layer
305: Electron Transport Layer
306: Electron Injection Layer
400: Negative Electrode

## Claims

1. A heterocyclic compound represented by the following Chemical Formula 1: 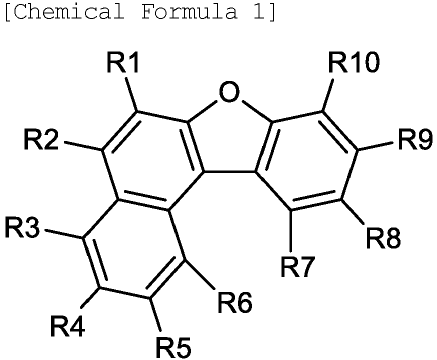
wherein, in Chemical Formula 1,
R1 to R10 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; Ar1; - P(=O)R101R102; -SiR101R102R103; and the following Chemical Formula 2, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group;
any one of R1 to R10 is the following Chemical Formula 2, and another one is Ar1; and
Ar1 is a substituted or unsubstituted C6 to C60 aryl group; or the following Chemical Formula 3, 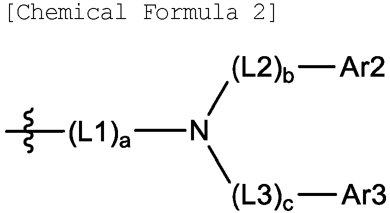
in Chemical Formula 2,
L1 to L3 are the same as or different from each other, and each independently a direct bond; a substituted or unsubstituted C6 to C60 arylene group; or a substituted or unsubstituted C2 to C60 heteroarylene group;
a is an integer of 0 to 5, and when a is 2 or greater, L1s are the same as or different from each other;
b is an integer of 0 to 5, and when b is 2 or greater, L2s are the same as or different from each other;
c is an integer of 0 to 5, and when c is 2 or greater, L3s are the same as or different from each other;
Ar2 and Ar3 are the same as or different from each other, and each independently a substituted or unsubstituted C6 to C60 aryl group; or the following Chemical Formula 3; and
any one of Ar1 to Ar3 is the following Chemical Formula 3, 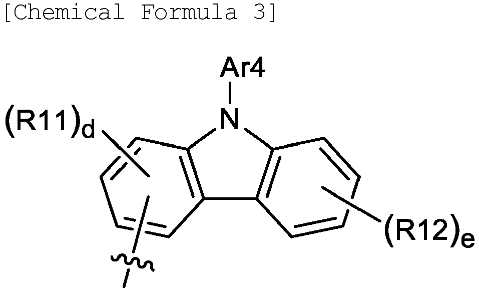
in Chemical Formula 3,
R11 and R12 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; -S1R101R102R103; and -NR101R102, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group;
d is an integer of 0 to 3, and when d is 2 or greater, R11s are the same as or different from each other;
e is an integer of 0 to 4, and when e is 2 or greater, R12s are the same as or different from each other; and
Ar4 is a substituted or unsubstituted C6 to C60 aryl group; or a substituted or unsubstituted C2 to C60 heteroaryl group.

2. The heterocyclic compound of claim 1, wherein the heterocyclic compound represented by Chemical Formula 1 is represented by any one of the following Chemical Formulae 1-1 to 1-3: 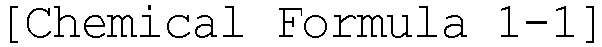 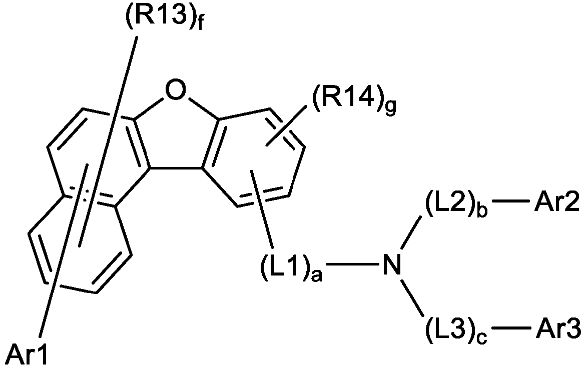 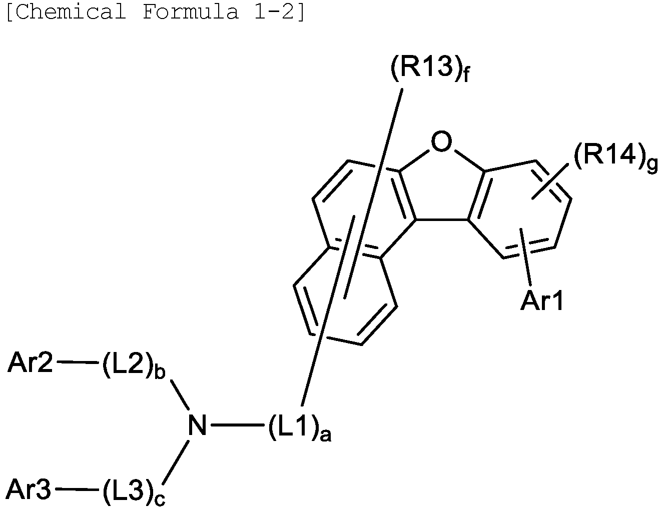 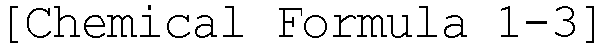 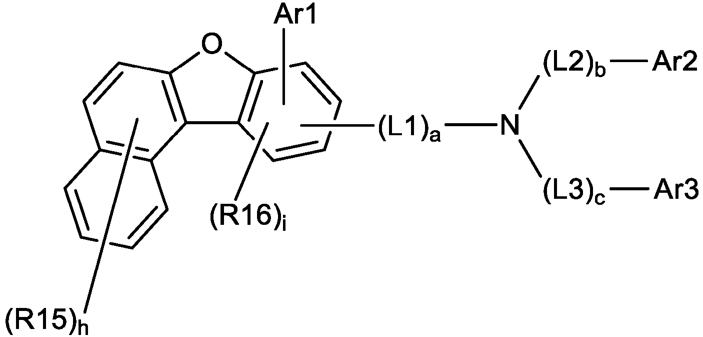
in Chemical Formulae 1-1 to 1-3,
R13 to R16 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; -SiR101R102R103; and -NR101R102, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group;
f is an integer of 0 to 5, and when f is 2 or greater, R13s are the same as or different from each other;
g is an integer of 0 to 3, and when g is 2 or greater, R14s are the same as or different from each other;
h is an integer of 0 to 6, and when h is 2 or greater, R15s are the same as or different from each other;
i is an integer of 0 to 2, and when i is 2 or greater, R16s are the same as or different from each other;
Ar1 has the same definition as in Chemical Formula 1; and L1 to L3, Ar2, Ar3 and a to c have the same definitions as in Chemical Formula 2.

3. The heterocyclic compound of claim 1, wherein Ar4 is a substituted or unsubstituted C6 to C60 aryl group.

4. The heterocyclic compound of claim 1, wherein L1 to L3 are the same as or different from each other, and each independently a direct bond; or a substituted or unsubstituted C6 to C60 arylene group.

5. The heterocyclic compound of claim 1, wherein the heterocyclic compound represented by Chemical Formula 1 does not include deuterium as a substituent, or has a deuterium content of 1% to 100% with respect to a total number of hydrogen atoms and deuterium atoms.

6. The heterocyclic compound of claim 1, wherein the heterocyclic compound represented by Chemical Formula 1 is represented by any one of the following compounds:

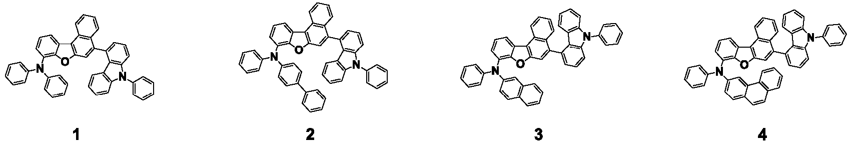

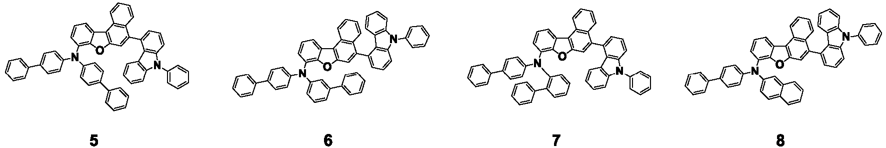

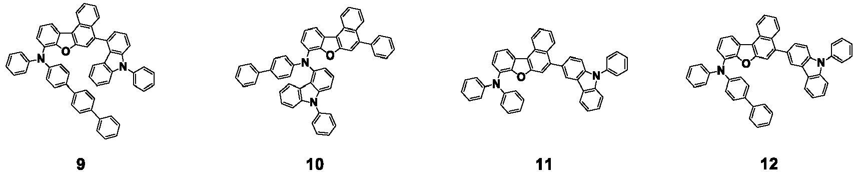

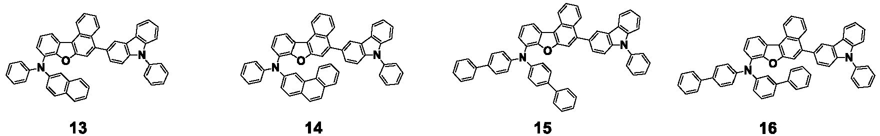

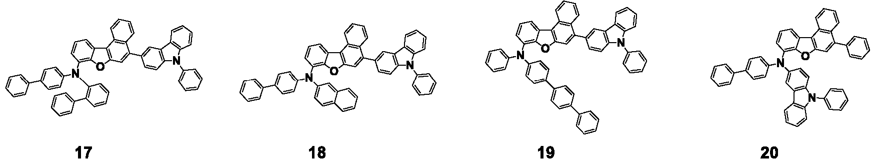

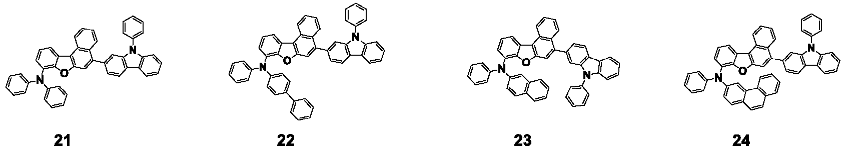

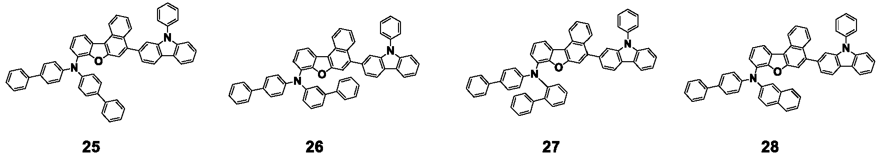

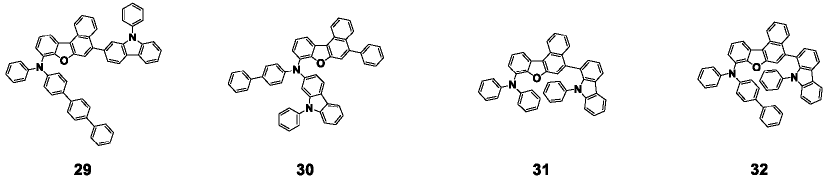

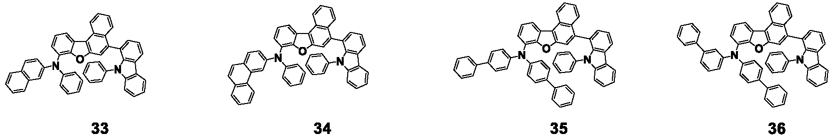

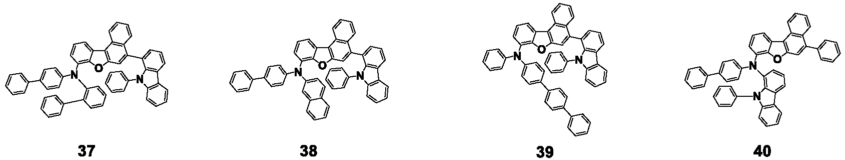

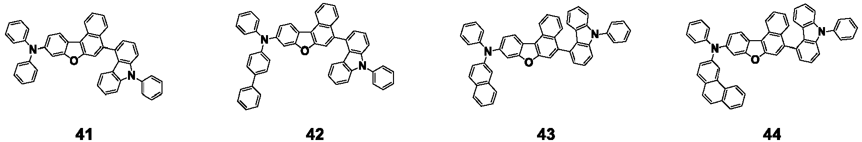

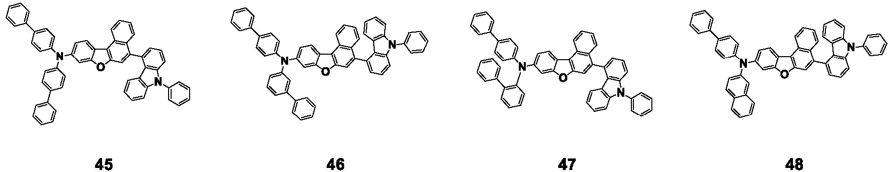

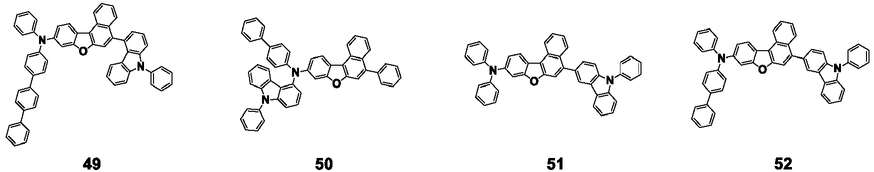

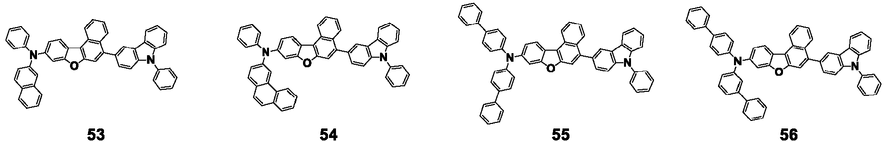

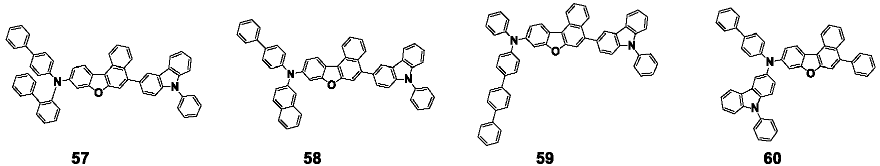

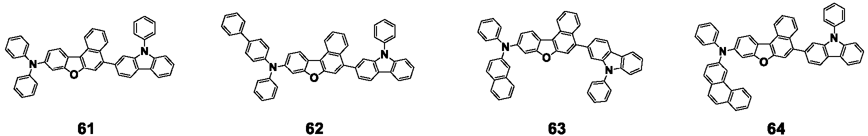

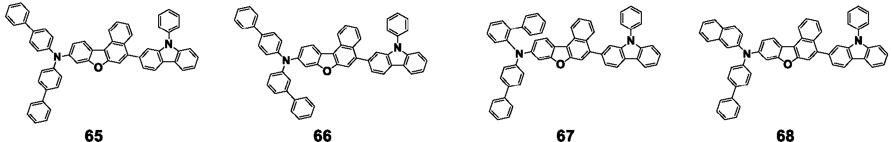

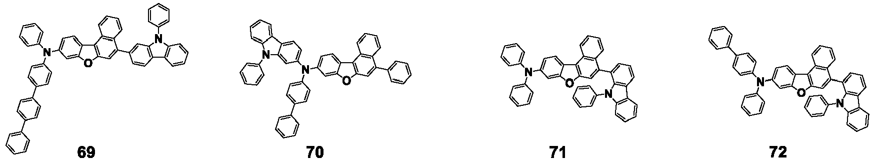

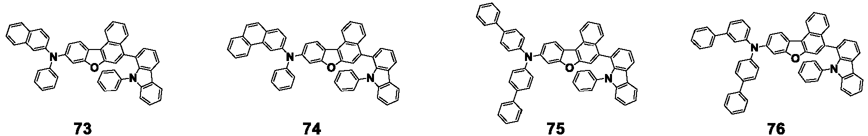

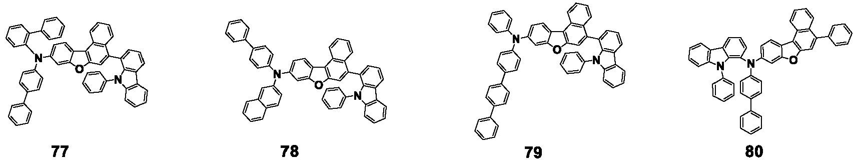

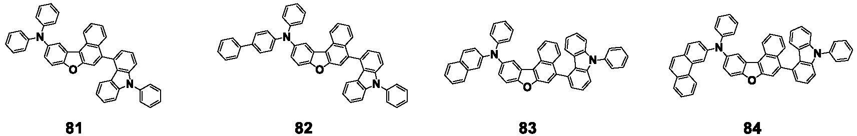

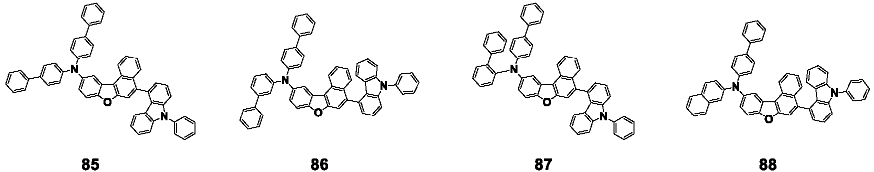

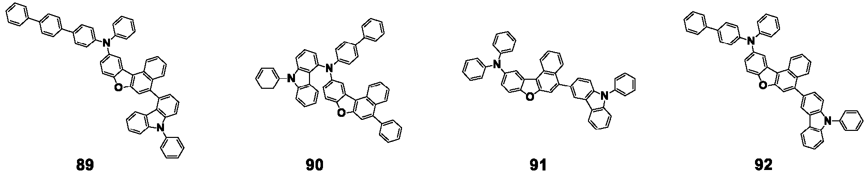

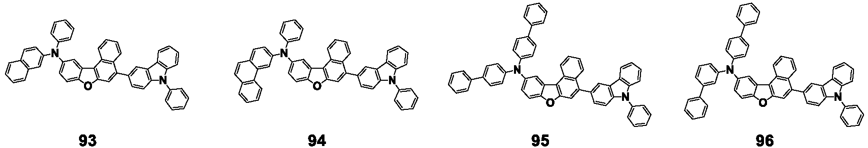

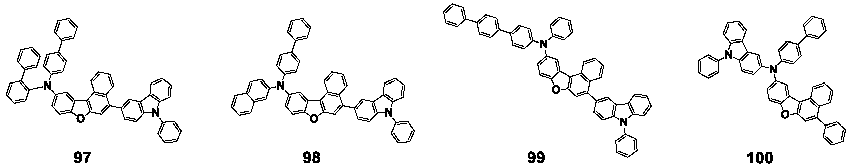

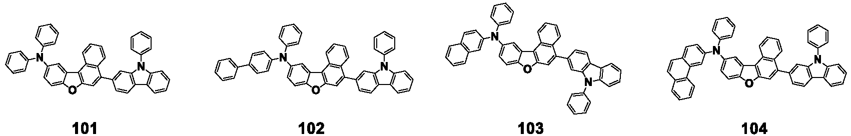

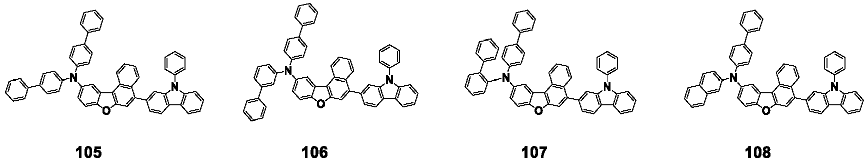

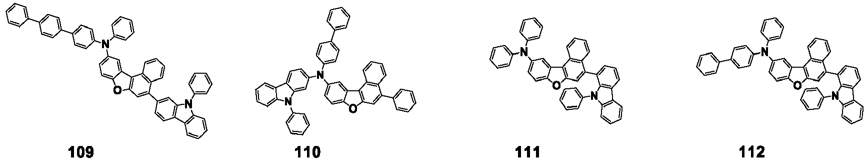

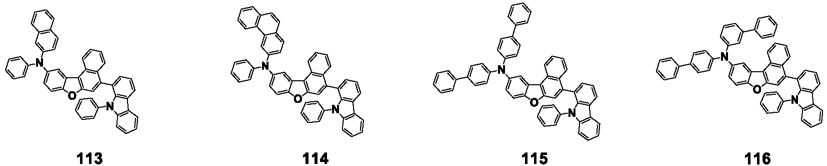

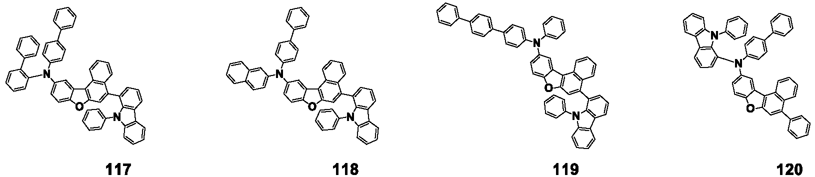

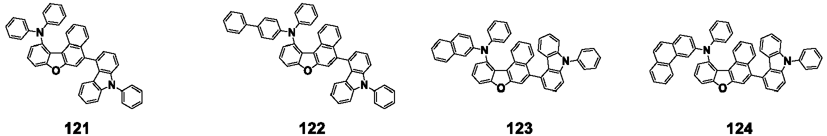

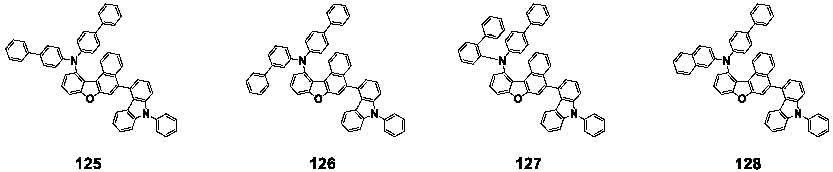

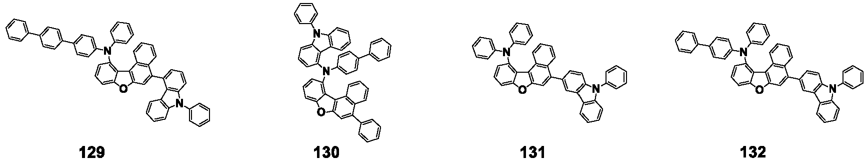

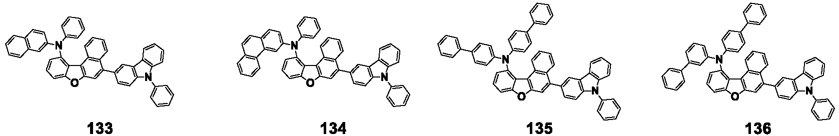

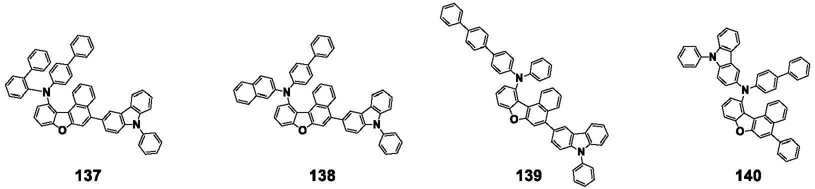

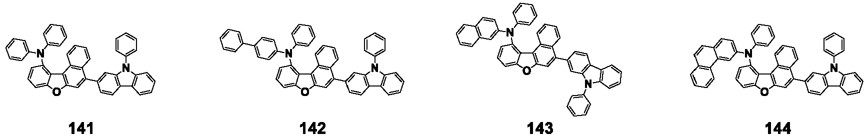

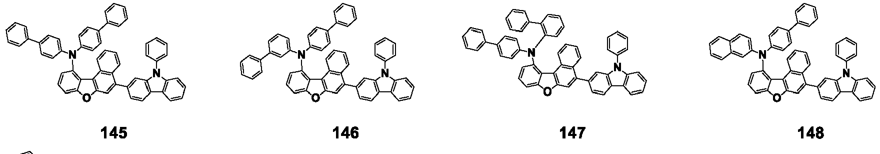

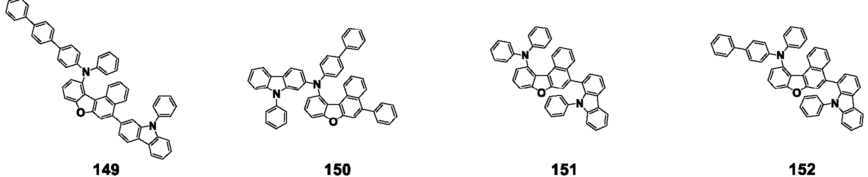

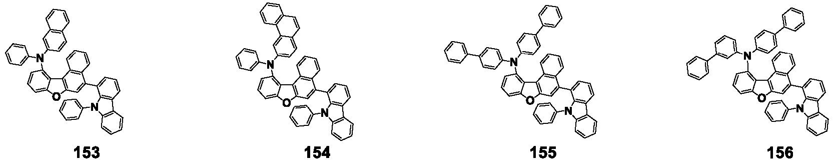

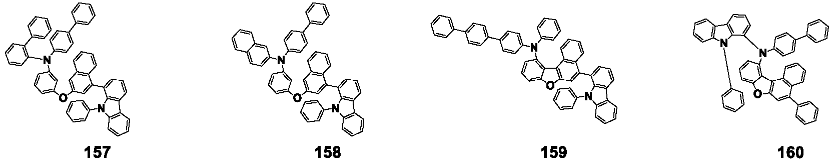

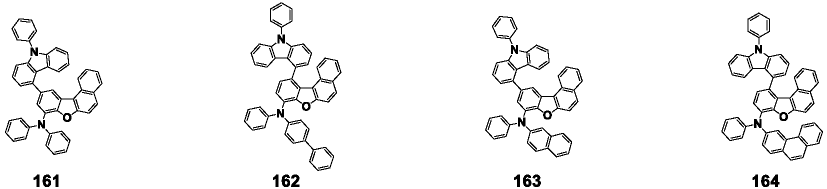

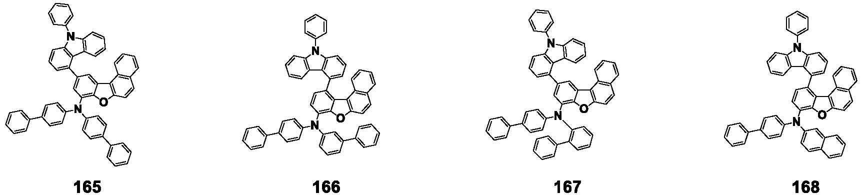

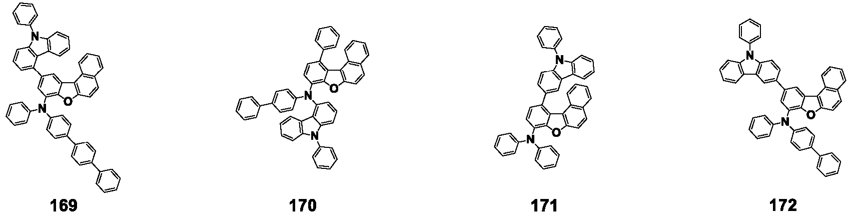

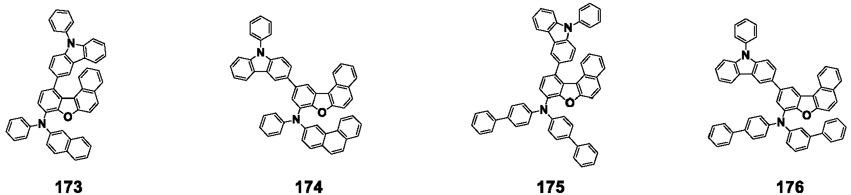

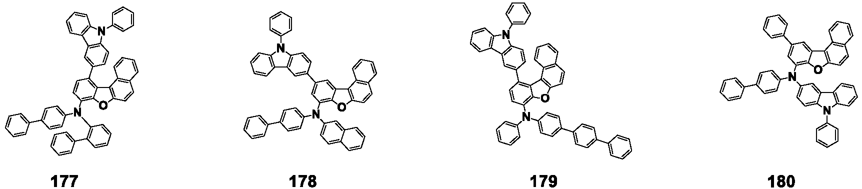

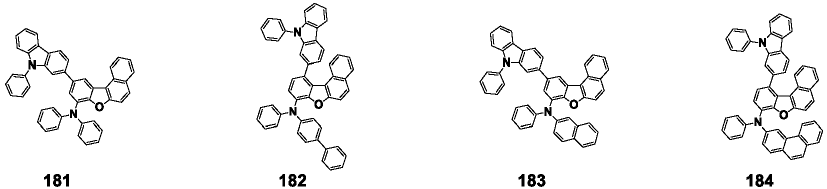

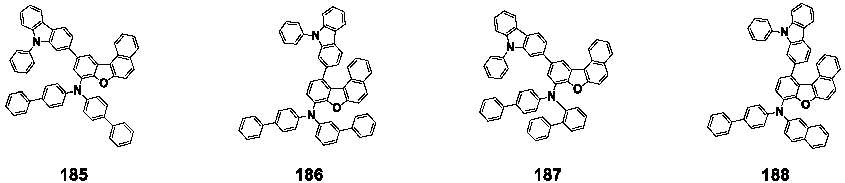

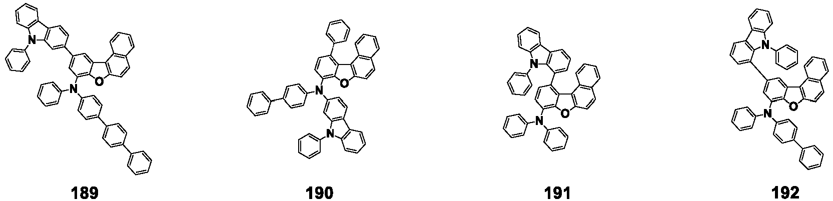

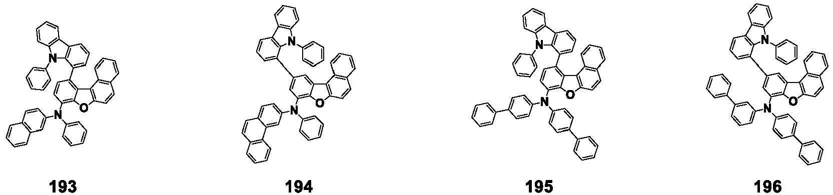

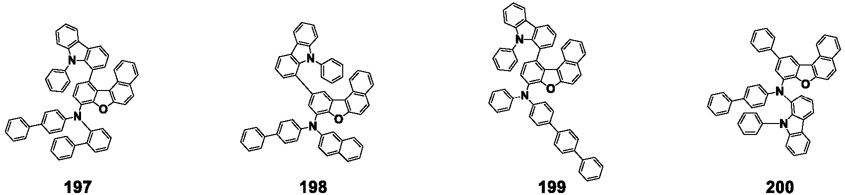

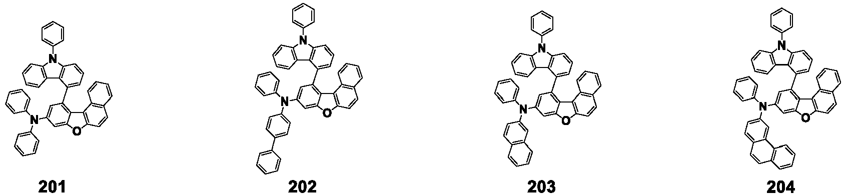

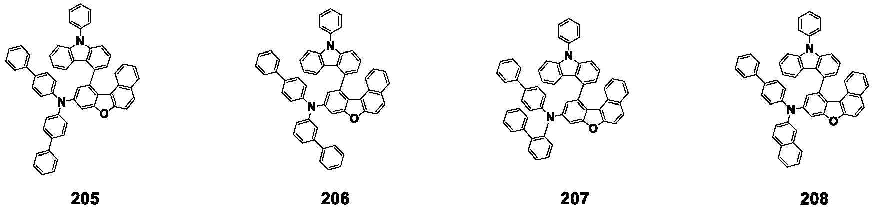

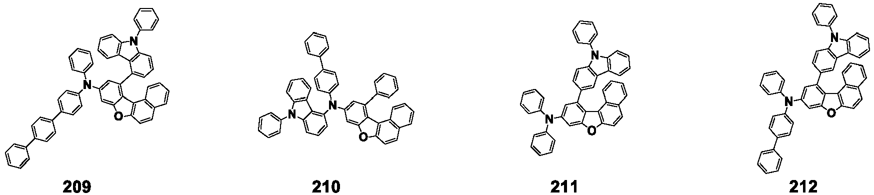

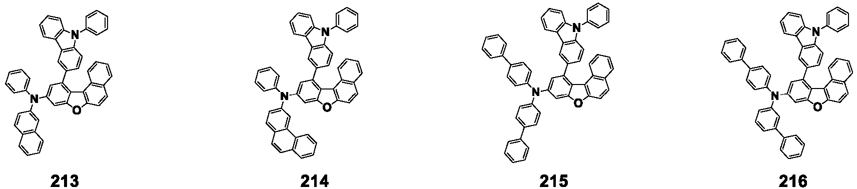

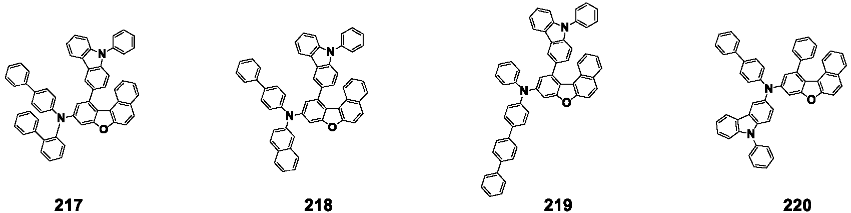

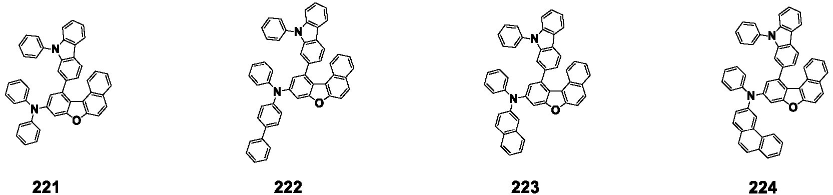

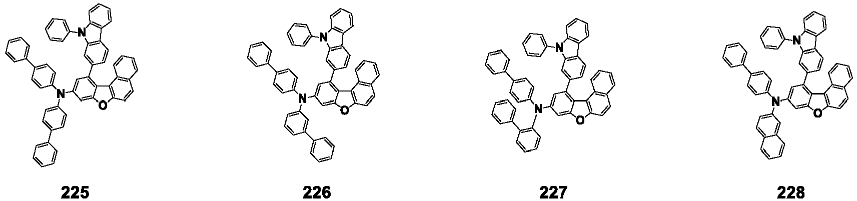

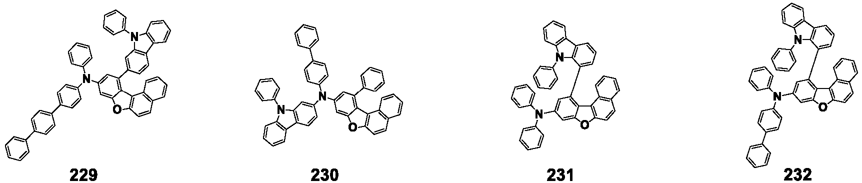

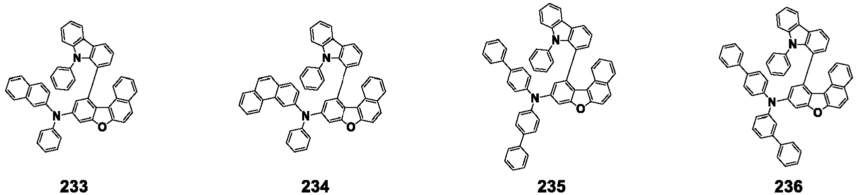

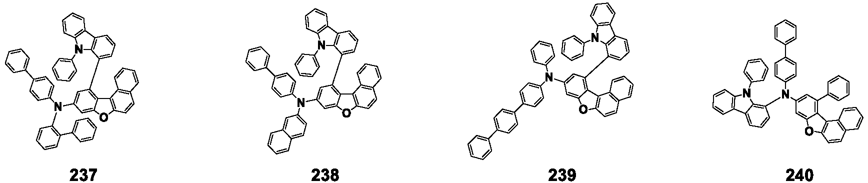

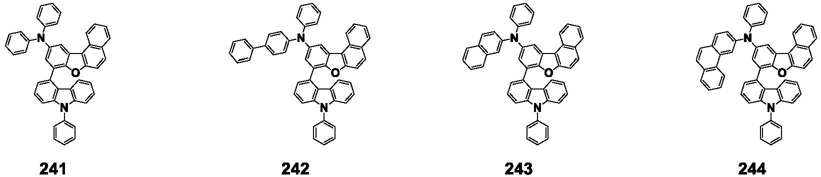

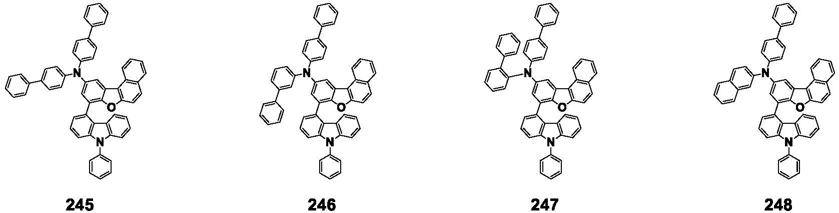

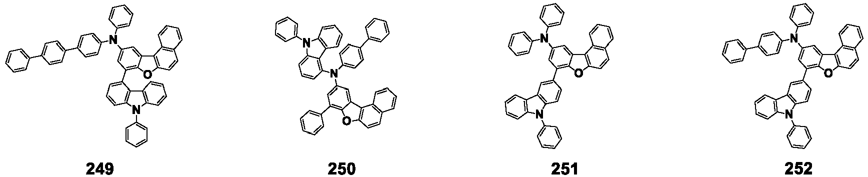

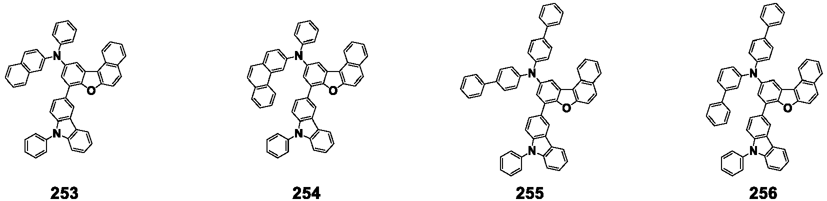

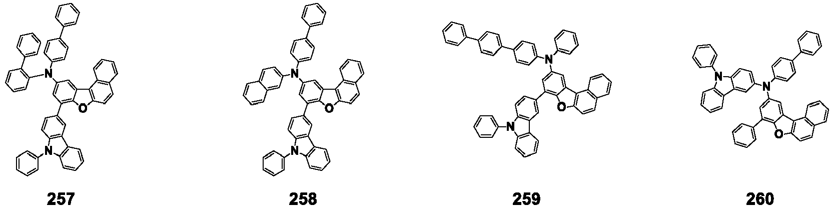

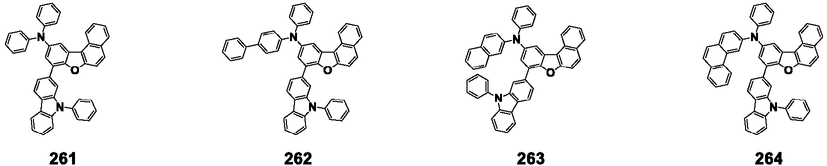

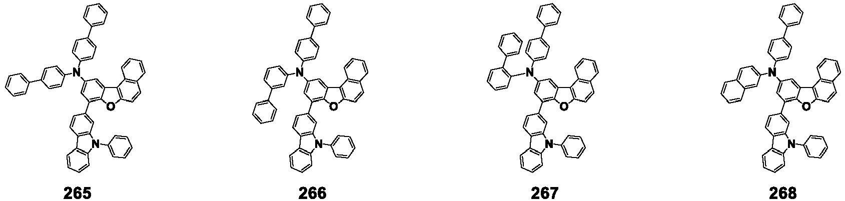

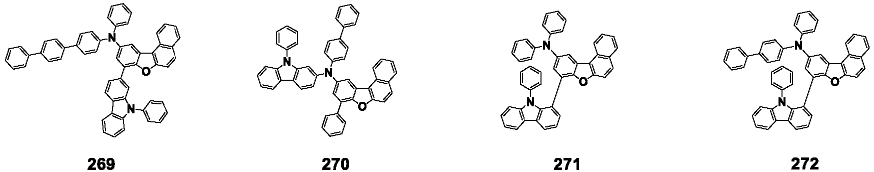

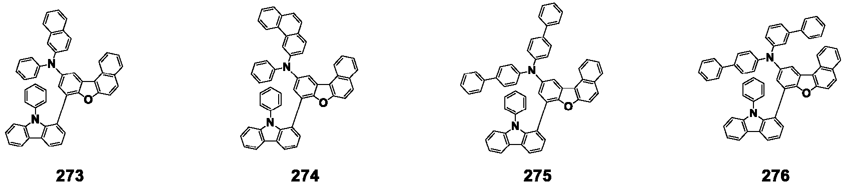

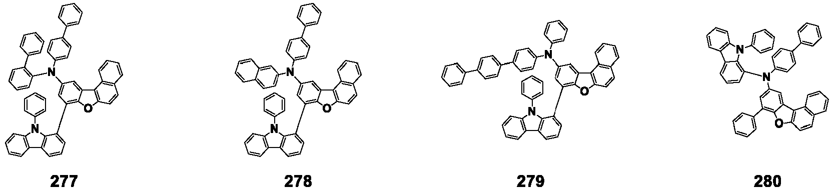

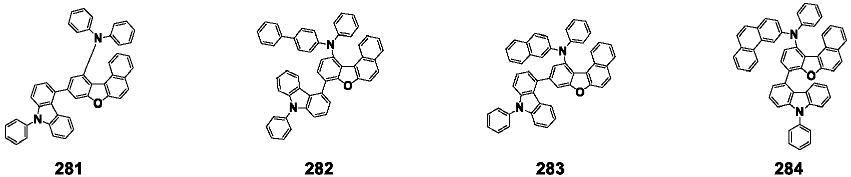

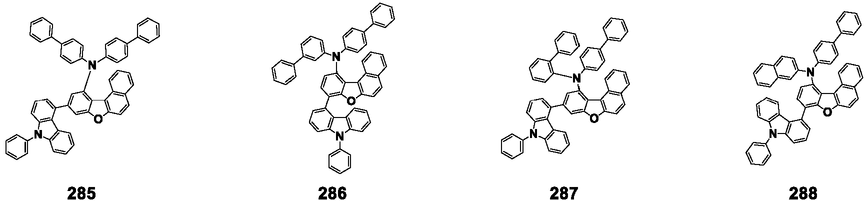

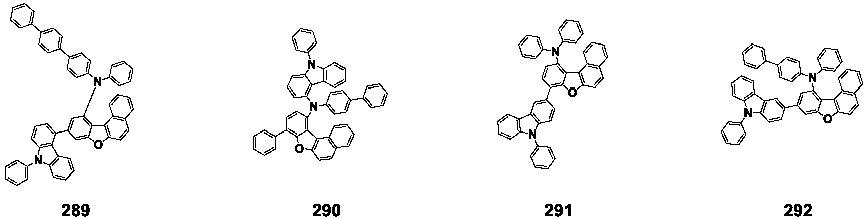

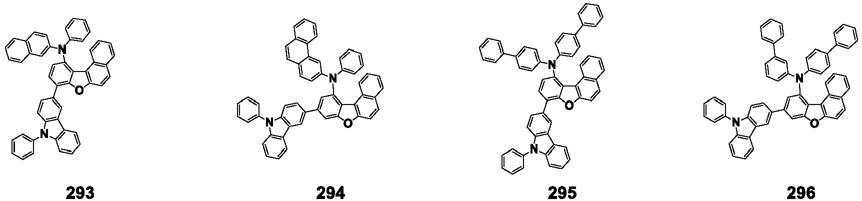

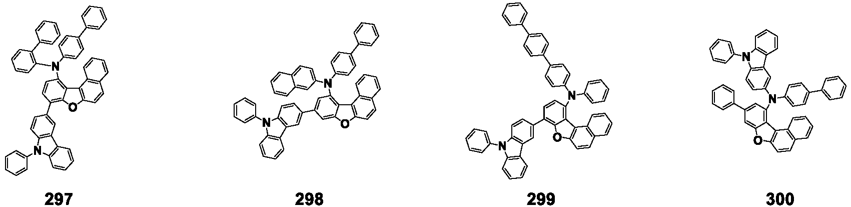

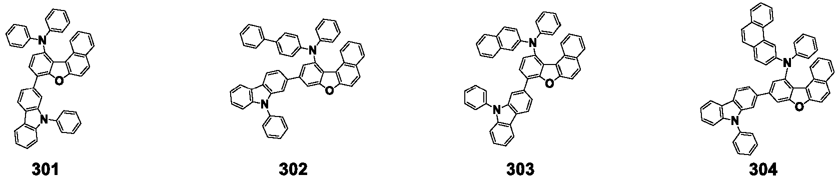

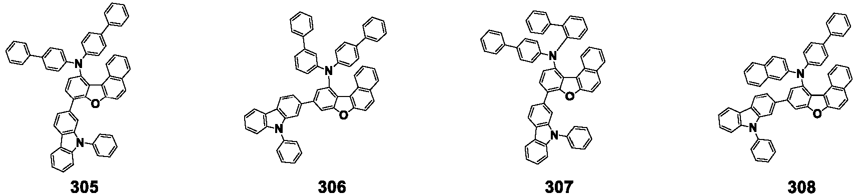

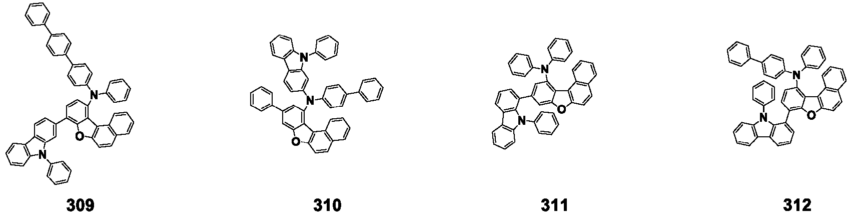

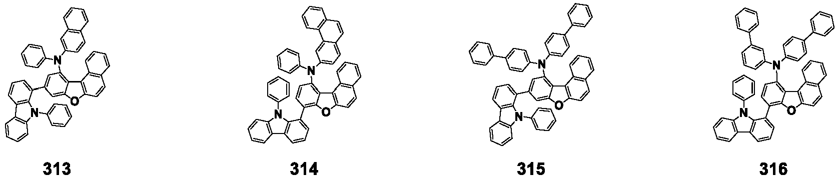

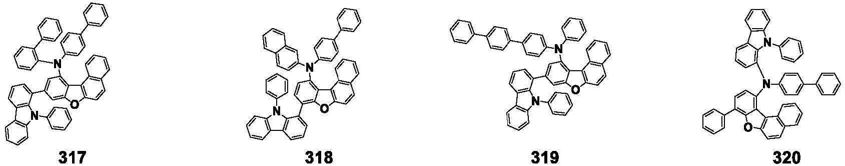

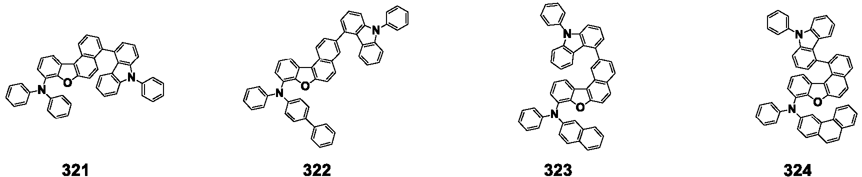

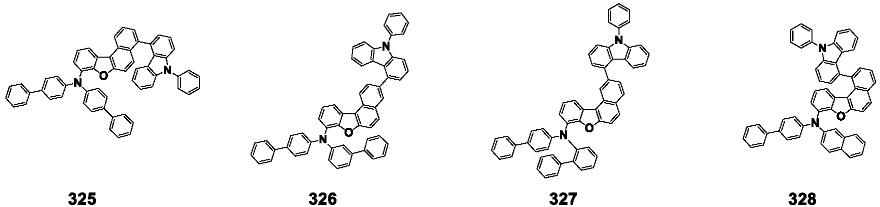

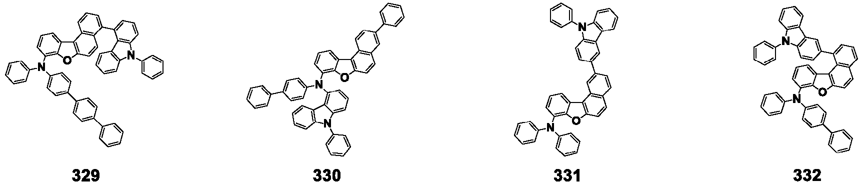

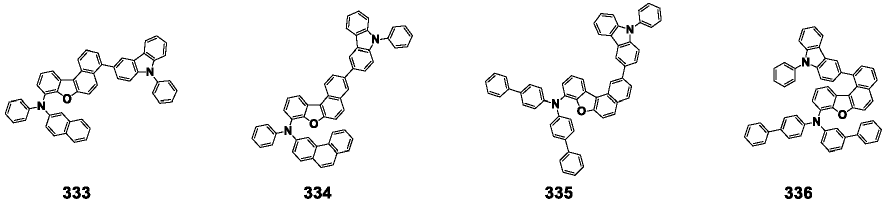

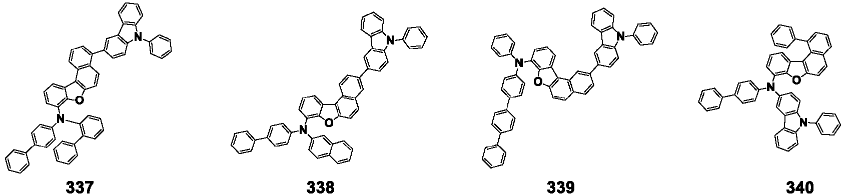

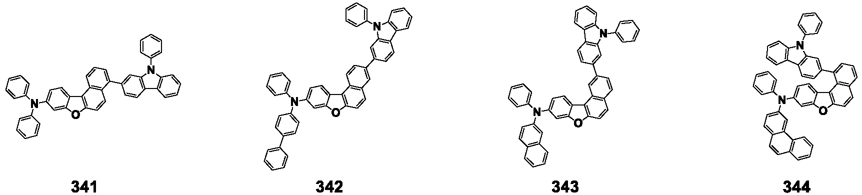

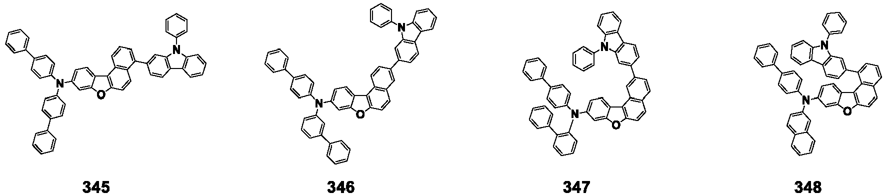

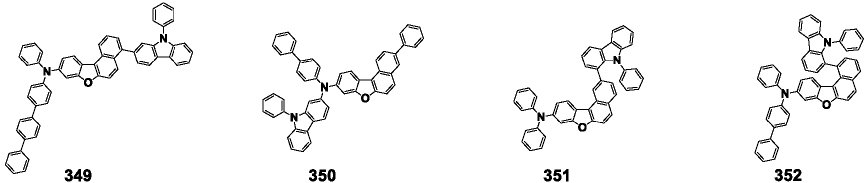

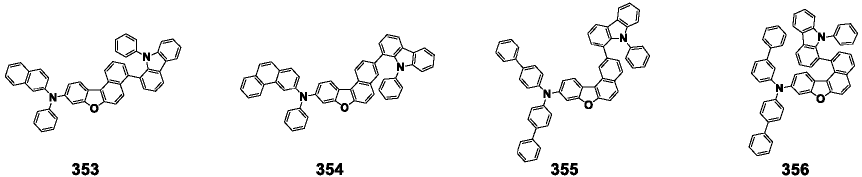

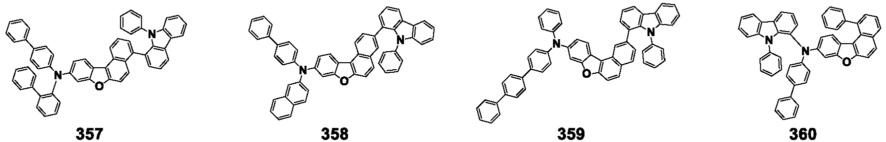

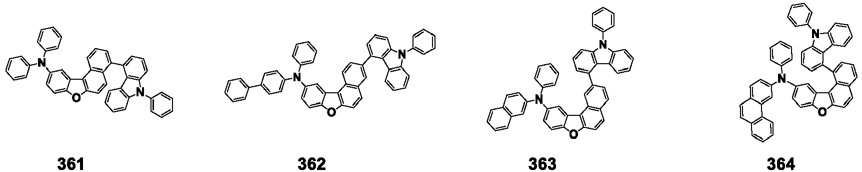

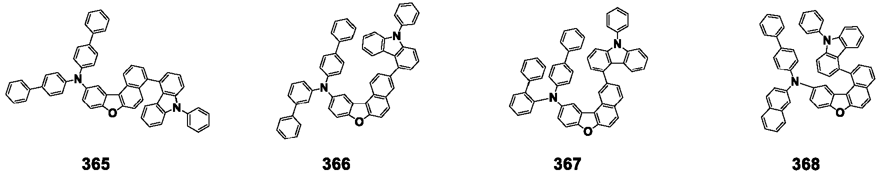

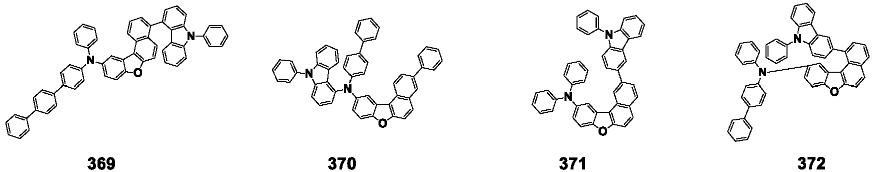

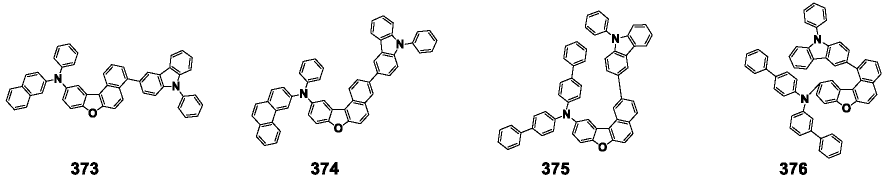

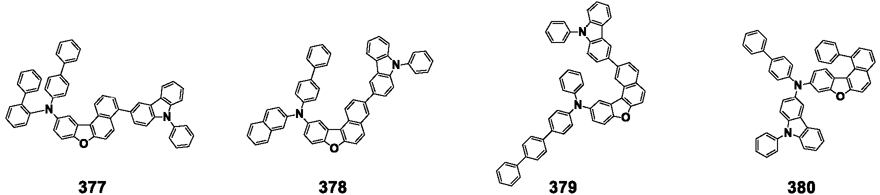

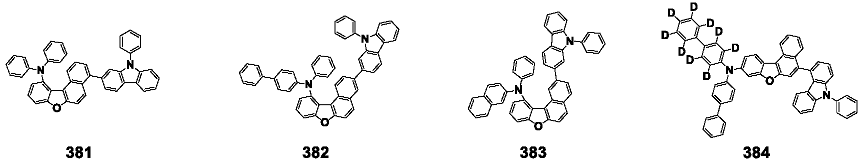

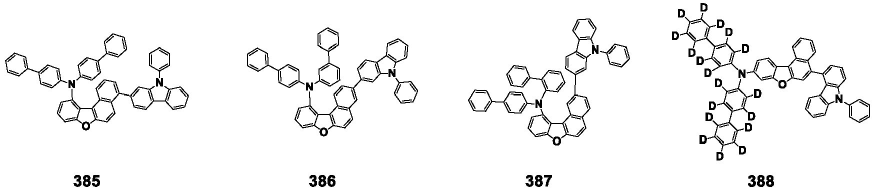

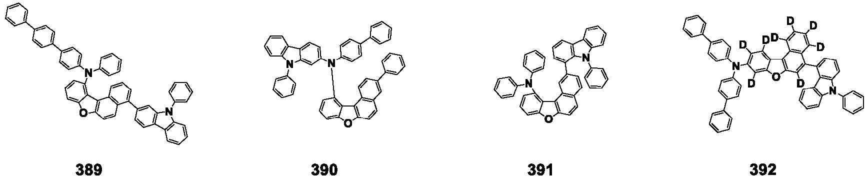

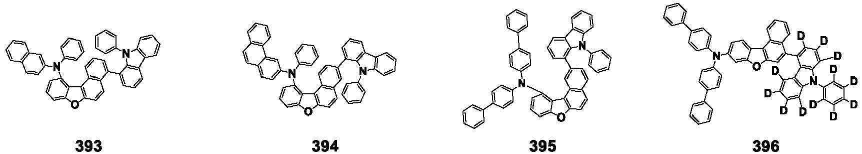

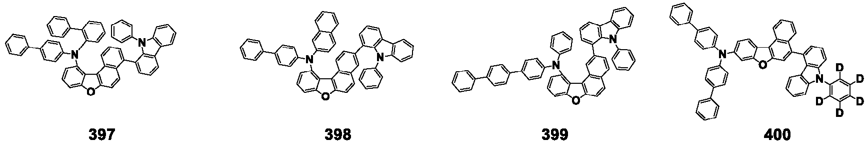

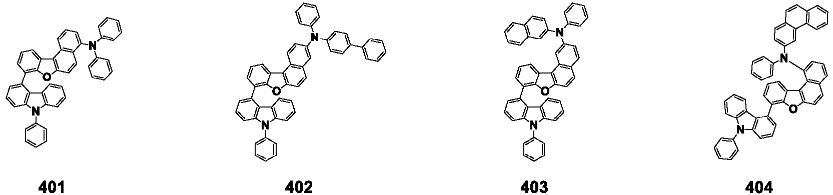

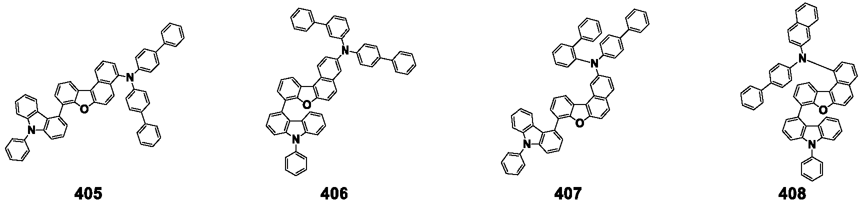

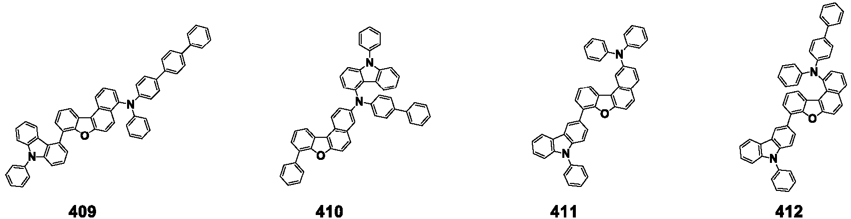

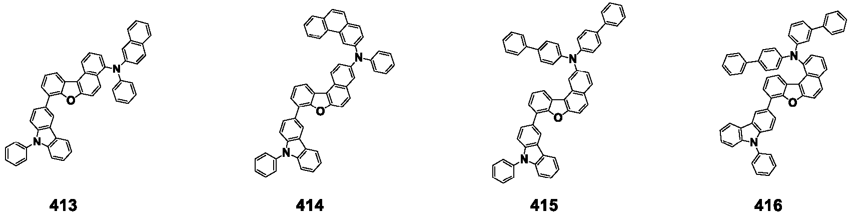

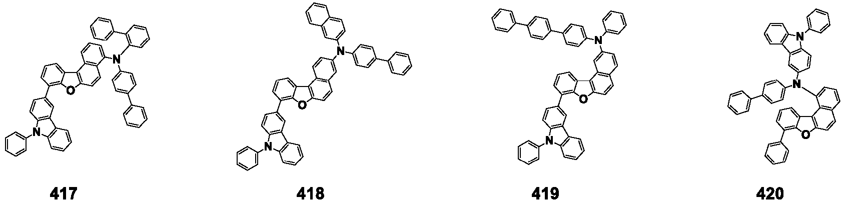

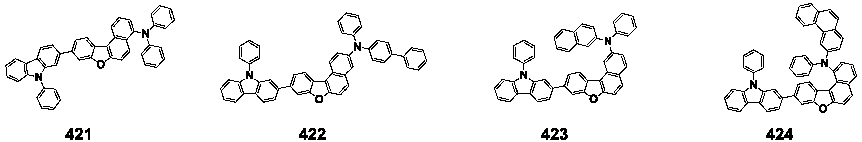

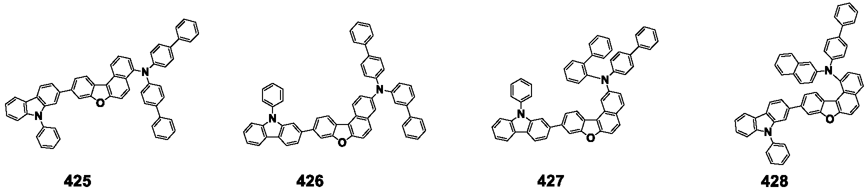

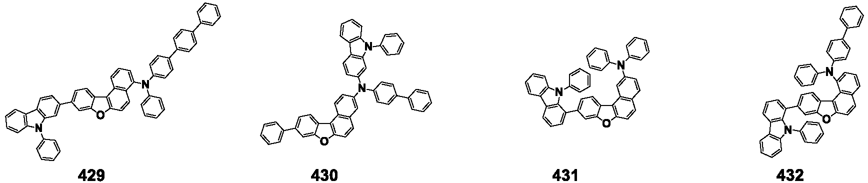

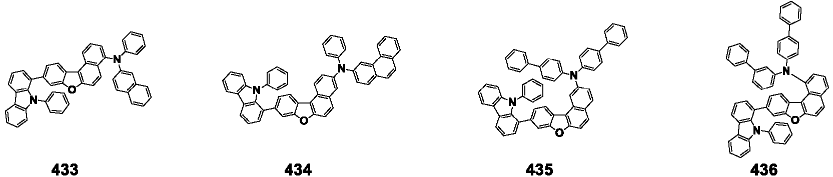

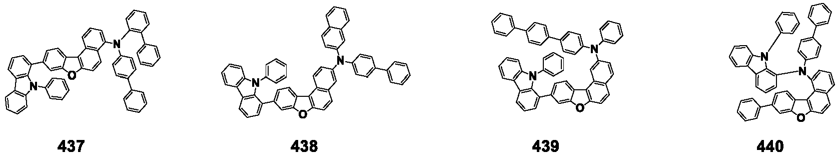

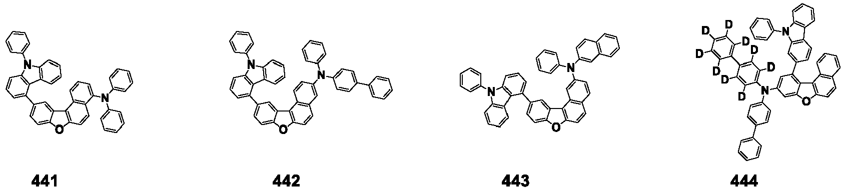

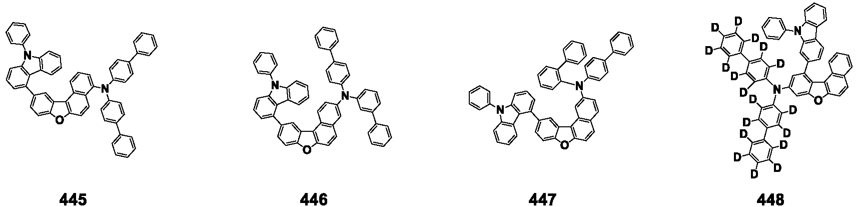

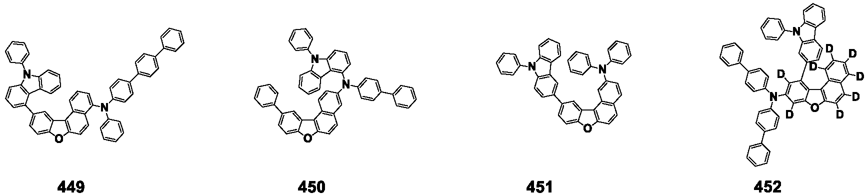

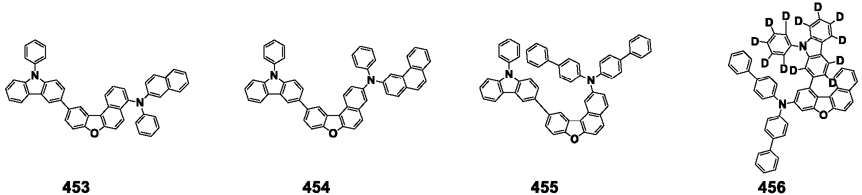

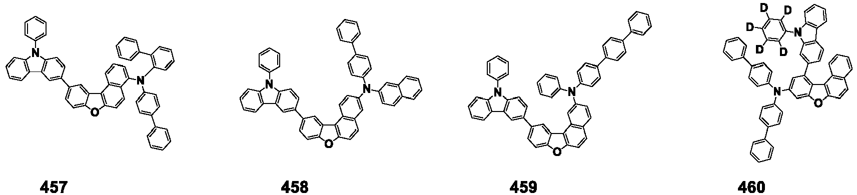

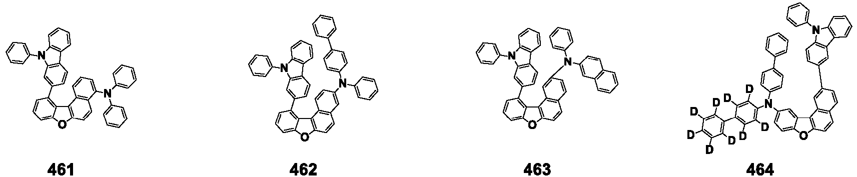

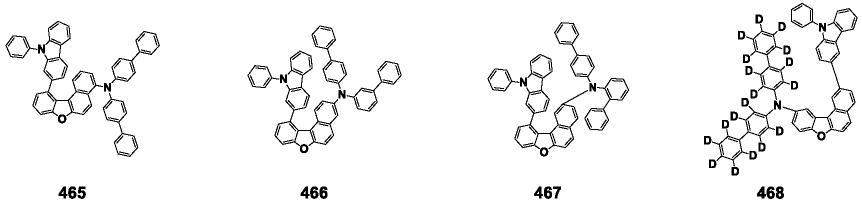

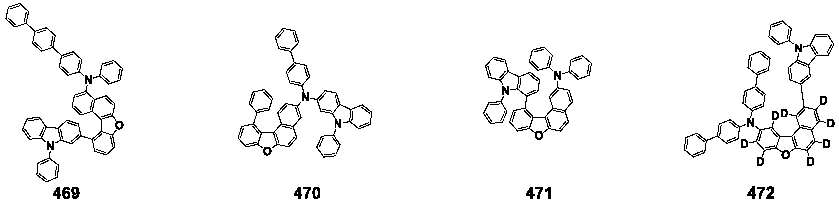

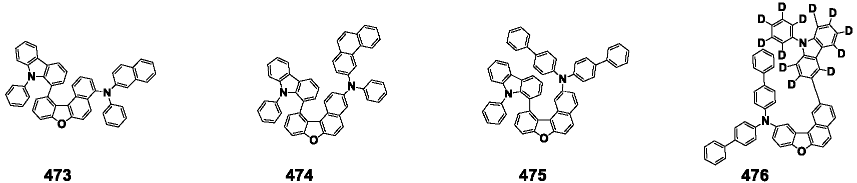

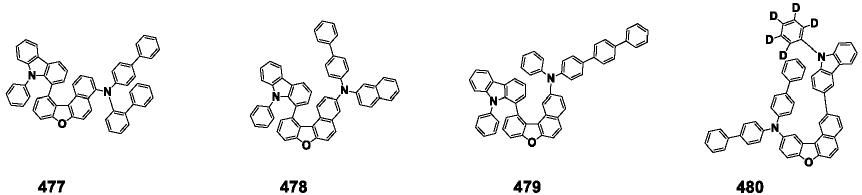

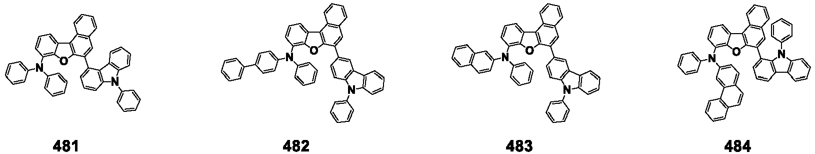

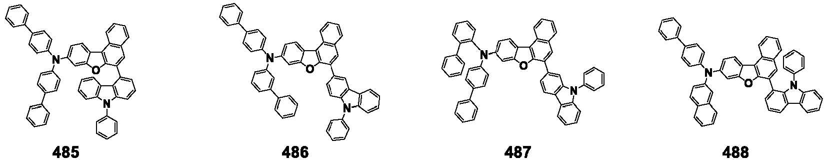

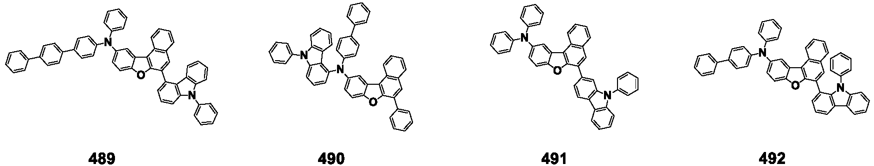

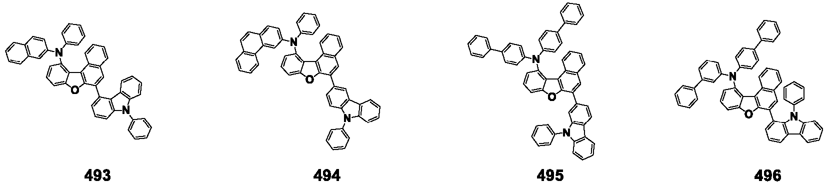

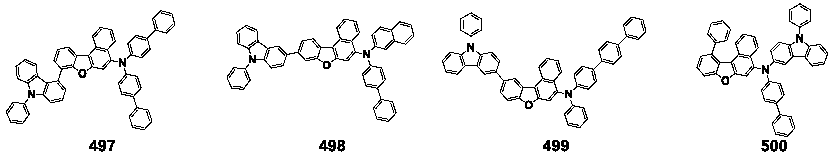

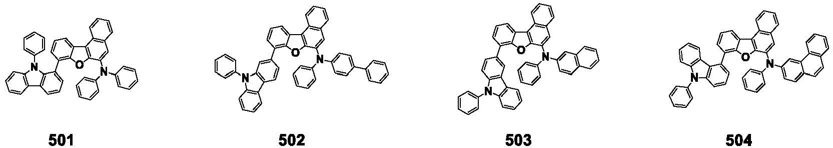

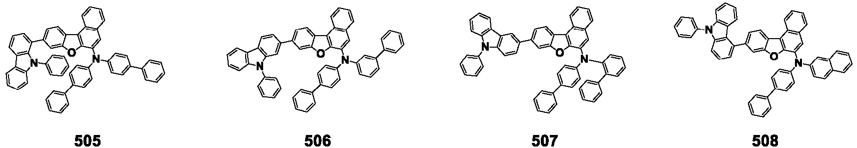

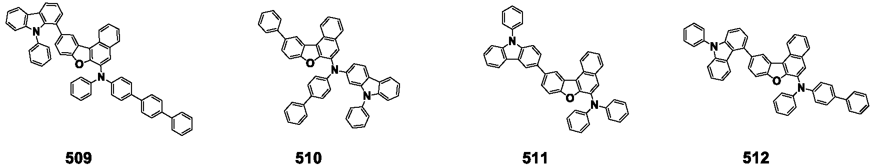

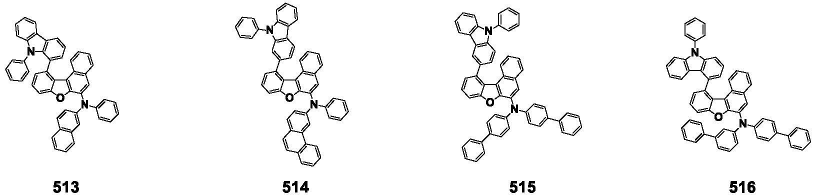

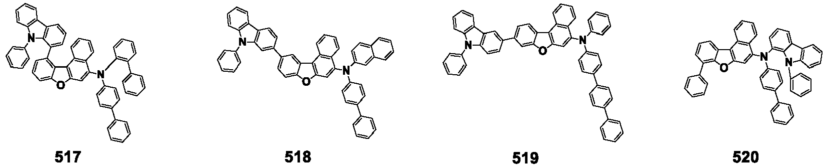

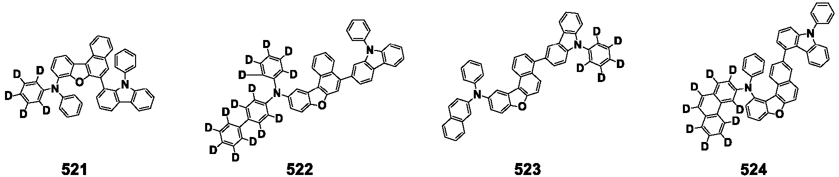

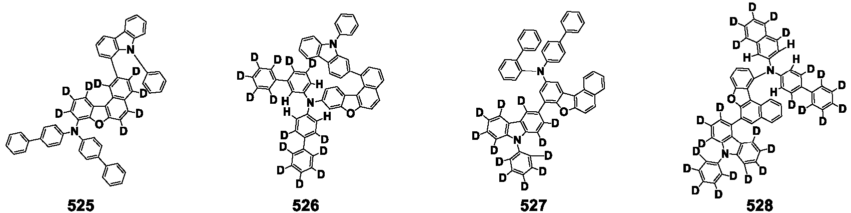

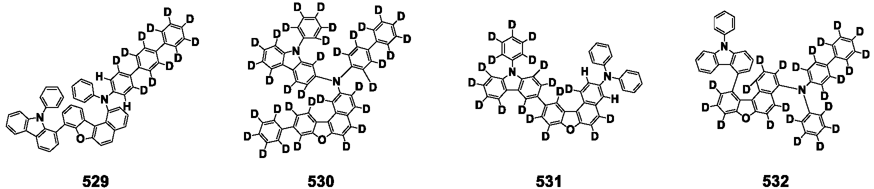

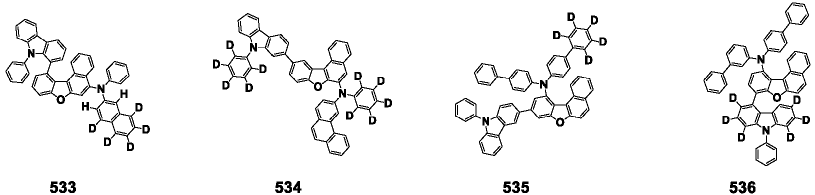

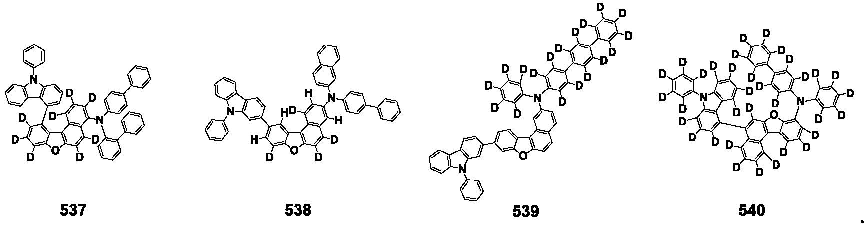

7. An organic light emitting device comprising:
a first electrode;
a second electrode provided opposite to the first electrode; and
one or more organic material layers provided between the first electrode and the second electrode,
wherein at least one of the one or more organic material layers comprise the heterocyclic compound of any one of claims 1 to 6.

8. The organic light emitting device of claim 7, wherein the organic material layer includes a hole transport layer, and the hole transport layer includes the heterocyclic compound.

9. The organic light emitting device of claim 7, wherein the organic material layer includes an electron blocking layer, and the electron blocking layer includes the heterocyclic compound.

10. The organic light emitting device of claim 7, wherein the organic material layer includes a light emitting layer, and the light emitting layer includes the heterocyclic compound.

11. The organic light emitting device of claim 7, wherein the organic material layer includes a light emitting layer, the light emitting layer includes a host material, and the host material includes the heterocyclic compound.

12. The organic light emitting device of claim 7, wherein the organic material layer further includes a heterocyclic compound represented by the following Chemical Formula 4: 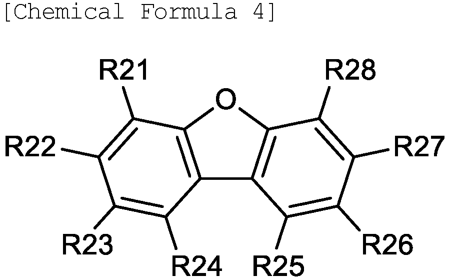
in Chemical Formula 4,
R21 to R28 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; -P(=O)R201R202; -SiR201R202R203; the following Chemical Formula 5; the following Chemical Formula 6; and the following Chemical Formula 7, or two or more groups adjacent to each other bond to each other to form a substituted or unsubstituted C6 to C60 aromatic hydrocarbon ring; or a substituted or unsubstituted C2 to C60 heteroring, and R201, R202 and R203 are the same as or different from each other and each independently a substituted or unsubstituted C1 to C60 alkyl group; a substituted or unsubstituted C6 to C60 aryl group; or a substituted or unsubstituted C2 to C60 heteroaryl group; and
any one of R21 to R28 is the following Chemical Formula 5, and another one is the following Chemical Formula 6; or the following Chemical Formula 7, 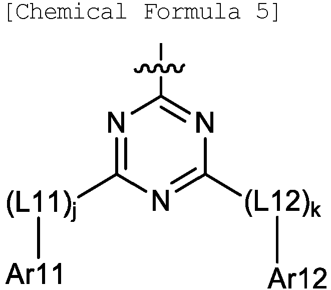 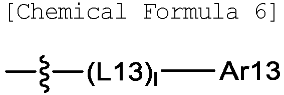 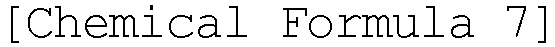 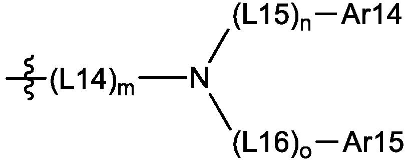
in Chemical Formulae 5 to 7,
L11 to L16 are the same as or different from each other, and each independently a direct bond; a substituted or unsubstituted C6 to C60 arylene group; or a substituted or unsubstituted C2 to C60 heteroarylene group;
j is an integer of 0 to 5, and when j is 2 or greater, L11s are the same as or different from each other;
k is an integer of 0 to 5, and when k is 2 or greater, L12s are the same as or different from each other;
l is an integer of 0 to 5, and when l is 2 or greater, L13s are the same as or different from each other;
m is an integer of 0 to 5, and when m is 2 or greater, L14s are the same as or different from each other;
n is an integer of 0 to 5, and when n is 2 or greater, L15s are the same as or different from each other;
o is an integer of 0 to 5, and when o is 2 or greater, L16s are the same as or different from each other; and
Ar11 to Ar15 are the same as or different from each other, and each independently a substituted or unsubstituted C6 to C60 aryl group; or a substituted or unsubstituted C2 to C60 heteroaryl group.

13. The organic light emitting device of claim 12, wherein the heterocyclic compound represented by Chemical Formula 4 is represented by any one of the following Chemical Formulae 4-1 to 4-4: 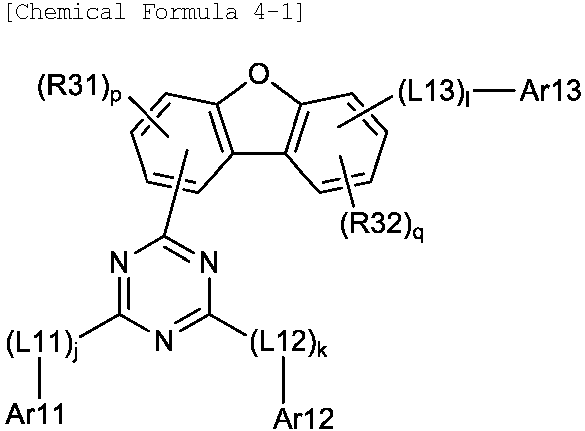 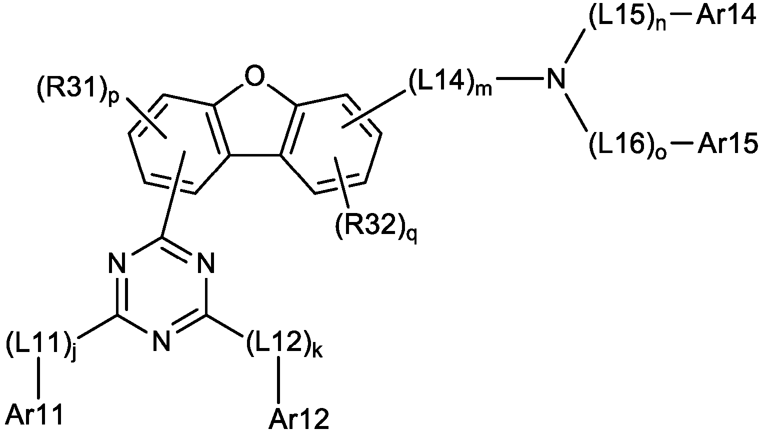 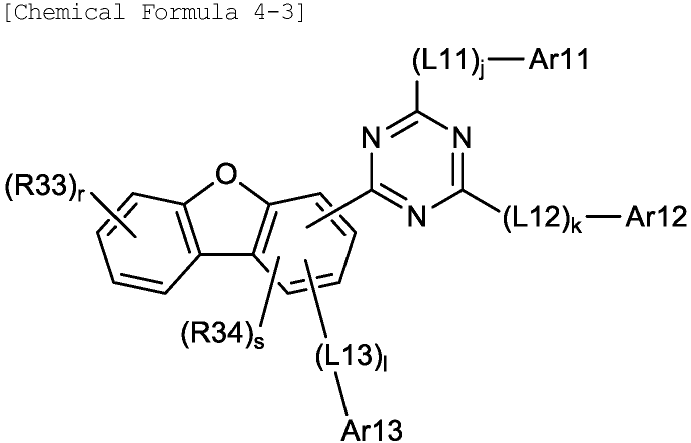 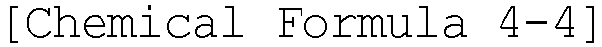 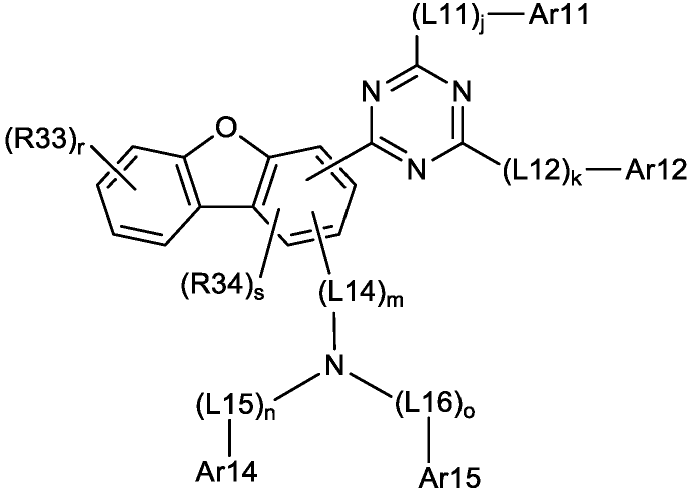
in Chemical Formulae 4-1 to 4-4,
R31 to R34 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R201R202; -SiR201R202R203; and -NR201R202, or two or more groups adjacent to each other bond to each other to form a substituted or unsubstituted C6 to C60 aromatic hydrocarbon ring; or a substituted or unsubstituted C2 to C60 heteroring, and R201, R202 and R203 are the same as or different from each other and each independently a substituted or unsubstituted C1 to C60 alkyl group; a substituted or unsubstituted C6 to C60 aryl group; or a substituted or unsubstituted C2 to C60 heteroaryl group;
p is an integer of 0 to 3, and when p is 2 or greater, R31s are the same as or different from each other;
q is an integer of 0 to 3, and when q is 2 or greater, R32s are the same as or different from each other;
r is an integer of 0 to 4, and when r is 2 or greater, R33s are the same as or different from each other;
s is an integer of 0 to 2, and when s is 2 or greater, R34s are the same as or different from each other;
L11, L12, Ar11, Ar12, j and k have the same definitions as in Chemical Formula 5;
L13, Ar13 and l have the same definitions as in Chemical Formula 6; and
L14 to L16, Ar14, Ar15, m, n and o have the same definitions as in Chemical Formula 7.

14. The organic light emitting device of claim 12, wherein the heterocyclic compound represented by Chemical Formula 4 does not include deuterium as a substituent, or has a deuterium content of 1% to 100% with respect to a total number of hydrogen atoms and deuterium atoms.

15. The organic light emitting device of claim 12, wherein the heterocyclic compound represented by Chemical Formula 4 is represented by any one of the following compounds:

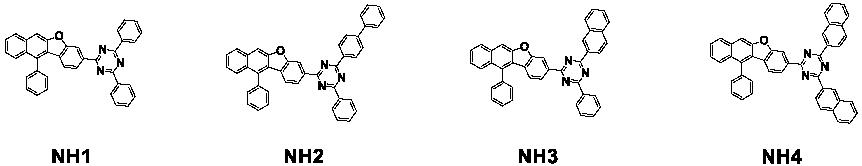

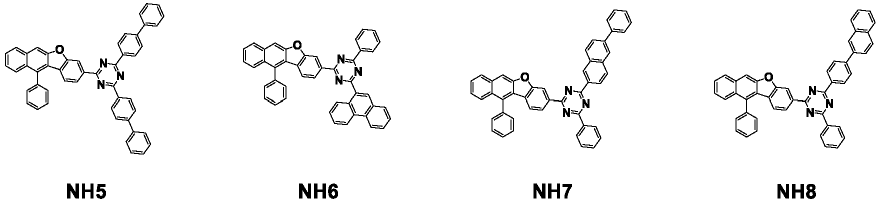

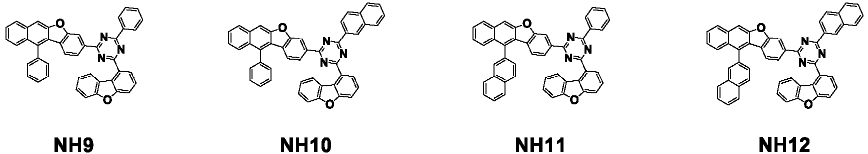

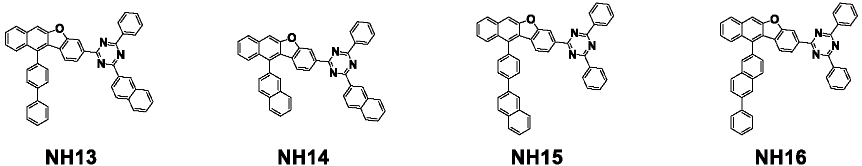

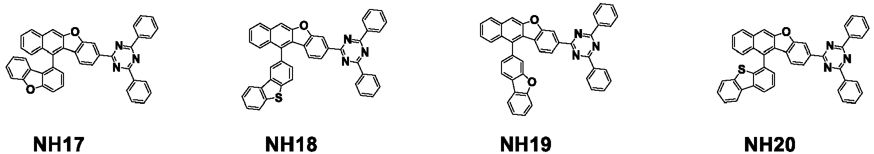

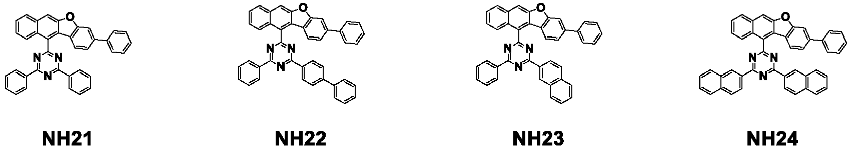

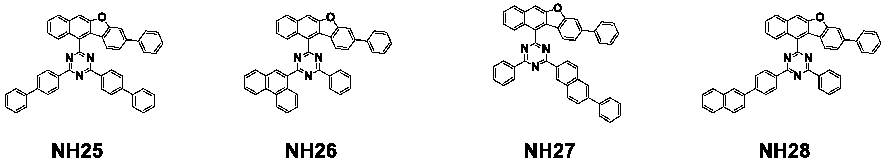

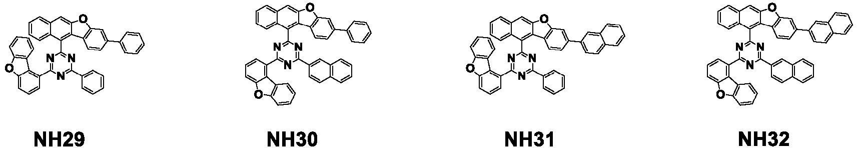

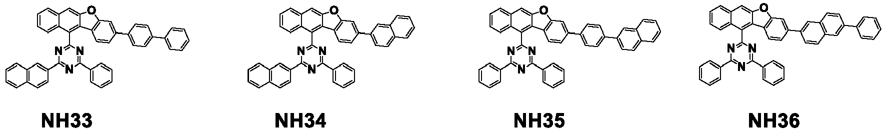

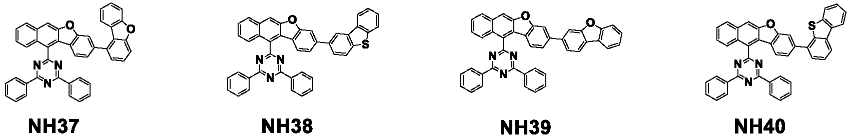

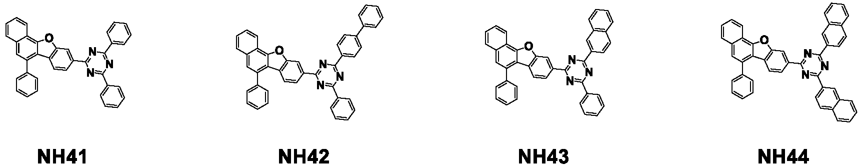

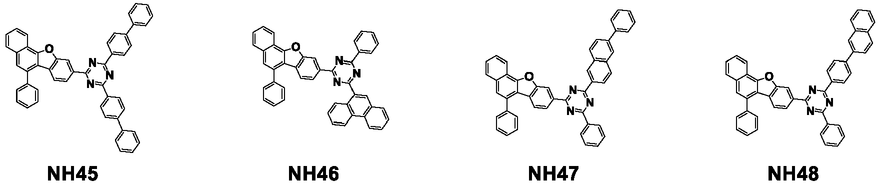

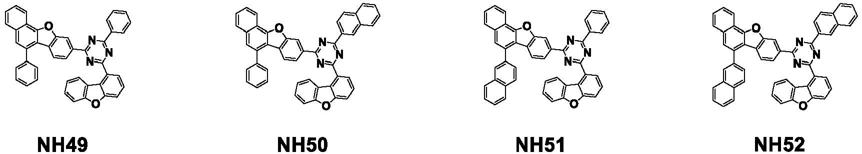

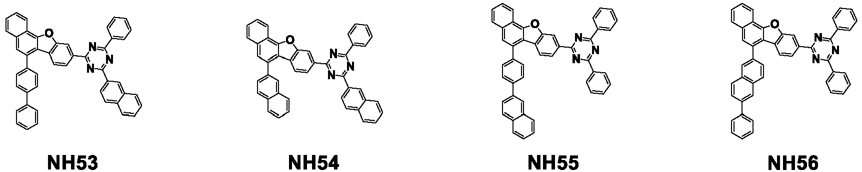

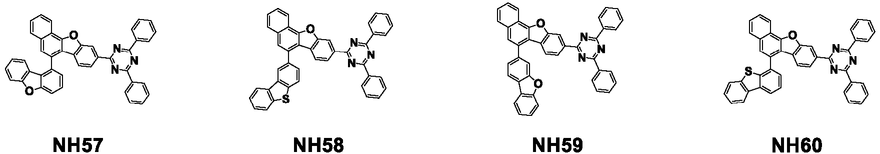

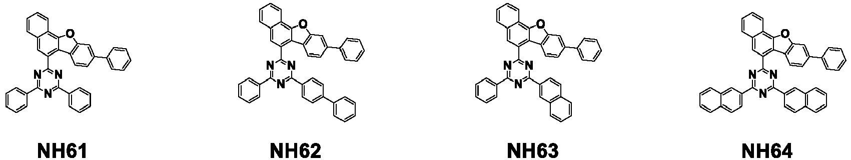

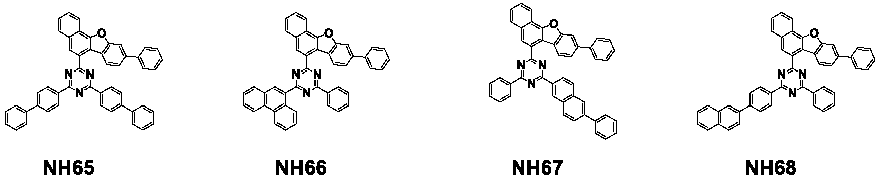

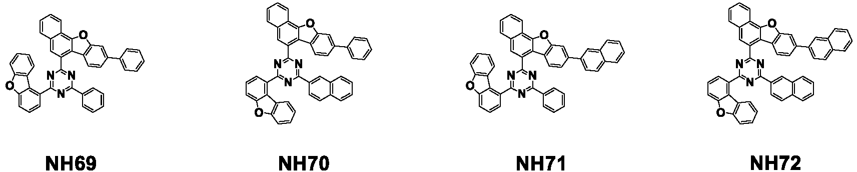

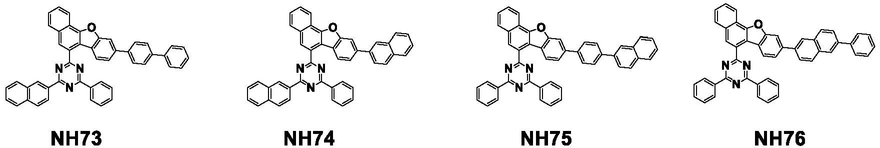

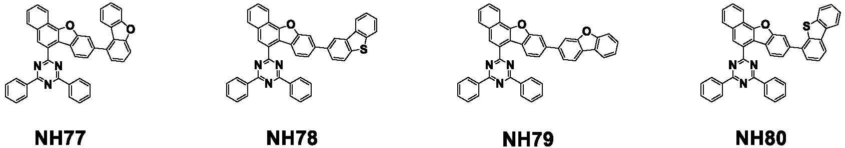

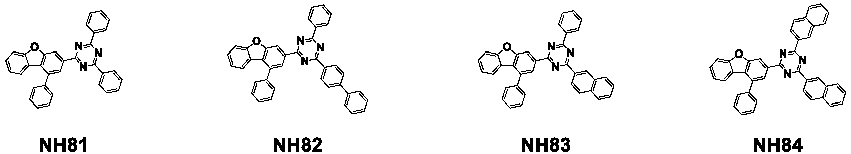

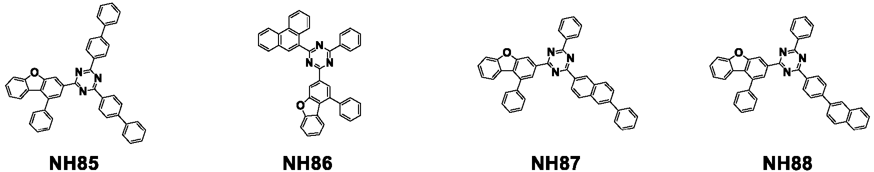

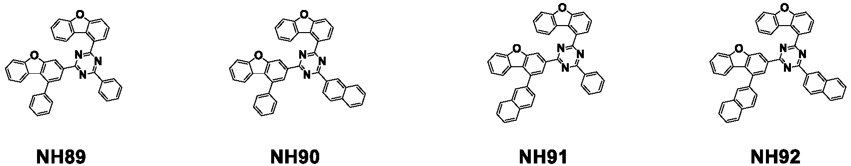

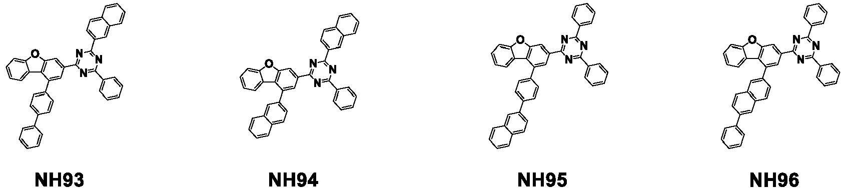

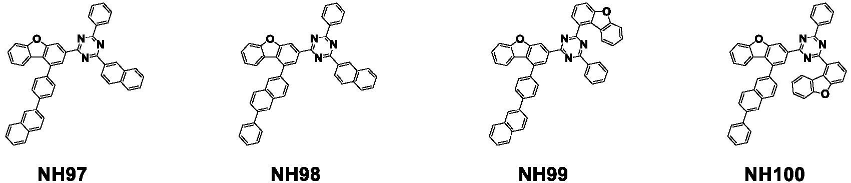

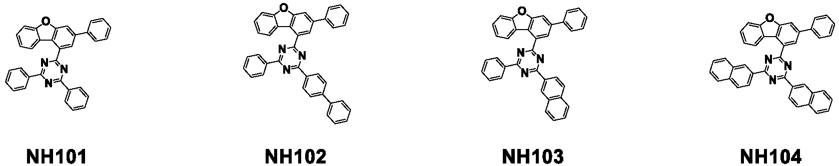

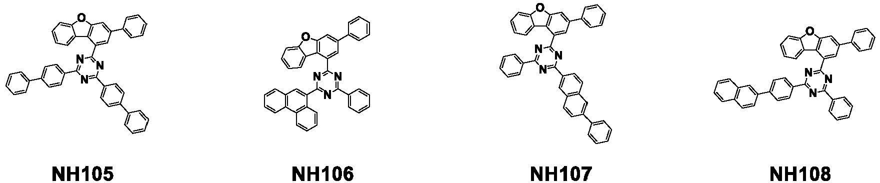

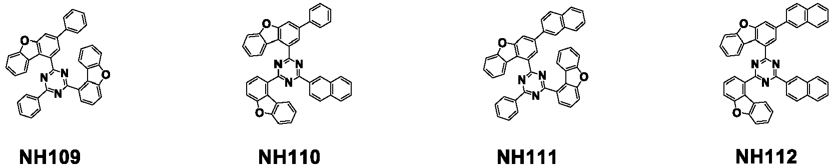

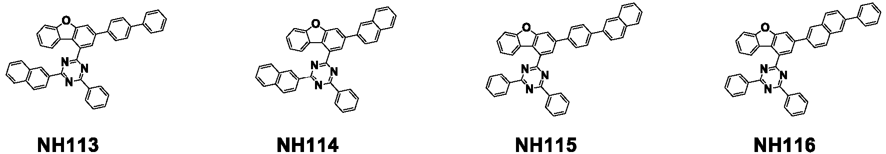

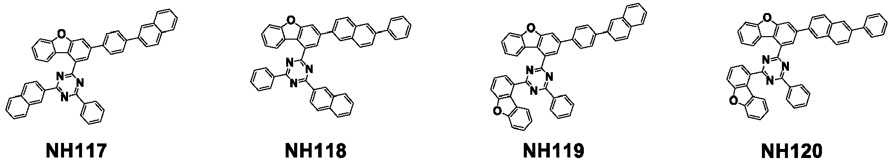

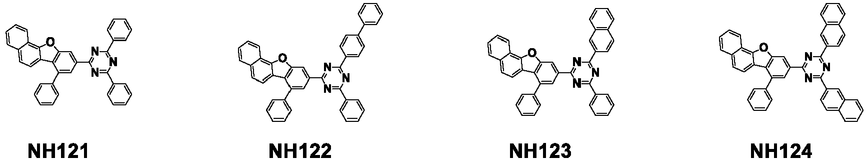

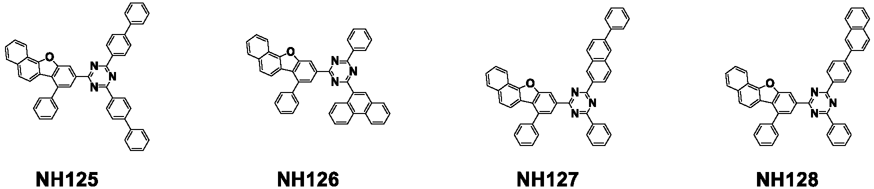

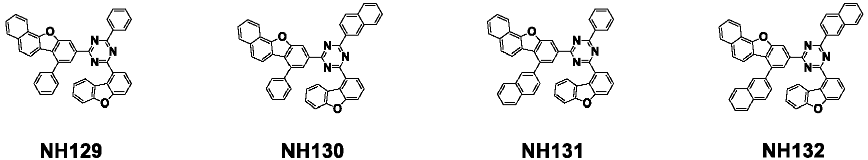

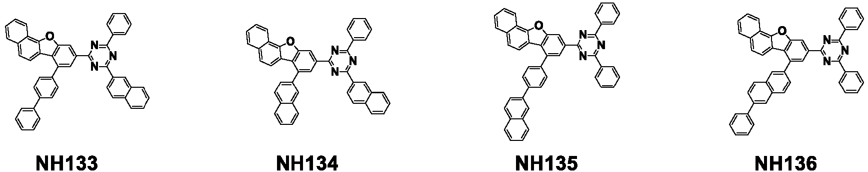

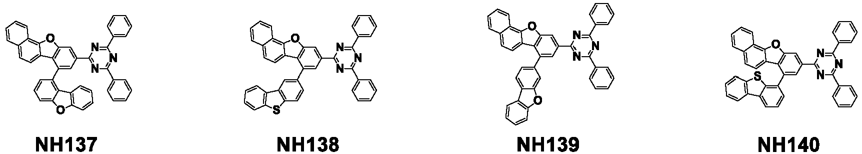

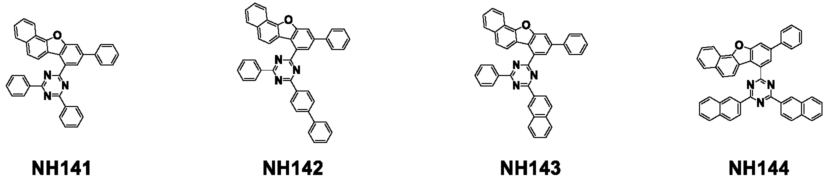

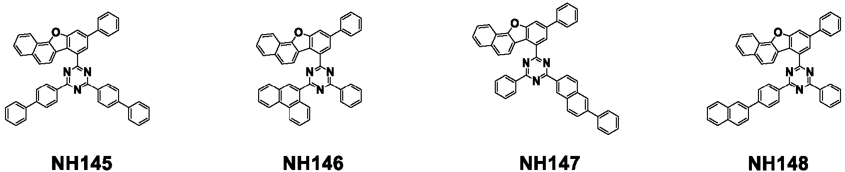

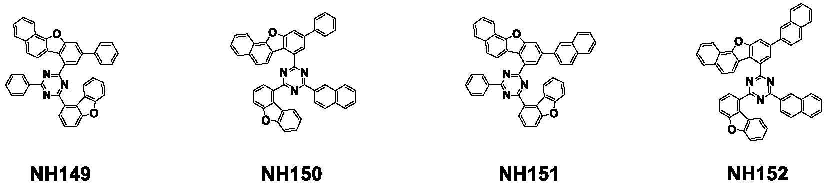

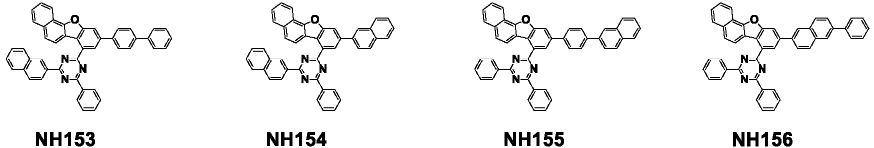

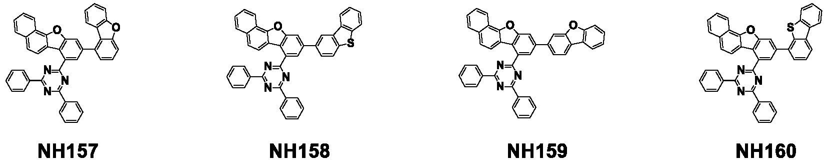

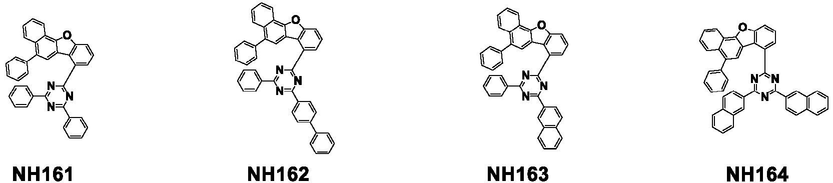

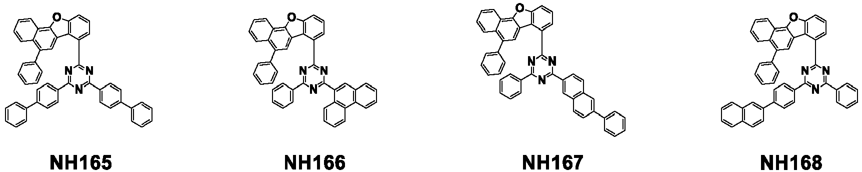

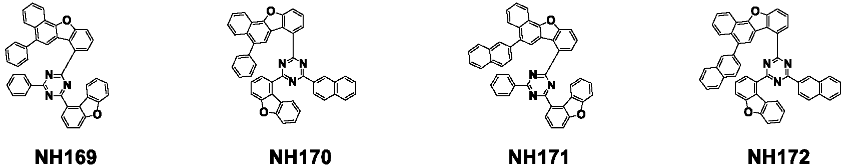

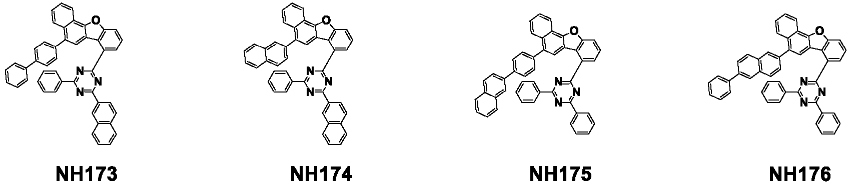

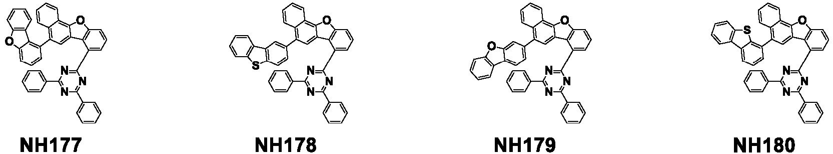

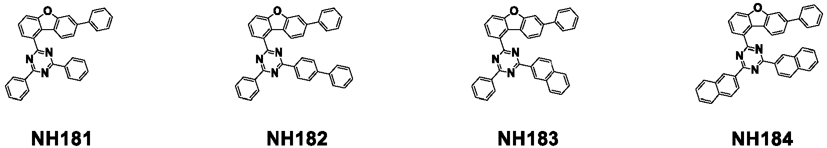

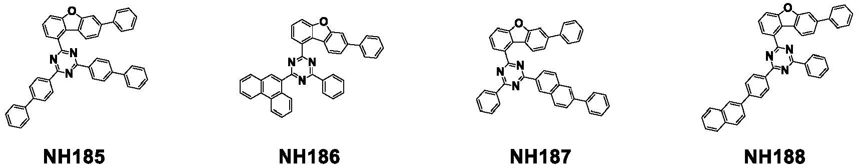

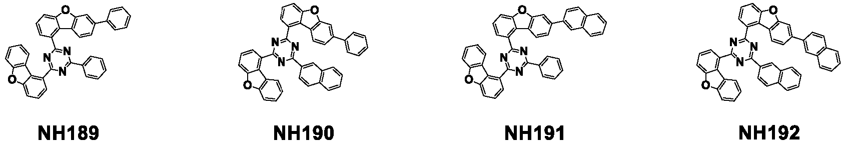

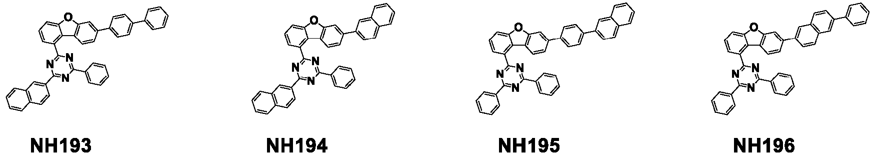

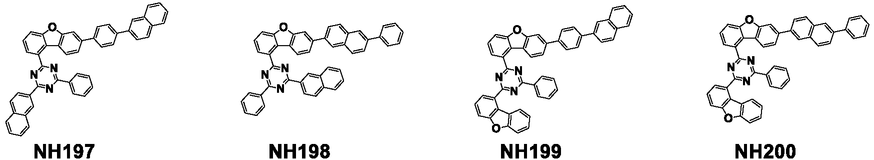

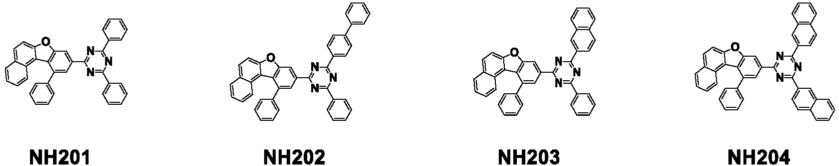

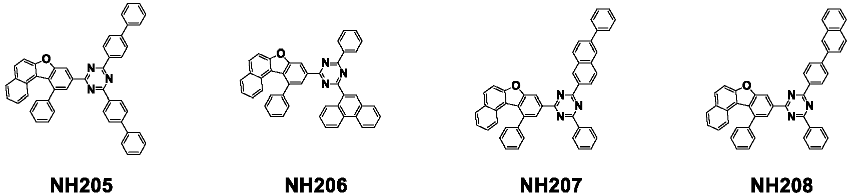

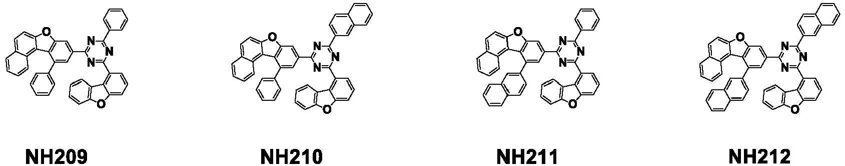

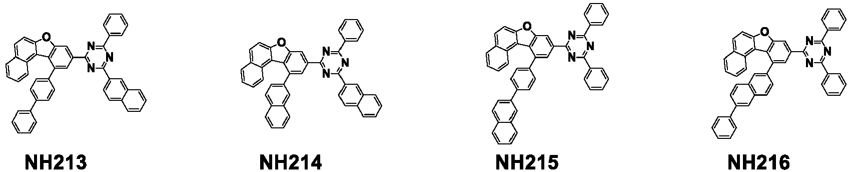

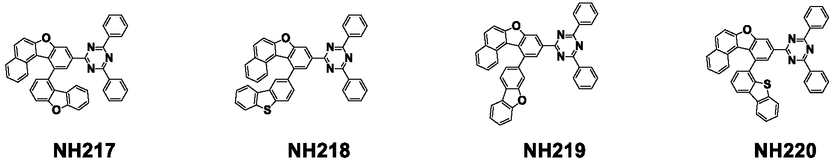

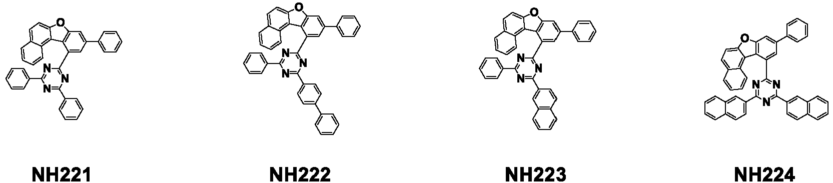

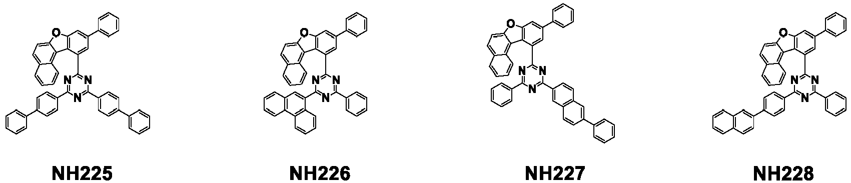

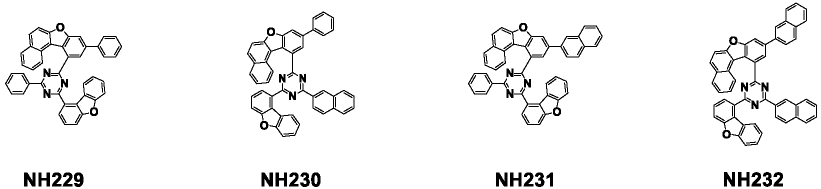

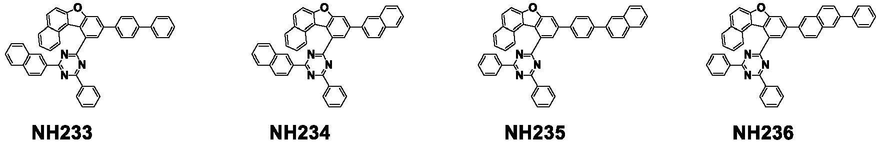

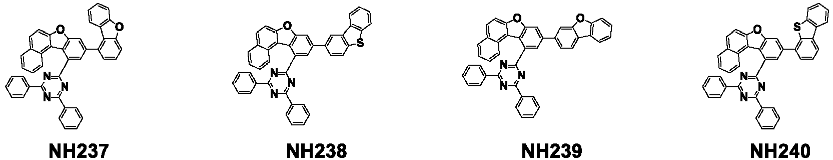

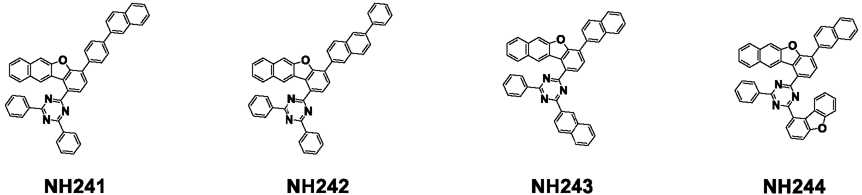

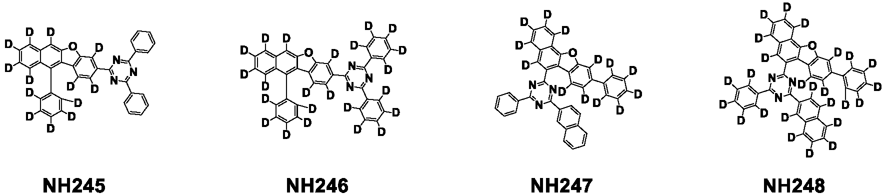

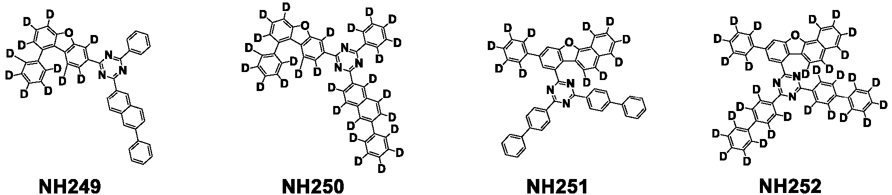

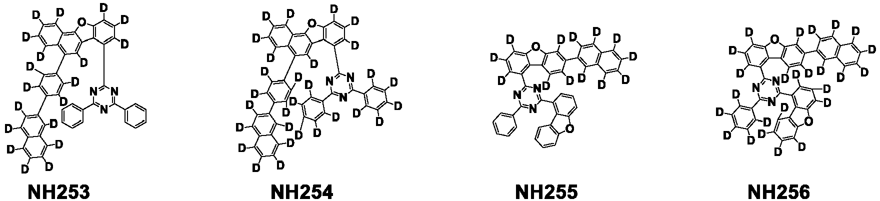

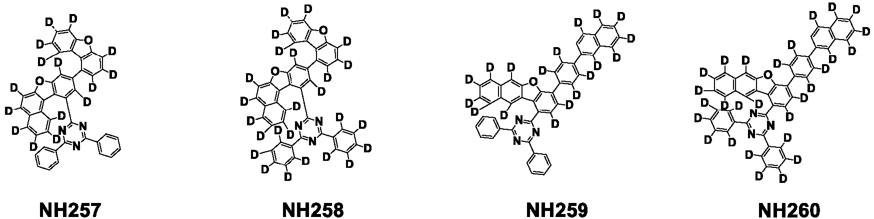

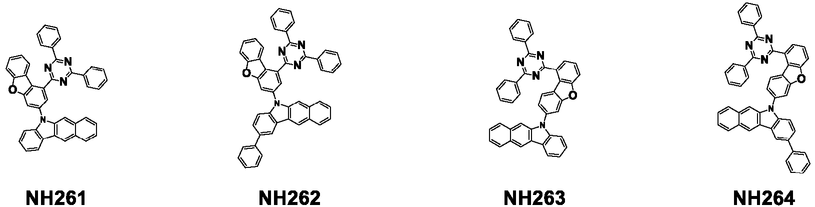

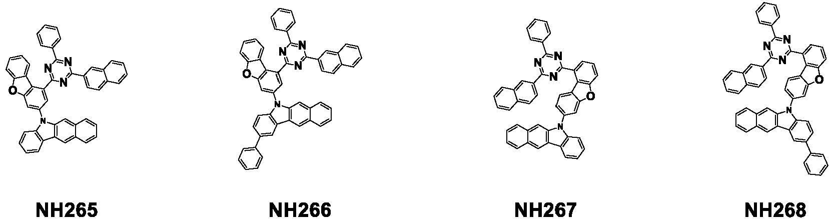

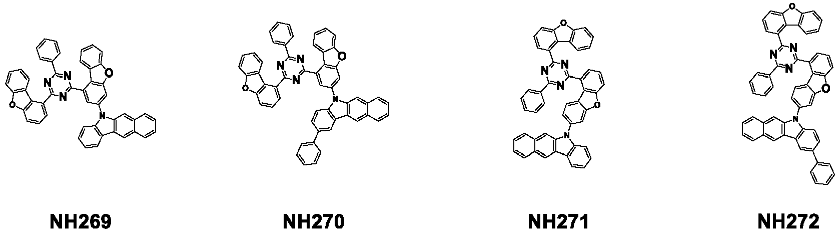

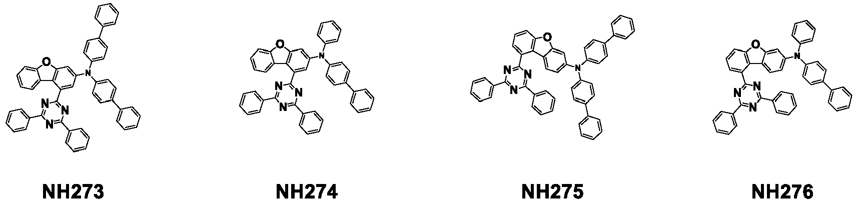

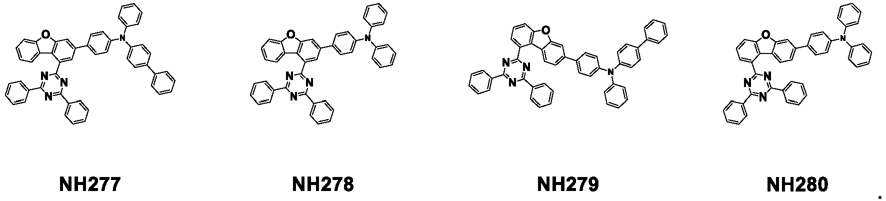

16. The organic light emitting device of claim 7, further comprising one, or two or more layers selected from the group consisting of a light emitting layer, a light emitting auxiliary layer, a hole injection layer, a hole transport layer, an electron injection layer, an electron transport layer, an electron blocking layer and a hole blocking layer.

17. A composition for an organic material layer, the composition comprising:
the heterocyclic compound represented by Chemical Formula 1 of claim 1; and
a heterocyclic compound represented by the following Chemical Formula 4: 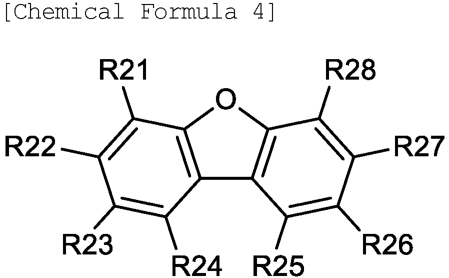
wherein, in Chemical Formula 4,
R21 to R28 are the same as or different from each other, and each independently selected from the group consisting of hydrogen; deuterium; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; -P(=O)R201R202; -SiR201R202R203; the following Chemical Formula 5; the following Chemical Formula 6; and the following Chemical Formula 7, or two or more groups adjacent to each other bond to each other to form a substituted or unsubstituted C6 to C60 aromatic hydrocarbon ring; or a substituted or unsubstituted C2 to C60 heteroring, and R201, R202 and R203 are the same as or different from each other and each independently a substituted or unsubstituted C1 to C60 alkyl group; a substituted or unsubstituted C6 to C60 aryl group; or a substituted or unsubstituted C2 to C60 heteroaryl group; and
any one of R21 to R28 is the following Chemical Formula 5, and another one is the following Chemical Formula 6; or the following Chemical Formula 7, 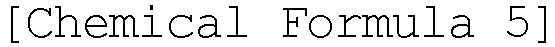 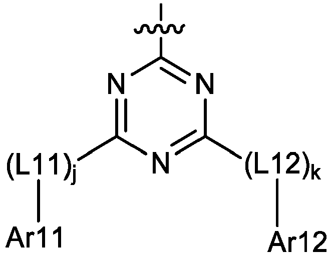 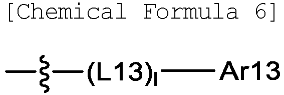 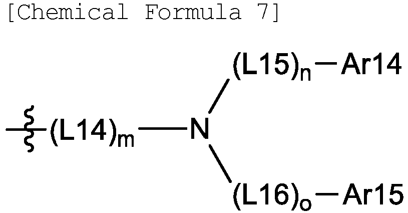
in Chemical Formulae 5 to 7,
L11 to L16 are the same as or different from each other, and each independently a direct bond; a substituted or unsubstituted C6 to C60 arylene group; or a substituted or unsubstituted C2 to C60 heteroarylene group;
j is an integer of 0 to 5, and when j is 2 or greater, L11s are the same as or different from each other;
k is an integer of 0 to 5, and when k is 2 or greater, L12s are the same as or different from each other;
l is an integer of 0 to 5, and when l is 2 or greater, L13s are the same as or different from each other;
m is an integer of 0 to 5, and when m is 2 or greater, L14s are the same as or different from each other;
n is an integer of 0 to 5, and when n is 2 or greater, L15s are the same as or different from each other;
o is an integer of 0 to 5, and when o is 2 or greater, L16s are the same as or different from each other; and
Ar11 to Ar15 are the same as or different from each other, and each independently a substituted or unsubstituted C6 to C60 aryl group; or a substituted or unsubstituted C2 to C60 heteroaryl group.

18. The composition of claim 17, wherein the heterocyclic compound represented by Chemical Formula 1 and the heterocyclic compound represented by Chemical Formula 4 have a weight ratio of 1:9 to 9:1.
